# EUROPÄISCHE PATENTSCHRIFT

(11) **EP 1 537 218 B1**
(45) Veröffentlichungstag und Bekanntmachung des Hinweises auf die Patenterteilung: **13.01.2010**
(21) Anmeldenummer: 03797257.7
(22) Anmeldetag: 29.08.2003
(51) Int. Cl.: C12N 15/82, C12Q 1/68, C07K 14/415, C12N 15/29, C12N 5/10

(54) **TRANSGENE EXPRESSIONSKASSETTEN ZUR EXPRESSION VON NUKLEINSÄUREN IN NICHT-REPRODUKTIVEN BLÜTENGEWEBEN VON PFLANZEN**
TRANSGENIC EXPRESSION CASSETTES FOR EXPRESSING NUCLEIC ACIDS IN NON-REPRODUCTIVE FLOWER TISSUES OF PLANTS
CASSETTES D'EXPRESSION TRANSGENIQUE DESTINEES A L'EXPRESSION D'ACIDES NUCLEIQUES DANS LES TISSUS DE FLEURS NON REPRODUCTIFS DE PLANTES

(30) Priorität: 03.09.2002 DE 10241124
(43) Veröffentlichungstag der Anmeldung: 08.06.2005
(73) Patentinhaber: SunGene GmbH, 06466 Gatersleben (DE)
(72) Erfinder: KLEBSATTEL, Martin, 06484 Quedlinburg (DE); KEETMAN, Ulrich, 06484 Quedlinburg (DE); HERBERS, Karin, 06484 Quedlinburg (DE); FLACHMANN, Ralf, 06484 Quedlinburg (DE); SAUER, Matt, 06484 Quedlinburg (DE); HILLEBRAND, Heike, 68159 Mannheim (DE)
(74) Vertreter: Heistracher, Elisabeth
(86) Internationale Anmeldenummer: PCT/EP2003/009594
(87) Internationale Veröffentlichungsnummer: WO 2004/027070

(56) Entgegenhaltungen:
- EP-A- 0 984 064
- WO-A-99/15679
- HILL THERESA A ET AL: "Discrete spatial and temporal cis-acting elements regulate transcription of the Arabidopsis floral homeotic gene APETALA3" DEVELOPMENT (CAMBRIDGE), Bd. 125, Nr. 9, Mai 1998 (1998-05), Seiten 1711-1721, XP002264345 ISSN: 0950-1991
- TAKATSUJI H ET AL: "A NEW FAMILY OF ZINC FINGER PROTEINS IN PETUNIA: STRUCTURE, DNA SEQUENCE RECOGNITION AND FLORAL ORGAN-SPECIFIC EXPRESSION" PLANT CELL, AMERICAN SOCIETY OF PLANT PHYSIOLOGISTS, ROCKVILLE, MD, US, Bd. 6, Nr. 7, Juli 1994 (1994-07), Seiten 947-958, XP001070764 ISSN: 1040-4651

## Beschreibung

Die Erfindung betrifft Verfahren zur gezielten, transgenen Expression von Nukleinsäuresequenzen in nicht-reproduktiven Blütengeweben von Pflanzen, sowie transgene Expressionskassetten und Expressionsvektoren, die Promotoren mit einer Expressionsspezifität für nicht-reproduktive Gewebe der Blüte enthalten. Die Erfindung betrifft ferner mit diesen transgenen Expressionskassetten oder Expressionsvektoren transformierte Organismen (bevorzugt Pflanzen), davon abgeleitete Kulturen, Teile oder Vermehrungsgut, sowie die Verwendung derselben zur Herstellung von Nahrungs-, Futtermitteln, Saatgut, Pharmazeutika oder Feinchemikalien.

Ziel biotechnologischer Arbeiten an Pflanzen ist die Herstellung von Pflanzen mit vorteilhaften, neuen Eigenschaften zum Beispiel zur Steigerung der landwirtschaftlichen Produktivität, zur Qualitätssteigerung bei Nahrungsmitteln oder zur Produktion bestimmter Chemikalien oder Pharmazeutika (Dunwell JM (2000) J Exp Bot 51 Spec No:487-96). Eine Grundvoraussetzung für die transgene Expression bestimmter Gene ist die Bereitstellung pflanzenspezifischer Promotoren. Promotoren sind wichtige Werkzeuge in der Pflanzenbiotechnologie, um die Expression bestimmter Gene in einer transgenen Pflanze zu steuern und so bestimmte Wesensmerkmals der Pflanze zu erzielen.

Verschiedene pflanzliche Promotoren sind bekannt, zum Beispiel konstitutive Promotoren wie der Promotor der Nopalinsynthase aus Agrobakterium, der TR-Doppelpromotor oder der Promotor des 35S-Transkriptes des Blumenkohlmosaikvirus (CaMV) (Odell et al.(1985) Nature 313:810-812). Nachteilig bei diesen Promotoren ist, dass sie in fast allen Geweben der Pflanze konstitutiv aktiv sind. Eine gezielte Expression von Genen in bestimmten Pflanzenteilen oder zu bestimmten Entwicklungszeitpunkten ist mit diesen Promotoren nicht möglich.

Beschrieben sind Promotoren mit Spezifitäten für verschiedene pflanzliche Gewebe wie Antheren, Ovarien, Blüten, Blätter, Stengel, Wurzeln, Knollen oder Samen. Die Stringenz der Spezifität, als auch die Expressionsaktivität dieser Promotoren ist sehr unterschiedlich.

Die pflanzliche Blüte dient der geschlechtlichen Fortpflanzung der Sameapflanzen. Pflanzliche Blüten - vor allem die Blütenblätter (Petalen) - akkumulieren häufig große Mengen sekundärer Pflanzenstoffe, wie beispielsweise Terpene, Anthocyane, Carotinoide, Alkaloide und Phenylpropanoide, die der Blüte als Duftstoffe, Abwehrstoffe oder als Farbstoffe dienen. Viele dieser Substanzen sind von ökonomischem Interesse. Zudem ist die Blütenknospe und die Blüte der Pflanze ein empfindliches Organ, besonders gegen Stressfaktoren wie Kälte.

Der Arabidopsis thaliana Gen-Locus At3g01980 (GenBank Acc.-No.: NC_003074; Arabidopsis thaliana chromosome 3; Basenpaare: komplement 327677 bis 329029) kodiert für eine putative Dehydrogenase (abgeleitete cDNA: GenBank Acc. -No: NM_111064; SEQ ID NO: 11). Der Arabidopsis thaliana Gen Locus At1g63140 (GenBank Acc.-No: NC_003070. 2; Arabidopsis thaliana chromosome 1; Basenpaare 23069430 bis 23070871) kodiert für eine putative Koffeinsäure-O-methyltransferase (abgeleitete cDNA: NM_104992.1; SEQ ID NO: 13). Die exakte Funktion, Transkription und die Expressionsmuster dieser Gene ist nicht beschrieben..

Blütenspezifische Promotoren, wie beispielsweise der Phytoensynthase Promotor (WO 92/16635), der Promotor des P-rr Gens (WO 98/22593) oder der Promoter des APETALA3 Gens (Hill TA et al. (1998) Development 125:1711-1721) sind bekannt. Diese Promotoren weisen jedoch alle einen oder mehrere Nachteile auf, die eine breite Nutzung beeinträchtigen:
1)Sie sind innerhalb der Blüte spezifisch für ein oder mehrere Blütengewebe und gewährleisten nicht die Expression in allen Geweben der Blüte.
2) Sie sind - wie im Beispiel des an der Blütenentwicklung beteiligten APETALA3 Gens - während der Blütenentwicklung stark reguliert und nicht zu allen Phasen der Blütenentwicklung aktiv.
3) Sie zeigen mitunter starke Nebenaktivitäten in anderen pflanzlichen Geweben. So zeigen die bekannten Promotoren (wie z.B. der APETALA3 Promotor) meist eine Aktivität in Samen, Antheren und den Ovarien der Blüte. Diese stellen empfindliche, direkt an der Fortpflanzung der Pflanzen beteiligte Blütenorganen dar. Eine Expression hier ist in vielen Fällen unnötig und unvorteilhaft, da sie mit der Reproduktion der Pflanze interferieren kann. Zudem kann das exprimierte Genprodukt durch Samen- und Pollenflug unerwünscht verbreitet werden. Dies ist im Sinne einer biotechnologischen Nutzung transgener Pflanzen weitgehend zu vermeiden.

Trotz der Vielzahl bekannter pflanzlicher Promotoren wurde bislang kein Promotor mit einer Spezifität für die pflanzliche Blüte identifiziert, der keine wesentliche Expression in den Pollen und Ovarien besitzt, also nur in den nicht-reproduktiven Geweben aktiv ist. Auch sind keine Promotoren bekannt, die neben der oben genannten Spezifität im wesentlichen während der der gesamten Blütenentwicklung aktiv sind.

Es bestand daher die Aufgabe, Verfahren und geeignete Promotoren für die gezielte, transgene Expression von Nukleinsäuren in den nicht-reproduktiven Blütengeweben bereitzustellen. Diese Aufgabe wurde durch Bereitstellung der Promotoren der Gene mit den Genlokusbezeichnungen At3g01980 (infolge "76L"-Promotor; SEQ ID NO: 1) und At1g63140 (infolge "84L"-Promotor; SEQ ID NO: 2) aus Arabidopsis thaliana gelöst. Diese Promotoren zeigen eine Expression in allen Blütenorganen außer den Pollen und den Ovarien. Dieses Expressionsmuster kann in der Blütenknospe, der Blüte und der welkenden Blüte beobachtet werden.

Ein erster Gegenstand der Erfindung betrifft Verfahren zur gezielten, transgenen Expression von Nukleinsäuresequenzen in nicht-reproduktiven Blütengeweben von Pflanzen, wobei nachfolgende Schritte umfasst sind:
I. Einbringen einer trans genen Expressionskassette in pflanzliche Zellen, wobei die trans gene Expressionskassette mindestens nachfolgende Elemente enthält
   a) mindestens eine Promotorsequenz vermittelnd eine Expression in allen nicht-reproduktiven Blütengeweben jedoch im wesentlichen nicht in Pollen und dem Ovarien, ausgewählt aus der Gruppe von Sequenzen bestehend aus
      i) den Promotorsequenzen gemäß SEQ ID NO: 1 oder 2 und
      ii) funktionellen Äquivalenten der Promotorsequenzen gemäß SEQ ID NO: 1 oder 2 mit im wesentlichen der gleichen Promotoraktivität wie ein Promotor gemäß SEQ ID NO: 1 oder 2 und die eine Identität von mindestens 80% zu der Sequenz eines der Promotoren gemäss SEQ ID No. 1 oder 2 aufweisen und
   b) mindestens eine weitere Nukleinsäuresequenz, und
   c) gegebenenfalls weitere genetische Kontrollelemente,
      wobei mindestens eine Promotorsequenz und eine weitere Nukleinsäuresequenz funktionell miteinander verknüpft sind und die weitere Nukleinsäuresequenz in Bezug auf die Promotorsequenz heterolog ist, und
II. Auswahl von transgenen Zellen, die besagte Expressionskassette stabil in das Genom integriert enthalten, und
III. Regeneration von vollständigen Pflanzen aus besagten transgenen Zellen, wobei mindestens eine der weiteren Nukleinsäuresequenzen in im wesentlichen allen nicht-reproduktiven Blütengeweben, jedoch im wesentlichen nicht im Pollen und den Ovarien exprimiert wird.

Ein weiterer Gegenstand betrifft transgene Expressionskassetten, wie sie z.B. in dem erfindungsgemäßen Verfahren zum Einsatz kommen können. Diese transgenen Expressionskassetten zur gezielten, trans genen Expression von Nukleinsäuresequenzen in nicht-reproduktiven Blütengeweben von Pflanzenn umfassen:
a) mindestens eine Promotorsequenz vermittelnd eine Expression in allen nicht-reproduktiven Blütengeweben jedoch im wesentlichen nicht in Pollen und den Ovarien ausgewählt aus der Gruppe von Sequenzen.bestehend aus
   i) den Promotorsequenzen gemäß SEQ ID NO: 1 oder 2 und
   ii) funktionellen Äquivalenten der Promotorsequenzen gemäß SEQ ID NO: 1 oder2 mit im wesentlichen der gleichen Promotoraktivität wie ein Promotor gemäß SEQ ID NO: 1 oder 2 und die eine Identität von mindestens 80% zu der Sequenz eines der Promotoren gemäss SEQ ID No. 1 oder 2 aufweisen und
b) mindestens eine weitere Nukleinsäuresequenz, und
c) gegebenenfalls weitere genetische Kontrollelemente,
wobei mindestens eine Promotorsequenz und eine weitere Nukleinsäuresequenz funktionell miteinander verknüpft sind und die weitere Nukleinsäuresequenz in Bezug auf die Promotorsequenz heterolog ist.

Die erfindungsgemäßen Expressionskassetten können weitere genetische Kontrollsequenzen und/oder zusätzliche Funktionselemente enthalten.

Bevorzugt können die transgenen Expressionskassetten durch die transgen zu exprimierende Nukleinsäuresequenz die Expression eines von besagter Nukleinsäuresequenz kodierten Proteins, und/oder die Expression einer von besagter Nukleinsäuresequenz kodierten sense-RNA, anti-sense-RNA oder doppelsträngigen RNA ermöglichen.

Ein weiterer Gegenstand der Erfindung betrifft transgene Expressionsvektoren, die eine der erfindungsgemäßen Expressionskassetten enthalten.

Ein weiterer Gegenstand der Erfindung betrifft transgene Organismen, die eine der erfindungsgemäßen Expressionskassetten oder Expressionsvektoren enthalten. Der Organismus kann ausgewählt sein aus der Gruppe bestehend aus Bakterien, Hefen, Pilzen, und pflanzlichen Organismen oder von diesen abgeleitete Zellen, Zellkulturen, Teile, Gewebe, Organe oder Vermehrungsgut, bevorzugt ist der Organismus ausgewählt aus der Gruppe der landwirtschaftlichen Nutzpflanzen.

Ein weiterer Gegenstand der Erfindung betrifft die Verwendung der besagten Organismen oder von diesen abgeleitete Zellen, Zellkulturen, Teile, Gewebe, Organe oder Vermehrungsgut zur Herstellung von Nahrungs-, Futtermittel, Saatgut, Pharmazeutika oder Feinchemikalien, wobei die Feinchemikalien bevorzugt Enzyme, Vitamine, Aminosäuren, Zucker, gesättigte oder ungesättigte Fettsäuren, natürliche oder synthetische Geschmacks-, Aroma- oder Farbstoffe sind. Erfindungsgemäß umfasst sind ferner Verfahren zur Herstellung besagter Nabrungs-, Futtermitteln, Saatgut, Pharmazeutika oder Feinchemikalien unter Einsatz der erfindungsgemäßen Organismen oder von diesen abgeleitete Zellen, Zellkulturen, Teile, Gewebe, Organe oder Vermehrungsgut.

Die erfindungsgemäßen transgenen Expressionskassetten sind aus nachfolgenden Gründen besonders vorteilhaft:
a) Sie gewähren eine selektive Expression in den nicht-reproduktiven Geweben der Blütenknospe und der Blüte der Pflanze und ermöglichen zahlreiche Anwendungen, wie beispielsweise eine Resistenz gegen Stressfaktoren wie Kälte oder eine gezielte Synthese von sekundäre Pflanzenstoffe. Die Expression ist im wesentlichen konstant über den gesamten Entwicklungszeitraum der Blütenknopse und Blüte.
b) Sie zeigen keine Expression in reproduktiven Geweben (z.B. Pollen oder Ovarien), wodurch Störungen der Fortpflanzung und eine Verbreitung des transgenen Proteins durch Pollen- oder Samenflug vermieden wird.

Die erfindungsgemäßen transgenen Expressionskassetten, die von ihnen abgeleiteten transgenen Expressionsvektoren und transgenen Organismen können funktionelle Äquivalente zu den unter SEQ ID NO: 1 oder 2 beschriebenen Promotorsequenzen umfassen.

Die Promotoraktivität eines funktionell äquivalenten Promotors wird als "im wesentlichen gleich" bezeichnet, wenn die Transkription einer bestimmten transgen zu exprimierenden Nukleinsäuresequenz unter Kontrolle des besagten funktionell äquivalenten Promotors unter ansonsten unveränderten Bedingungen eine gezielte Expression in im wesentlichen allen nicht-reproduktiven Blütengeweben zeigt, jedoch in den Pollen und Ovarien im wesentlichen keine Expression zeigt.

"Blüte" meint allgemein einen Spross begrenzten Wachstums, dessen Blätter zu Fortpflanzungsorganen umgewandelt sind. Die Blüte besteht aus verschiedenen "Blütengeweben" wie z.B. den Kelchblätter (Sepalen), den Kronblätter (Petalen), den Staubblätter (oder Staub"gefäßen"; Stamina) oder den Fruchtblätter (Karpellen). Als Androeceum wird in der Blüte die Gesamtheit der Staubblätter (Stamina) bezeichnet. Die Staubblätter befinden sich innerhalb des Petalen- bzw. Sepalenkreises. Ein Staubblatt gliedert sich in ein Filament und eine am Ende sitzende Anthere. Diese wiederum unterteilt sich in zwei Theken, welche durch ein Konvektiv miteinander verbunden sind. Jede Theke besteht aus je zwei Pollensäcken, in denen der Pollen gebildet wird.

"Nicht-reproduktives Blütengewebe" meint alle Gewebe der Blüte bis auf den Pollen und die Ovarien.

"Im wesentlichen alle nicht-reproduktiven Blütengewebe" meint in Bezug auf die nicht-reproduktiven Blütengewebe, dass einzelne dieser Gewebe insgesamt oder zu bestimmten Zeitpunkten der Entwicklung keine wesentliche Expression aufweisen können, wobei der Anteil dieser Gewebe jedoch bevorzugt weniger als 20 Gew%, bevorzugt weniger als 10 Gew.-%, besonders bevorzugt weniger als 5 Gew.-%, ganz besonders bevorzugt weniger als 1 Gew% an dem Gesamtgewicht der nicht-reproduktiven Blütengeweben beträgt.

"Gezielt" meint in Bezug auf die Expression in nicht-reproduktiven Blütengeweben bevorzugt, dass die Expression unter Kontrolle eines der erfindungsgemäßen Promotoren in den nicht-reproduktiven Blütengeweben bevorzugt mindestens das zehnfache, ganz besonders bevorzugt mindestens das fünfzigfache, am meisten bevorzugt mindestens das hundertfache beträgt als in einem anderen Gewebe, beispielsweise dem Pollen oder den Ovarien oder einem Nicht-Blütengewebe wie beispielsweise den Blättern.

Dass die erfindungsgemäßen Promotoren "im wesentlichen keine Expression in den Pollen und Ovarien zeigen", meint bevorzugt, dass die der statistischen Mittelwert der Expression über alle reproduktiven Blütengewebe maximal 10 %, bevorzugt maximal 5 %, am meisten bevorzugt maximal 1% des statistischen Mittelwerts der Expression über alle nicht-reproduktiven Blütengewebe unter den gleichen Bedingungen beträgt.

Bevorzugt ist die Expression innerhalb der nicht-reproduktiven Blütengewebe im wesentlichen konstant. "Im wesentlichen Konstant" bedeutet dabei bevorzugt, dass die Standartabweichung der Expression zwischen den einzelnen nicht-reproduktiven Blütengeweben bezogen auf den statistischen Mittelwert der Expression über alle nicht-reproduktiven Blütengewebe geringer ist als 50 %, bevorzugt 20 %, besonders bevorzugt 10 %, ganz besonders bevorzugt 5 %.

Bevorzugt ist die Expression innerhalb mindestens eines bestimmten nicht-reproduktiven Blütengewebes über alle Entwicklungsstufen der Blüte im wesentlichen konstant. "Im wesentlichen konstant" bedeutet dabei bevorzugt, dass die Standartabweichung der Expression zwischen den einzelnen Entwicklungszeitpunkten des jeweiligen nicht-reproduktiven Blütengewebes bezogen auf den statistischen Mittelwert der Expression über alle Entwicklungszeitpunkte geringer ist als 50 %, bevorzugt 20 %, besonders bevorzugt 10 %, ganz besonders bevorzugt 5 %.

Bevorzugt werden im Rahmen der Ermittlung der Expressionshöhe solche Nukleinsäuresequenzen in funktioneller Verknüpfung mit dem zu prüfenden Promotor eingesetzt, die für leicht quantifizierbare Proteine kodieren. Ganz besonders bevorzugt sind dabei Reporter-proteine (Schenborn E, Groskreutz D (1999) Mol Biotechnol 13(1): 29-44) wie "green fluorescence protein" (GFP) (Chui WL et al. (1996) Curr Biol 6:325-330; Leffel SM et al.(1997) Biotechniques 23(5):912-8), Chloramphenicoltransferase, Luziferase (Millar et al. (1992) Plant Mol Biol Rep 10:324-414), β-Glucuronidase oder β-Galactosidase. Ganz besonders bevorzugt ist die β-Glucuronidase (Jefferson et al. (1987) EMBO J 6:3901-3907).

"Ansonsten unveränderte Bedingungen" bedeutet, dass die Expression, die durch eine der zu vergleichenden transgenen Expressionskassetten initiiert wird, nicht durch Kombination mit zusätzlichen genetischen Kontrollsequenzen, zum Beispiel Enhancer-Sequenzen, modifiziert wird. Unveränderte Bedingungen bedeutet ferner, dass alle Rahmenbedingungen wie beispielsweise Pflanzenart, Entwicklungsstadium der Pflanzen, Zuchtbedingungen, Assaybedingungen (wie Puffer, Temperatur, Substrate etc.) zwischen den zu vergleichenden Expressionen identisch gehalten werden.

"Transgen" meint - zum Beispiel bezüglich einer Expressionskassette (oder einen diese umfassenden Expressionsvektor oder transgenen Organismus) alle solche durch gentechnische Methoden zustandegekommene Konstruktionen, in denen sich entweder
a) der Promotor gemäß SEQ ID NO: 1 oder 2 oder ein funktionelles Äquivalent desselben oder ein funktionell äquivalentes Teil der vorgenannten, oder
b) eine mit a) funktionell verknüpfte weitere Nukleinsäuresequenz, oder
c) (a) und (b)
   sich nicht in ihrer natürlichen, genetischen Umgebung befinden oder durch gentechnische Methoden modifiziert wurden, wobei die Modifikation beispielhaft eine Substitutionen, Additionen, Deletionen, Inversion oder Insertionen eines oder mehrerer Nukleotidreste sein kann. Bevorzugt ist die in den Expressionskassetten enthaltene erfindungsgemäße Promotorsequenz (z.B. die Sequenz gemäß SEQ ID NO: 1, 2, 3 oder 4) heterolog in Bezug auf die mit ihr funktionell verknüpfte, transgen zu exprimierende weitere Nukleinsäuresequzenz. "Heterolog" meint in diesem Zusammenhang, dass die weitere Nukleinsäuresequenz nicht für das Gen kodiert, das natürlicherweise unter der Kontrolle des besagten Promotors steht.

"Natürliche genetische Umgebung" meint den natürlichen chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erholten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommendes Expressionskassette - beispielsweise die natürlich vorkommende Kombination des Promotors eines Gens kodierend für ein Protein gemäß SEQ ID NO: 12 oder 14 oder ein funktionelles Äquivalent desselben mit seinen entsprechenden kodierenden Sequenzen wird zu einem transgenen Expressionskonstrukt, wenn diese durch nicht-natürliche, synthetische ("künstliche") Verfahren wie beispielsweise einer Mutagenisierung geändert wird. Entsprechende Verfahren sind beschrieben (US 5,565,350; WO 00/15815; siehe auch oben).

"Transgen" meint in Bezug auf eine Expression ("transgene Expression") bevorzugt all solche unter Einsatz einer transgenen Expressionskassette, eines transgenen Expressionsvektors oder transgenen Organismus - entsprechend dem oben gegebenen Definitionen - realisierten Expressionen.

Funktionelle Äquivalente eines Promotors gemäß SEQ ID NO: 1 oder 2 meint insbesondere natürliche oder künstliche Mutationen eines Promotors gemäß SEQ ID NO: 1 oder 2 sowie homologe Sequenzen aus anderen Organismen, bevorzugt aus pflanzlichen Organismen, die im wesentlichen die gleiche Promotoraktivität wie einer der Promotoren gemäß SEQ ID NO: 1 oder 2 aufweisen.

Funktionelle Äquivalente umfasst auch all die Sequenzen, die von dem komplementären Gegenstrang der durch SEQ ID NO: 1 oder 2 definierten Sequenzen abgeleitet sind und im wesentlichen die gleiche Promotoraktivität aufweisen.

Funktionelle Äquivalente zu den Promotoren gemäß SEQ ID NO: 1 oder 2 umfasst bevorzugt solche Sequenzen die
a) im wesentlichen die gleiche Promotoraktivität wie einer der Promotoren gemäß SEQ ID NO: 1 oder 2 aufweisen und
b) die eine Homologie aufweisen von mindestens 50 %, bevorzugt 70 %, vorzugsweise mindestens 80 %, besonders bevorzugt mindestens 90 %, ganz besonders bevorzugt mindestens 95 %, am meisten bevorzugt 99% zu der Sequenz eines der Promotoren gemäß SEQ ID NO: 1 oder 2, wobei sich die Homologie über eine Länge von von mindestens 100 Basenpaaren, bevorzugt mindestens 200 Basenpaaren, besonders bevorzugt von mindestens 300 Basenpaaren, ganz besonders bevorzugt von mindestens 400 Basenpaaren, am meistens bevorzugt von mindestens 500 Basenpaaren erstreckt.

Dabei kann die Expressionshöhe der funktionellen Äquivalente sowohl nach unten als auch nach oben im Vergleich zu einem Vergleichswert abweichen. Bevorzugt sind dabei solche Sequenzen, deren Expressionshöhe, gemessen anhand der transkribierten mRNA oder dem infolge translatierten Protein, unter ansonsten unveränderten Bedingungen quantitativ um nicht mehr als 50 %, bevorzugt 25 %, besonders bevorzugt 10 % von einem Vergleichswert erhalten mit denen durch SEQ ID NO: 1 oder 2 beschriebenen Promotoren unterscheidet. Besonders bevorzugt sind solche Sequenzen, deren Expressionshöhe, gemessen anhand der transkribierten mRNA oder dem infolge translatierten Protein, unter ansonsten unveränderten Bedingungen quantitativ um mehr als 50 %, bevorzugt 100 %, besonders bevorzugt 500 %, ganz besonders bevorzugt 1000 % einen Vergleichswert erhalten mit dem durch SEQ ID NO: 1 oder 2 beschriebenen Promotor übersteigt.

Beispiele für die in den erfindungsgemäßen transgenen Expressionskassetten oder transgenen Expressionsvektoren zum Einsatz kommenden Promotorsequenzen lassen sich beispielsweise in verschiedenen Organismen, deren genomische Sequenz bekannt ist, wie beispielsweise Arabidopsis thaliana, Brassica napus, Nicotiana tabacum, Solanum tuberosum, Helianthium annuus, Linum sativum durch Homologievergleiche in Datenbanken leicht auffinden. Bevorzugt kann man dazu von den kodierenden Regionen der Gene ausgehen, deren Promotoren durch SEQ ID NO 1 oder 2 beschrieben sind. Ausgehend von beispielsweise den cDNA Sequenzen dieser Gene beschrieben durch SEQ ID NO: 11 oder 13 oder den davon abgeleiteten Proteinsequenzen beschrieben durch SEQ ID NO: 12 oder 14 können die entsprechenden homologen Gene in anderen Pflanzenarten durch Durchmusterung von Datenbanken oder Genbanken (unter Verwendung von entsprechenden Gensonden) leicht in der dem Fachmann geläufigen Weise identifiziert werden.

In einer bevorzugten Ausführungsform der Erfindung umfassen funktionelle Äquivalente des Promotors beschrieben durch SEQ ID NO:1 all solche Promotoren, die sich in einem pflanzlichen Organismus in 5'-Richtung vor einer genomischen Sequenz befinden, die für ein Protein mit einer Homologie von mindestens 60%, bevorzugt mindestens 80%, besonders bevorzugt mindestens 90%, am meisten bevorzugt mindestens 95% zu dem Protein gemäß SEQ ID NO: 12 kodiert, wobei besagte Promotoren den natürlichen Promotor der besagten genomischen Sequenz darstellen. Besonders bevorzugt umfassen funktionelle Äquivalente des Promotors beschrieben durch SEQ ID NO: 1 all solche Promotoren, die sich in einem pflanzlichen Organismus in 5'-Richtung vor einer genomischen Sequenz befinden, die für eine Nukleinsäuresequenz kodiert, deren abgeleitete cDNA eine Homologie von mindestens 60%, bevorzugt mindestens 80%, besonders bevorzugt mindestens 90%, am meisten bevorzugt mindestens 95% zu der Nukleinsäuresequenz gemäß SEQ ID NO: 11 hat, wobei besagte Promotoren den natürlichen Promotor der besagten genomischen Sequenz darstellen. Bevorzugt sind Promotoren, die einen Sequenzbereich von mindestens 250 Basenpaaren, bevorzugt mindestens 500 Basenpaaren, besonders bevorzugt 1000 Basenpaaren, am meisten bevorzugt mindestens 2000 Basenpaaren in 5'-Richtung gerechnet vom ATG-Startkodon der besagten genomischen Sequenzen umfassen. Besonders bevorzugt sind funktionelle Äquivalente des Promotors beschrieben durch SEQ ID NO: 1 all solche Promotoren, die sich in einem pflanzlichen Organismus in 5'-Richtung vor einer genomischen Sequenz befinden, die für ein Protein kodiert das mindestens eines der nachfolgenden Sequenzmotive enthält:

| | | |
|---|---|---|
| 1. | NGD(E/Q)VSRNIA | (SEQ ID NO: 23) |
| 2. | LAKHGC(R/K)LV | (SEQ ID NO: 24) |
| 3. | MGNEXSLRSXVDXIR | (SEQ ID NO: 25) |
| 4. | TYQGKXQDILXVS(Q/E)DEF | (SEQ ID NO: 26) |
| 5. | IT(K/R) INLTAXWFXLKAVA | (SEQ ID NO: 27) |

Ganz besonders bevorzugt sind als funktionelle Äquivalente des Promotors beschrieben durch SEQ ID NO: 1 solche Promotoren, die sich in einem pflanzlichen Organismus in 5'-Richtung vor einer genomischen Sequenz befinden, welche für ein Protein kodiert, wobei besagtes Protein mindestens eine der nachfolgenden Sequenzen umfasst:
1. die homologe Sequenz (H2) aus Raps gemäß SEQ ID NO: 16
2. die homologe Sequenz (H3) aus Raps gemäß SEQ ID NO: 18

Am meisten bevorzugt sind als funktionelle Äquivalente des Promotors beschrieben durch SEQ ID NO: 1 solche Promotoren, die sich in einem pflanzlichen Organismus in 5'-Richtung vor einer genomischen Sequenz befinden, welche für eine Nukleinsäuresequenz kodiert, deren abgeleitete cDNA mindestens eine der nachfolgenden Sequenzen umfasst:
1. die homologe Sequenz (H2) aus Raps gemäß SEQ ID NO: 15
2. die homologe Sequenz (H3) aus Raps gemäß SEQ ID NO: 17

In einer bevorzugten Ausführungsform der Erfindung umfassen funktionelle Äquivalente des Promotors beschrieben durch SEQ ID NO: 2 all solche Promotoren, die sich in einem pflanzlichen Organismus in 5'-Richtung vor einer genomischen Sequenz befinden, die für ein Protein mit einer Homologie von mindestens 60 %, bevorzugt mindestens 80 %, besonders bevorzugt mindestens 90 %, am meisten bevorzugt mindestens 95 % zu dem Protein gemäß SEQ ID NO: 14 kodiert, wobei besagte Promotoren den natürlichen Promotor der besagten genomischen Sequenz darstellen. Besonders bevorzugt umfassen funktionelle Äquivalente des Promotors beschrieben durch SEQ ID NO: 2 all solche Promotoren, die sich in einem pflanzlichen Organismus in 5'-Richtung vor einer genomischen Sequenz befinden, die für eine Nukleinsäuresequenz kodiert, deren abgeleitete cDNA eine Homologie von mindestens 60%, bevorzugt mindestens 80%, besonders bevorzugt mindestens 90%, am meisten bevorzugt mindestens 95% zu der Nukleinsäuresequenz gemäß SEQ ID NO: 13 hat, wobei besagte Promotoren den natürlichen Promotor der besagten genomischen Sequenz darstellen. Bevorzugt sind Promotoren, die einen Sequenzbereich von mindestens 250 Basenpaaren, bevorzugt mindestens 500 Basenpaaren, besonders bevorzugt 1000 Basenpaaren, am meisten bevorzugt mindestens 2000 Basenpaaren in 5'-Richtung gerechnet vom ATG-Startkodon der besagten genomischen Sequenzen umfassen.Besonders bevorzugt sind funktionelle Äquivalente des Promotors beschrieben durch SEQ ID NO: 2 all solche Promotoren, die sich in einem pflanzlichen Organismus in 5'-Richtung vor einer genomischen Sequenz befinden, die für ein Protein kodiert das mindestens eines der nachfolgenden Sequenzmotive enthält:

| | | |
|---|---|---|
| 1. | AEPVCTXFL | (SEQ ID NO: 28) |
| 2. | EGKDXFXSAHGMXXFE | (SEQ ID NO: 29) |
| 3. | EQFAXMFNXAM | (SEQ ID NO: 30) |
| 4. | ATXIMKK(V/I)LEVY(K/R)GFED | (SEQ ID NO: 31) |
| 5. | TLVD(V/I)GGGXGT | (SEQ ID NO: 32) |

Ganz besonders bevorzugt sind als funktionelle Äquivalente des Promotors beschrieben durch SEQ ID NO: 2 solche Promotoren, die sich in einem pflanzlichen Organismus in 5'-Richtung vor einer genomischen Sequenz befinden, welche für ein Protein kodiert, wobei besagtes Protein mindestens eine der nachfolgenden Sequenzen umfasst:
1. die homologe Sequenz (H4) aus Raps gemäß SEQ ID NO: 20
2. die homologe Sequenz (H5) aus Raps gemäß SEQ ID NO: 22

Am meisten bevorzugt sind als funktionelle Äquivalente des Promotors beschrieben durch SEQ ID NO: 2 solche Promotoren, die sich in einem pflanzlichen Organismus in 5'-Richtung vor einer genomischen Sequenz befinden, welche für eine Nukleinsäuresequenz kodiert, deren abgeleitete cDNA mindestens eine der nachfolgenden Sequenzen umfasst:
1. die homologe Sequenz (H4) aus Raps gemäß SEQ ID NO: 19
2. die homologe Sequenz (H5) aus Raps gemäß SEQ ID NO: 21

Ein weiterer Gegenstand der Erfindung betrifft die Verwendung mindestens einer Nukleinsäuresequenz oder eines Teils derselben in Verfahren zur Identifikation und/oder Isolation von Promotoren mit Spezifität für nicht-reproduktive Blütengewebe von Genen, die für besagte Nukleinsäuresequenz kodieren, wobei besagte Nukleinsäuresequenz für eine Aminosäuresequenz kodiert, die mindestens eine Sequenz gemäß SEQ ID NO: 23, 24, 25, 26, 27, 28, 29, 30, 31 oder 32 oder eine für diese Sequenzen angegebene Variation umfasst. Bevorzugt kodiert besagte Nukleinsäuresequenz für eine Aminosäuresequenz umfassend eine Sequenz gemäß SEQ ID NO: 12, 14, 16, 18, 20 oder 22. Besonders bevorzugt umfasst besagte Nukleinsäuresequenz eine Sequenz gemäß SEQ ID NO: 11, 13, 15, 17, 19 oder 21. "Teil" meint in Bezug auf die Nukleinsäuresequenz bevorzugt eine Sequenz von mindestens 10 Basen, bevorzugt 15 Basen, besonders bevorzugt 20 Basen, am meisten bevorzugt 30 Basen.

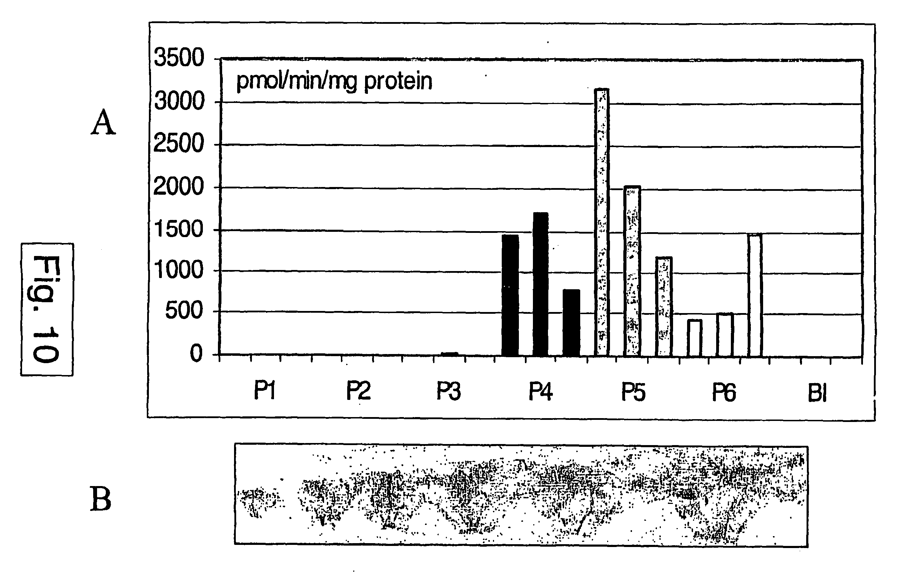

Dem Fachmann sind verschiedene Verfahren bekannt, um ausgehend von einer Nukleinsäuresequenz (z.B. einem Gentranskript wie beispielsweise einer cDNA) den Promotor des entsprechenden Genes zu identifizieren und zu isolieren. Dazu stehen beispielsweise prinzipiell alle Methoden zur Amplifikation flankierender chromosomaler Sequenzen zur Verfügung. Die beiden am häufigsten genutzten Verfahren sind die inverse PCR ("PCR"; schematisch dargestellt in Fig. 10) und die "Thermal Asymmetric Interlaced PCR" ("TAIL PCR").

Für die "iPCR" wird genomische DNA des Organismus, aus dem der funktionell äquivalente Promotor zu isolieren ist, mit einem gegebenen Restriktionsenzym komplett verdaut und anschließend werden in einem verdünnten Ansatz die einzelnen Fragmente rückligiert, also mit sich selbst zu einem ringförmigen Molekül verbunden. In der Vielzahl enstehender ringförmiger DNA-Moleküle befinden sich auch solche, die die bekannte Sequenz (beispielsweise die Sequenz kodierend für das homologe Protein) enthalten. Ausgehend davon kann das ringförmige Molekül mittels PCR amplifiziert werden, indem ein Primerpaar verwendet wird, bei dem beide Primer sich an den bekannten Sequenzabschnitt anlagern können. Eine Ausführungsmöglichkeit für die "iPCR" ist beispielhaft in Beispiel 6 wiedergegeben.

Die "TAIL-PCR" beruht auf der Verwendung von einerseits einem Satz sukzessive verkürzter hochspezifischer Primer, die sich an die bekannte genomische Sequenz (beispielsweise die Sequenz kodierend für das homologe Protein) anlagern, und andererseits einem Satz kürzerer Zufallsprimer mit geringer Schmelztemperatur, so dass eine sequenzunspezifischere Anlagerung an die bekannte genomische Sequenz flankierende genomische DNA erfolgt. Die Anlagerung der Primer an die zu amplifizierende DNA kann mit einer solchen Primerkombimation so gestaltet werden, dass eine spezifische Amplifikation der gewünschten Zielsequenz möglich wird. Eine Ausführungsmöglichkeit für die "TAIL-PCR" ist beispielhaft in Beispiel 5 wiedergegeben.

Ein weiterer Gegenstand der Erfindung betrifft daher Verfahren zur Herstellung einer transgenen Expressionskassette mit Spezifität für nicht-reproduktive Blütengewebe, umfassend nachfolgende Schritte:
I. Isolation eines Promotors mit Spezifität für nicht-reproduktive Blütengewebe, wobei bei der Isolation mindestens eine Nukleinsäuresequenz oder ein Teil derselben zum Einsatz kommt, wobei besagte Nukleinsäuresequenz für eine Aminosäuresequenz kodiert, die mindestens eine Sequenz gemäß SEQ ID NO: 23, 24, 25, 26, 27, 28, 29, 30, 31 oder 32 oder eine für diese Sequenzen angegebene Variation umfasst.
II. Funktionelle Verknüpfung besagten Promotors mit einer weiteren Nukleinsäuresequenz, wobei besagte Nukleinsäuresequenz in Bezug auf den Promotor heterolog ist.

Bevorzugt kodiert besagte Nukleinsäuresequenz für eine Aminosäuresequenz umfassend eine Sequenz gemäß SEQ ID NO: 12, 14, 16, 18, 20 oder 22. Besonders bevorzugt umfasst besagte Nukleinsäuresequenz eine Sequenz gemäß SEQ ID NO: 11, 13, 15, 17, 19 oder 21. "Teil" meint in Bezug auf die Nukleinsäuresequenz bevorzugt eine Sequenz von mindestens 10 Basen, bevorzugt 15 Basen, besonders bevorzugt 20 Basen, am meisten bevorzugt 30 Basen. In einer Bevorzugten Ausführungsform basiert das erfindungsgemäße Verfahren auf der Polymerasekettenreaktion, wobei die besagte Nukleinsäuresequenz oder ein Teil derselben als Primer eingesetzt wird. Im Rahmen der funktionellen Verknüpfung können dem fachmann bekannte Verfahren wie z.B. Ligation etc. eingesetzt werden (s.u.).

"Mutation" meint Substitution, Addition, Deletion, Inversion oder Insertion eines oder mehrerer Nukleotidreste. Somit werden beispielsweise auch solche Nukleotidsequenzen durch die vorliegende Erfindung mit umfasst, welche man durch Modifikation der Promotoren gemäß SEQ ID NO: 1 oder 2 erhält. Ziel einer solchen Modifikation kann die weitere Eingrenzung der darin enthaltenen Sequenz oder z.B. auch die Einfügung weiterer Restriktionsenzymschnittstellen, die Entfernung überflüssiger DNA oder das Hinzufügen weiterer Sequenzen, zum Beispiel weiterer regulatorischer Sequenzen, sein.

Wo Insertionen, Deletionen oder Substitutionen, wie z.B. Transitionen und Transversionen, in Frage kommen, können an sich bekannte Techniken, wie in vitro-Mutagenese, "primer repair", Restriktion oder Ligation verwendet werden. Transition meint einen Basenpaaraustausch eines Purin/Pyrimidin-Paares in ein anderes Puria/Pyrimidin-Paar (z.B. A-T gegen G-C). Transversion meint einen Basenpaaraustausch eines Purin/Pyrimidin-Paares gegen ein Pyrimidin/Purin-Paar (z.B. A-T gegen T-A). Deletion meint die Entfernung eines oder mehrerer Basenpaare. Insertion meint die Einführung eines oder mehrerer Basenpaare.

Durch Manipulationen, wie z.B. Restriktion, "chewing-back" oder Auffüllen von Überhängen für "blunt ends" können komplementäre Enden der Fragmente für die Ligation zur Verfügung gestellt werden. Zu analogen Ergebnissen kann man auch unter Verwendung der Polymerasekettenreaktion (PCR) unter Verwendung spezifischer Oligonukleotid-Primer kommen.

Unter Homologie zwischen zwei Nukleinsäuren wird die Identität der Nukleinsäuresequenz über die jeweils gesamte Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 12 | Length Weight: 4 |
| Average Match: 2,912 | Average Mismatch:-2,003 |

Beispielhaft wird unter einer Sequenz, die eine Homologie von mindestens 50 % auf Nukleinsäurebasis mit der Sequenz SEQ ID NO: 1 aufweist, eine Sequenz verstanden, die bei einem Vergleich mit der Sequenz SEQ ID NO: 1 nach obigem Programmalgorithmus mit obigem Parametersatz eine Homologie von mindestens 50 % aufweist.

Unter Homologie zwischen zwei Polypeptiden wird die Identität der Aminosäuresequenz über die jeweilige Sequenzlänge verstanden, die durch Vergleich mit Hilfe des Programmalgorithmus GAP (Wisconsin Package Version 10.0, University of Wisconsin, Genetics Computer Group (GCG), Madison, USA) unter Einstellung folgender Parameter berechnet wird:

| | |
|---|---|
| Gap Weight: 8 | Length Weight: 2 |
| Average Match: 2,912 | Average Mismatch:-2,003 |

Beispielhaft wird unter einer Sequenz, die eine Homologie von mindestens 60 % auf Proteinbasis mit der Sequenz SEQ ID NO: 12 aufweist, eine Sequenz verstanden, die bei einem Vergleich mit der Sequenz SEQ ID NO: 12 nach obigem Programmalgorithmus mit obigem Parametersatz eine Homologie von mindestens 60 % aufweist.

Funktionelle Äquivalente meint ferner DNA Sequenzen, die unter Standardbedingungen mit einer der Nukleinsäuresequenzen kodierend für einen der Promotoren gemäß SEQ ID NO: 1 oder 2 oder der zu diesen komplementären Nukleinsäuresequenzen hybridisieren und die im wesentlichen gleichen Promotoreigenschaften haben.

Der Begriff der Standardhybridisierungsbedingungen ist breit zu verstehen und meint sowohl stringente als auch weniger stringente Hybridisierungsbedingungen. Solche Hybridisierungsbedingungen sind unter anderem bei Sambrook J, Fritsch EF, Maniatis T et al., in Molecular Cloning - A Laboratory Manual, 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6. beschrieben. Beispielhaft können die Bedingungen während des Waschschrittes ausgewählt sein aus dem Bereich von Bedingungen begrenzt von solchen mit geringer Stringenz (mit ungefähr 2X SSC bei 50°C) und solchen mit hoher Stringenz (mit ungefähr 0.2X SSC bei 50°C bevorzugt bei 65°C) (20X SSC: 0,3 M Natriumcitrat, 3 M NaCl, pH 7.0). Darüber hinaus kann die Temperatur während des Waschschrittes von niedrig stringenten Bedingungen bei Raumtemperatur, ungefähr 22°C, bis zu stärker stringenten Bedingungen bei ungefähr 65°C angehoben werden. Beide Parameter, Salzkonzentration und Temperatur, können gleichzeitig variiert werden, auch kann einer der beiden Parameter konstant gehalten und nur der andere variiert werden. Während der Hybridisierung können auch denaturierende Agenzien wie zum Beispiel Formamid oder SDS eingesetzt werden. In Gegenwart von 50 % Formamid wird die Hybridisierung bevorzugt bei 42°C ausgeführt. Einige beispielhafte Bedingungen für Hybridisierung und Waschschritt sind infolge gegeben:
(1) Hybridisierungsbedingungen mit zum Beispiel
   a) 4X SSC bei 65°C, oder
   b) 6X SSC, 0,5% SDS, 100 µg/ml denaturierte, fragmentierte Lachssperma-DNA bei 65°C, oder
   c) 4X SSC, 50% Formamid, bei 42°C, oder
   d) 2X oder 4X SSC bei 50°C (schwach stringente Bedingung), oder
   e) 2X oder 4X SSC, 30 bis 40% Formamid bei 42°C (schwach stringente Bedingung), oder
   f) 6x SSC bei 45°C, oder,
   g) 0,05 M Natriumphosphatpuffer pH 7,0, 2 mM EDTA, 1% BSA und 7% SDS.
(2) Waschschritte mit zum Beispiel
   a) 0,1X SSC bei 65°C, oder
   b) 0,1X SSC, 0,5% SDS bei 68°C, oder
   c) 0,1X SSC, 0,5% SDS, 50% Formamid bei 42°C, oder
   d) 0,2X SSC, 0,1% SDS bei 42°C, oder
   e) 2X SSC bei 65°C (schwach stringente Bedingung), oder
   f) 40 mM Natriumphosphatpuffer pH 7,0, 1% SDS, 2 mM EDTA.

Verfahren zur Herstellung erfindungsgemäßer funktioneller Äquivalente umfassen bevorzugt die Einführung von Mutationen in einen der Promotoren gemäß SEQ ID NO: 1 oder 2. Eine Mutagenese kann ungerichtet ("random") erfolgen, wobei die mutagenisierten Sequenzen anschließend bezüglich ihrer Eigenschaften nach einer "trial-and-error" Prozedur durchmustert werden. Besonders vorteilhafte Selektionskriterien umfassen beispielsweise die Höhe der resultierenden Expression der eingeführten Nukleinsäuresequenz in einem nicht-reproduktiven Blütengewebe.

Verfahren zur Mutagenisierung von Nukleinsäuresequenzen sind dem Fachmann bekannt und schließen beispielhaft die Verwendung von Oligonukleotiden mit einer oder mehr Mutationen im Vergleich zu der zu mutierenden Region ein (z.B. im Rahmen einer "siespecific mutagenesis"). Typischerweise kommen Primer mit ungefähr 15 bis ungefähr 75 Nukleotiden oder mehr zum Einsatz, wobei bevorzugt ca. 10 bis ca. 25 oder mehr Nukleotidreste an beiden Seiten der zu verändernden Sequenz lokalisiert sind. Details und Durchführung besagter Mutageneseverfahren sind dem Fachmann geläufig (Kunkel et al. (1987) Methods Enzymol 154:367-382; Tomic et al. (1990) Nucl Acids Res 12:1656; Upender et al. (1995) Biotechniques 18(1):29-30; US 4,237,224). Eine Mutagenese kann auch durch Behandlung von beispielsweise transgenen Expressionsvektoren, die eine der erfindungsgemäßen Nukleinsäuresequenzen enthalten, mit mutagenisierenden Agentien wie Hydroxylamin realisiert werden.

Alternativ können nicht-essentielle Sequenzen eines erfindungsgemäßen Promotors deletiert werden, ohne die genannten wesentlichen Eigenschaften signifikant zu beeinträchtigen. Derartige Deletionsvarianten stellen funktionell äquivalente Fragmente zu den Promotoren beschrieben durch SEQ ID NO: 1 oder 2 oder zu funktionellen Äquivalentes derselben dar. Die Eingrenzung der Promotorsequenz auf bestimmte, essentielle regulatorische Regionen kann z.B. mit Hilfe von Suchroutine zur Suche von Promotorelementen vorgenommen werden. Oft sind in den für die Promotoraktivität relevanten Regionen bestimmte Promotorelemente gehäuft vorhanden. Diese Analyse kann beispielsweise mit Computerprogrammen wie dem Programm PLACE ("Plant Cis-acting Regulatory DNA Elements"; Higo K et al. (1999) Nucl Acids Res 27(1): 297-300), der BIOBASE Datenbank "Transfac" (Biologische Datenbanken GmbH, Braunschweig; Wingender E et al. (2001) Nucleic Acids Res 29(1):281-3) oder Datenbank PlantCARE (Lescot M et al. (2002) Nucleic Acids Res 30(1):325-7) vorgenommen werden.

Bevorzugt umfassen die funktionell äquivalenten Fragmente eines der erfindungsgemäßen Promotoren - beispielsweise der Promotoren beschrieben durch SEQ ID NO: 1 oder 2 - mindestens 200 Basenpaar, ganz besonders bevorzugt mindestens 500 Basenpaare, am meisten bevorzugt mindestens 1000 Basenpaare des 3'-Endes des jeweiligen erfindungsgemäßen Promotors - beispielsweise der Promotoren beschrieben durch SEQ ID NO: 1 oder 2 -, wobei die Länge vom Transkriptionsstart ("ATG"-Kodon) in 5'-Richtung stromaufwärts gerechnet wird. Ganz besonders bevorzugte funktionell äuivalente Fragmente sind die Promotorsequenzen beschrieben durch SEQ ID NO: 3 oder 4. Weitere funktionell äquivalente Fragmente können beispielsweise durch Deletion eventuell noch vorhandener 5'-untranslatierter Bereiche erzeugt werden. Zu diesem Zweck kann der Transkriptionsstart der entsprechenden Gene durch dem Fachmann geläufige Verfahren (wie beispielsweise 5'-RACE) bestimmt und die 5'-untranslatierten durch PCR-vermittelte Methoden oder Endonukleaseverdau deletiert werden.

In erfindungsgemäßen transgenen Expressionskassetten steht mindestens einer der erfindungsgemäßen Promotoren (z.B. beschrieben durch SEQ ID NO: 1, 2, 3 oder 4) in funtioneller Verknüpfung mit mindestens einer transgen zu exprimierenden Nukleinsäuresequenz.

Unter einer funktionellen Verknüpfung versteht man zum Beispiel die sequentielle Anordnung eines der erfindungsgemäßen Promotoren (z.B. beschrieben durch SEQ ID NO: 1, 2, 3 oder 4) mit einer transgen zu exprimierenden Nukleinsäuresequenz und ggf. weiterer genetischer Kontrollsequenzen wie zum Beispiel einem Terminator oder einer Polyadenylierungssequenz derart, dass der Promotor seine Funktion bei der transgenen Expression der Nukleinsäuresequenz unter geeigneten Bedingungen erfüllen kann und die Expression der Nukleinsäuresequenz (d.h. Transkription und gegebenenfalls Translation) erfolgt. "Geeignete Bedingungen" meint dabei bevorzugt das Vorliegen der Expressionskassette in einer pflanzlichen Zelle, bevorzugt einer pflanzlichen Zelle umfasst von einem nicht-reproduktiven Blütengewebe einer Pflanze.

Bevorzugt sind Anordnungen, in denen die transgen zu exprimierende Nukleinsäuresequenz hinter einem der erfinduasggemäßen Pomotoren (z.B. beschrieben durch SEQ ID NO: 1, 2, 3 oder 4) positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Die Herstellung einer funktionellen Verknüpfung als auch die Herstellung eines trans genen Expressionskonstruktes kann mittels gängiger Rekombinations- und Klonierungstechniken realisiert werden, wie sie beispielsweise in Maniatis T, Fritsch EF und Sambrook J (1989) Molecular Cloning: A Laboratory Manual, Cold Spring Harbor Laboratory, Cold Spring Harbor (NY), in Silhavy TJ, Berman ML und Enquist LW (1984) Experiments with Gene Fusions, Cold Spring Harbor Laboratory, Cold Spring Harbor (NY) und in Ausubel FM et al. (1987) Current Protocols in Molecular Biology, Greene Publishing Assoc. and Wiley Interscience beschrieben sind. Zwischen beide Sequenzen können aber auch weitere Sequenzen positioniert werden, die zum Beispiel die Funktion eines Linkers mit bestimmten Restriktionsenzymschnittstellen oder eines Signalpeptides haben. Auch kann die Insertion von Sequenzen zur Expression von Fusionsproteinen führen. Bevorzugt kann das transgene Expressionskonstrukt, bestehend aus einer Verknüpfung von Promoter und zu exprimierender Nukleinsäuresequenz, integriert in einem Vektor vorliegen und durch zum Beispiel Transformation in ein pflanzliches Genom insertiert werden.

Unter einer Expressionskassette sind aber auch solche Konstruktionen zu verstehen, bei denen einer der erfindungsgemäßen Promotoren (z.B. beschrieben durch SEQ ID NO: 1, 2, 3 oder 4), ohne dass er zuvor notwendigerweise mit einer zu exprimierenden Nukleinsäuresequenz funktionell verknüpft wurde, zum Beispiel über eine gezielte homologe Rekombination oder eine zufällige Insertion in ein Wirtsgenom eingeführt wird, dort regulatorische Kontrolle über mit ihm dann funktionell verknüpfte endogene Nukleinsäuresequenzen übernimmt und die transgene Expression derselben steuert. Durch Insertion des Promotors - zum Beispiel durch eine homologe Rekombination - vor eine für ein bestimmtes Polypeptid kodierende Nukleinsäure erhält man eine erfindungsgemässe Expressionskassette, die die Expression des bestimmten Polypeptides selektiv in den nicht-reproduktiven Gweben der Blüte steuert. Auch kann beispielsweise der natürliche Promotor eines endogenen Gens gegen einen der erfindungsgemäßen Promotoren (z.B. beschrieben durch SEQ ID NO: 1, 2, 3 oder 4) ausgetauscht und so das Expressionsverhalten des endogenen Gens modifiziert werden.

Ferner kann die Insertion des Promotors auch derart erfolgen, dass antisense-RNA zu der für ein bestimmtes Polypeptid kodierenden Nukleinsäure exprimiert wird. Damit wird selektiv die Expression des bestimmten Polypeptides in den nicht reproduktiven Organen der Blüte herrunterreguliert oder ausgeschaltet.

Analog kann auch eine transgen zu exprimierende Nukleinsäuresequenz - zum Beispiel durch eine homologe Rekombination - hinter die Sequenz kodierend für einen der erfindungsgemäßen Promotoren (z.B. beschrieben durch SEQ ID NO: 1, 2, 3 oder 4), die sich in ihrem natürlichen chromosomalen Kontext befindet, so plaziert werden, dass man eine erfindungsgemässe Expressionskassette erhält, die die Expression der transgen zu exprimierenden Nukleinsäureseuquenz in den nicht-reproduktiven Blütengeweben steuert.

Die erfindungsgemäßen transgenen Expressionskassetten können weitere genetische Kontrollsequenzen umfassen. Der Begriff der genetischen Kontrollsequenzen ist breit zu verstehen und meint all solche Sequenzen, die einen Einfluss auf das Zustandekommen oder die Funktion einer erfindungsgemäßen transgenen Expressionskassette haben. Genetische Kontrollsequenzen modifizieren zum Beispiel die Transkription und Translation in prokaryotischen oder eukaryotischen Organismen. Vorzugsweise umfassen die erfindungsgemäßen transgenen Expressionskassetten 3'-stromabwärts von der jeweiligen transgen zu exprimierenden Nukleinsäuresequenz eine Terminatorsequenz als zusätzliche genetische Kontrollsequenz, sowie gegebenenfalls weitere übliche regulative Elemente, und zwar jeweils funktionell verknüpft mit der transgen zu exprimierenden Nukleinsäuresequenz.

Genetische Kontrollsequenzen umfassen auch weitere Promotoren, Promotorelemente oder Minimalpromotoren, die die expressionssteuernden Eigenschaften modifizieren können. So kann durch genetische Kontrollsequenzen zum Beispiel die gewebespezifische Expression zusätzlich abhängig von bestimmten Stressfaktoren erfolgen. Entsprechende Elemente sind zum Beispiel für Wasserstress, Abscisinsäure (Lam E und Chua NH, J Biol Chem 1991; 266(26):17131-17135) und Hitzestress (Schoffl F et al. (1989) Mol Gen Genetics 217(2-3):246-53) beschrieben.

Es können ferner weitere Promotoren funktionell mit der zu exprimierenden Nukleinsäuresequenz verknüpft sein, die eine transgene Expression in weiteren Pflanzengeweben oder in anderen Organismen, wie zum Beispiel *E.coli* Bakterien ermöglichen. Als Promotoren kommen im Prinzip alle pflanzenspezifischen Promotoren in Frage. Pflanzenspezifische Promotoren meint grundsätzlich jeden Promotor, der die Expression von Genen, insbesondere Fremdgenen, in Pflanzen oder Pflanzenteilen, -zellen, -geweben, -kulturen steuern kann. Dabei kann die Expression beispielsweise konstitutiv, induzierbar oder entwicklungsabhängig sein. Bevorzugt sind konstitutive Promotoren, gewebespezifische Promotoren, entwicklungsabhängige Promotoren, chemisch-induzierbare stress-induzierbare oder pathogen-induzierbare Promotoren. Entsprechende Promotoren sind dem Fachmann allgemein bekannt.

Weitere vorteilhafte Kontrollsequenzen sind beispielsweise in den Promotoren gram-positiver Bakterien wie amy und SPO2 oder in den Hefe- oder Pilzpromotoren ADC1, MFa, AC, P-60, CYC1, GAPDH, TEF, rp28, ADH zu finden.

Prinzipiell können alle natürlichen Promotoren mit ihren Regulationssequenzen wie die oben genannten für das erfindungsgemäße Verfahren verwendet werden. Darüberhinaus können auch synthetische Promotoren vorteilhaft verwendet werden.

Genetische Kontrollsequenzen umfassen ferner auch die 5'-untranslatierte Regionen, Introns oder nichtkodierende 3'-Region von Genen wie beipielsweise das Actin-1 Intron, oder die Adh1-S Introns 1, 2 und 6 (allgemein: The Maize Handbook, Chapter 116, Freeling and Walbot, Eds., Springer, New York (1994)), bevorzugt der Gene mit dem Genlocus At3g01980 und At1g63140 aus Arabidopsis thaliana. Es kann gezeigt werden, dass derartige Regionen eine signifikante Funktion bei der Regulation der Genexpression spielen können. So wurde gezeigt, dass 5'-untranslatierte Sequenzen die transiente Expression heterologer Gene verstärken können. Beispielhaft für Translationsverstärker sei die 5'-Leadersequenz aus dem Tabak-Mosaik-Virus zu nennen (Gallie et al. (1987) Nucl Acids Res 15:8693-8711) und dergleichen. Sie können ferner die Gewebsspezifität fördern (Rouster J et al. (1998) Plant J 15:435-440). Die unter SEQ ID NO: 1, 2, 3 oder 4 angegebenen Nukleinsäuresequenzen repräsentieren jeweils die Promotorregion und die 5'-untranslatierte Regionen bis vor das ATG-Startcodon der jeweiligen Gene mit dem Genlocus At3g01980 und At1g63140.

Das transgene Expressionskonstrukt kann vorteilhafterweise eine oder mehrere sogenannte "enhancer Sequenzen" funktionell verknüpft mit dem Promoter enthalten, die eine erhöhte transgene Expression der Nukleinsäuresequenz ermöglichen. Auch am 3'-Ende der transgen zu exprimierenden Nukleinsäuresequenzen können zusätzliche vorteilhafte Sequenzen inseriert werden, wie weitere regulatorische Elemente oder Terminatoren. Die transgen zu exprimierenden Nukleinsäuresequenzen können in einer oder mehreren Kopien im Genkonstrukt enthalten sein.

Als Kontrollsequenzen geeignete Polyadenylierungssignale sind pflanzliche Polyadenylierungssignale, vorzugsweise solche, die im wesentlichen T-DNA Polyadenylierungssignale aus *Agrobakterium tumefaciens*. Beispiele für besonders geeignete Terminatorsequenzen sind der OCS (Octopin-Synthase)-Terminator und der NOS (Nopalin-Synthase)-Terminator.

Als Kontrollsequenzen sind weiterhin solche zu verstehen, die eine homologe Rekombination bzw. Insertion in das Genom eines Wirtsorganismus ermöglichen oder die Entfernung aus dem Genom erlauben. Bei der homologen Rekombination kann zum Beispiel die kodierende Sequenz eines bestimmten endogenen Gens gegen die für eine dsRNA kodierende Sequenz gezielt ausgetauscht werden. Methoden wie die cre/lox-Technologie erlauben eine gewebespezifische, unter Umständen induzierbare Entfernung des transgenen Expressionskonstruktes aus dem Genom des Wirtsorganismus (Sauer B (1998) Methods 14(4):381-92). Hier werden bestimmte flankierende Sequenzen dem Zielgen angefügt (lox-Sequenzen), die später eine Entfernung mittels der cre-Rekombinase ermöglichen.

Eine transgene Expressionskassette und/oder die von ihm abgeleiteten transgenen Expressionsvektoren können weitere Funktionselemente enthalten. Der Begriff Funktionselement ist breit zu verstehen und meint all solche Elemente, die einen Einfluss auf Herstellung, Vermehrung oder Funktion der erfindungsgemäßen transgenen Expressionskonstrukte, der transgenen Expressionsvektoren oder der transgenen Organismen haben. Beispielhaft aber nicht einschränkend seien zu nennen:
a) Selektionsmarker, die eine Resistenz gegen Biozide wie Metabolismusinhibitoren (z.B. 2-Desoxyglucose-6-phosphat; WO 98/45456), Antibiotika (z.B. Kanamycin, G 418, Bleomycin, Hygromycin) oder - bevorzugt - Herbizide (z.B. Phosphinotricin) verleihen. Als Selektionsmarker seien beispielhaft genannt: Phosphinothricinacetyltransferasen (bar und pat Gen), welche Glutaminsynthaseinhibitoren inaktivieren, 5-Enolpyruvylshikimat-3-phosphatsynthasen (EPSP Synthasegene), die eine Resistenz gegen Glyphosat^{®} (N-(phosphonomethyl)glycin) verleihen, Glyphosat^{®} degradierende Enzyme (gox-Genprodukt; Glyphosatoxidoreduktase), Dehalogenasen, welche z.B. Dalapon inaktivieren (deh Genprodukt), Sulfonylurea- und Imidazolinon inaktivierende Acetolactatsynthasen sowie Nitrilasen, welche z.B. Bromoxynil degradieren (bxn Genprodukt), das aasa-Genprodukt, das eine Resistenz gegen das Antibiotikum Apectinomycin verleih, Streptomycinphosphotransferasen (SPT), die eine Resistenz gegen Streptomycin gewähren, Neomycinphosphotransferasen (NPTII), die eine Resistenz gegen Kanamycin oder Geneticidin verleihen, das Hygromycinphosphotransferasen (HPT), die eine Resistenz gegen Hygromycin vermitteln, das Acetolactatsynthasen (ALS), die eine Resistenz gegen Sulfonylharnstoff-Herbizide verleihen (z.B. mutierte ALS-Varianten mit z.B. der S4 und/oder Hra Mutation).
b) Reportergene, die für leicht quantitizierbare Proteine kodieren und über Eigenfarbe oder Enzymaktivität eine Bewertung der Transformationseffizienz oder des Expressionsortes oder -zeitpunktes gewährleisten. Ganz besonders bevorzugt sind dabei Reporter-Proteine (Schenborn E, Groskreutz D. Mol Biotechnol. 1999; 13(1):29-44) wie das "green fluorescence protein" (GFP) (Sheen et al.(1995) Plant Journal 8(5):777-784), die Chloramphenicoltransferase, eine Luziferase (Ow et al. (1986) Science 234:856-859), das Aequorin-Gen (Prasher et al. (1985) Biochem Biophys Res Commun 126(3):1259-1268), die β-Galactosidase, ganz besonders bevorzugt ist die β-Glucuronidase (Jefferson et al. (1987) EMBO J 6:3901-3907).
c) Replikationsursprünge, die eine Vermehrung der erfindungsgemäßen transgenen Expressionskonstrukte oder transgenen Expressionsvektoren in zum Beispiel E.coli gewährleisten. Beispielhaft seien genannt ORI (origin of DNA replication), der pBR322 ori oder der P15A ori (Sambrook et al.: Molecular Cloning. A Laboratory Manual, 2nd ed. Cold Spring Harbor Laboratory Press, Cold Spring Harbor, NY, 1989).
d) Elemente, die für eine Agrobakterium vermittelte Pflanzentransformation erforderlich sind, wie zum Beispiel die rechte oder linke Begrenzung der T-DNA oder die vir-Region.

"Einführen" umfasst im Rahmen der Erfindung alle Verfahren, die dazu geeignet sind, eine Nukleinsäuresequenz (beispielsweise eine erfindungsgemäße Expressionskassette) direkt oder indirekt, in einen Organismus (z.B. ein Pflanze) oder eine Zelle, Kompartiment, Gewebe, Organ oder Vermehrungsmaterial (z.B. Samen oder FRüchte) derselben einzuführen oder dort zu generieren. Direkte und indirekte Verfahren sind umfasst. Das Einbringen kann zu einer vorübergehenden (transienten) Präsenz besagter Nukleinsäuresequenz führen oder aber auch zu einer dauerhaften (stabilen). Einführen umfasst beispielsweise Verfahren wie Transfektion, Transduktion oder Transformation. Die in den Verfahren verwendeten Organismen werden je nach Wirtsorganismus in dem Fachmann bekannter Weise angezogen bzw. gezüchtet.

Das Einführen einer erfindungsgemässen transgenen Expressionskassette in einen Organismus oder Zellen, Geweben, Organe, Teile bzw. Samen desselben (bevorzugt in Pflanzen bzw. pflanzliche Zellen, Gewebe, Organe, Teile oder Samen) kann vorteilhaft unter Verwendung von Vektoren realisiert werden, in denen die transgenen Expressionskassetten enthalten sind. Vektoren können beispielhaft Plasmide, Cosmide, Phagen, Viren oder auch Agrobakterien sein. Die transgenen Expressionskassetten können in den Vektor (bevorzugt ein Plasmidvektor) über eine geeignete Restriktionsschnittstelle insertiert werden. Der entstandene Vektor kann zunächst in E.coli eingeführt und amplifiziert werden. Korrekt transformierte E.coli werden selektioniert, gezüchtet und der rekombinante Vektor mit dem Fachmann geläufigen Methoden gewonnen. Restriktionsanalyse und Sequenzierung können dazu dienen, den Klonierungsschritt zu überprüfen. Bevorzugt sind solche Vektoren, die eine stabile Integration der Expressionskassette in das Wirtsgenom ermöglichen.

Die Herstellung eines transformierten Organismus (bzw. einer transformierten Zelle oder Gewebes) erfordert, dass die entsprechende DNA (z.B. der Expressionsvektor) oder RNA in die entsprechende Wirtszelle eingebracht wird. Für diesen Vorgang, der als Transformation (oder Transduktion bzw. Transfektion) bezeichnet wird, steht eine Vielzahl von Methoden zur Verfügung (Keown et al. (1990) Methods in Enzymology 185:527-537). So kann die DNA oder RNA beispielhaft direkt durch Mikroinjektion oder durch Bombardierung mit DNA-beschichteten Mikropartikeln eingeführt werden. Auch kann die Zelle chemisch, zum Beispiel mit Polyethylenglycol, permeabilisiert werden, so dass die DNA durch Diffusion in die Zelle gelangen kann. Die DNA kann auch durch Protoplastenfusion mit anderen DNA-enthaltenden Einheiten wie Minicells, Zellen, Lysosomen oder Liposomen erfolgen. Elektroporation ist eine weitere geeignete Methode zum Einführen von DNA, bei der die Zellen reversibel durch einen elektrischen Impuls permeabilisert werden. Entsprechende Verfahren sind beschrieben (beispielsweise bei Bilang et al. (1991) Gene 100:247-250; Scheid et al. (1991) Mol Gen Genet 228:104-112; Guerche et al. (1987) Plant Science 52:111-116; Neuhause et al. (1987) Theor Appl Genet 75:30-36; Klein et al. (1987) Nature 327:70-73; Howell et al. (1980) Science 208:1265; Horsch et al. (1985) Science 227:1229-1231; DeBlock et al. (1989) Plant Physiology 91:694-701; Methods for Plant Molecular Biology (Weissbach and Weissbach, eds.) Academic Press Inc. (1988); and Methods in Plant Molecular Biology (Schuler and Zielinski, eds.) Academic Press Inc. (1989)).

Als Vektoren zur Expression in E.coli sind bevorzugt pQE70, pQE60 und pQE-9 (QIAGEN, Inc.); pBluescript Vektoren, Phagescript Vektoren, pNH8A, pNH16a, pNH18A, pNH46A (Stratagene Cloning Systems, Inc.); ptrc99a, pKK223-3, pKK233-3, pDR540, pRIT5 (Pharmacia Biotech, Inc.).

Bevorzugte Vektoren zur Expression in Säugerzellen umfassen pWLNE0, pSV2CAT, pOG44, pXT1 und pSG (Stratagene Inc.); pSVK3, pBPV, pMSG und pSVL (Pharmacia Biotech, Inc.). Als. induzierbare Vektoren seien pTet-tTak, pTet-Splice, pcDNA4/TO, pcDNA4/TO / LacZ, pcDNA6/TR, pcDNA4/TO/Myc-His /LacZ, pcDNA4/TO/Myc-His A, pcDNA4/TO/Myc-His B, pcDNA4/TO/Myc-His C, pVgRXR (Invitrogen, Inc.) oder die pMAM-Serie (Clontech, Inc.; GenBank Accession No.: U02443) zunennen. Diese stellen bereits das induzierbare regulatorische Kontrollelement beispielsweise für eine chemisch, induzierbare Expression zur Verfügung.

Vektoren für die Expression in Hefe umfassen beispielhaft pYES2, pYD1, pTEFl/Zeo, pYES2/GS, pPICZ, pGAPZ, pGAPZalph, pPIC9, pPIC3.5, PHIL-D2, PHIL-Sl, pPIC3SK, pPIC9K, und PA0815 (Invitrogen, Inc.).

Klonierungsvektoren und Techniken zur genetischen Manipulation von Ciliaten und Algen sind dem Fachmann bekannt (WO 98/01572; Falciatore et al. (1999) Marine Biotechnology 1(3):239-251; Dunahay et al. (1995) J Phycol 31:10004-1012).

Prinzipiell sind für die Transformation tierischer Zellen oder von Hefezellen ähnliche Verfahren wie für die "direkte" Tranformation von pflanzlichen Zellen anzuwenden. Insbesondere Verfahren wie die Calciumphosphat oder Liposomen vermittelte Transformation oder aber Elektroporation sind bevorzugt.

Verschiedene Methoden und Vektoren zum Einschleusen von Genen in das Genom von Pflanzen sowie zur Regeneration von Pflanzen aus Pflanzengeweben oder Pflanzenzellen sind bekannt (Plant Molecular Biology and Biotechnology (CRC Press, Boca Raton, Florida), Kapitel 6/7, S. 71-119 (1993); White FF (1993) Vectors for Gene Transfer in Higher Plants; in: Transgenic Plants, Bd. 1, Engineering and Utilization, Hrsgb.: Kung und Wu R, Academic Press, 15-38; Jenes B et al. (1993) Techniques for Gene Transfer, in: Transgenic Plants, Bd. 1, Engineering and Utilization, Hrsgb.: Kung und R. Wu, Academic Press, S.128-143; Potrykus (1991) Annu Rev Plant Physiol Plant Molec Biol 42:205-225; Halford NG, Shewry PR (2000) Br Med Bull 56(1):62-73). Dazu zählen beispielhaft die oben erwähnten. Bei Pflanzen werden dabei die beschriebenen Methoden zur Transformation und Regeneration von Pflanzen aus Pflanzengeweben oder Pflanzenzellen zur transienten oder stabilen Transformation genutzt. Geeignete Methoden sind vor allem die Protoplastentransformation durch Polyethylenglykol-induzierte DNA-Aufnahme, Calciumphosphat-vermittelte Transformation, DEAE-Dextran-vermittelte Transformation, Liposomen vermittelte Transformation (Freeman et al. (1984) Plant Cell Physiol. 29:1353ff; US 4,536,475), biolistische Verfahren mit der Genkanone ("particle bombardment" Methode; US 5,100,792; EP-A 0 444 882; EP-A 0 434 616; Fromm ME et al. (1990) Bio/Technology 8(9):833-9; Gordon-Kamm et al. (1990) Plant Cell 2:603), die Elektroporation, die Inkubation trockener Embryonen in DNA-haltiger Lösung, Elektroporation (EP-A 290 395, WO 87/06614), Mikroinjektion (WO 92/09696, WO 94/00583, EP-A 0 331 083, EP-A 0 175 966) oder andere Methoden der direkten DNA-Einführung (DE 4 005 152, WO 90/12096, US 4,684,611). Physikalische Methoden der DNA-Einführung in pflanzliche Zellen sind im Überblick dargestellt bei Oard (1991) Biotech Adv 9:1-11.

Im Falle dieser "direkten" Transformationsmethoden sind keine besonderen Anforderungen an das verwendete Plasmid gestellt. Einfache Plasmide wie die der pUC-Reihe, pBR322, M13mp Reihe, pACYC184 etc. können verwendet werden. Sollen vollständige Pflanzen aus den transformierten Zellen regeneriert werden, so ist es erforderlich, dass sich auf dem Plasmid ein zusätzliches selektionierbares Markergen befindet.

Neben diesen "direkten" Transformationstechniken kann eine Transformation auch durch bakterielle Infektion mittels Agrobakterium (z.B. EP 0 116 718), virale Infektion mittels viraler Vektoren (EP 0 067 553; US 4,407,956; WO 95/34668; WO 93/03161) oder mittels Pollen (EP 0 270 356; WO 85/01856; US 4,684,611) durchgeführt werden.

Bevorzugt erfolgt die Transformation mittels Agrobakterien, die "entwaffnete" (disarmed) Ti-Plasmidvektoren enthalten, wobei deren natürliche Fährigkeit zum Gentransfer auf Pflanzen genutzt wird (EP-A 0 270 355; EP-A 0 116 718).

Agrobakterium-Transformation ist weit verbreitet für die Transformation von Dicotyledonen, wird aber auch zunehmend auf Monocotyledonen angewandt (Toriyama et al. (1988) Bio/Technology 6: 1072-1074; Zhang et al. (1988) Plant Cell Rep 7:379-384; Zhang et al. (1988) Theor Appl Genet 76:835-840; Shimamoto et al. (1989) Nature 338:274-276; Datta et al. (1990) Bio/Technology 8: 736-740; Christou et al. (1991) Bio/Technology 9:957-962; Peng et al. (1991) International Rice Research Institute, Manila, Philippines 563-574; Cao et al. (1992) Plant Cell Rep 11:585-591; Li et al. (1993) Plant Cell Rep 12:250-255; Rathore et al. (1993) Plant Mol Biol 21:871-884; Fromm et al. (1990) Bio/Technology 8:833-839; Gordon-Kamm et al. (1990) Plant Cell 2:603-618; D'Halluin et al. (1992) Plant Cell 4:1495-1505; Walters et al. (1992) Plant Mol Biol 18:189-200; Koziel et al. (1993) Biotechnology 11:194-200; Vasil IK (1994) Plant Mol Biol 25:925-937; Weeks et al. (1993) Plant Physiol 102:1077-1084; Somers et al. (1992) Bio/Technology 10:1589-1594; WO 92/14828; Hiei et al. (1994) Plant J 6:271-282).

Die für die Agrobakterium-Transformation meist verwendeten Stämme Agrobakterium tumefaciens oder Agrobakterium rhizogenes enthalten ein Plasmid (Ti bzw. Ri Plasmid), das auf die Pflanze nach Agrobakterium-Infektion übertragen wird. Ein Teil dieses Plasmids, genannt T-DNA (transferred DNA), wird in das Genom der Pflanzenzelle integriert. Alternativ können durch Agrobakterium auch binäre Vektoren (Mini-Ti-Plasmide) auf Pflanzen übertragen und in deren Genom integriert werden.

Die Anwendung von Agrobakterium tumefaciens für die Transformation von Pflanzen unter Verwendung von Gewebekulturexplantaten ist beschrieben (u.a. Horsch RB et al. (1985) Science 225:1229ff.; Fraley et al. (1983) Proc Natl Acad Sci USA 80: 4803-4807; Bevans et al. (1983) Nature 304:184-187). Viele Stämme von Agrobakterium tumefaciens sind in der Lage, genetisches Material - beispielsweise die erfindungsgemäßen Expressionskassetten - zu übertragen, wie z.B. die Stämme EHA101[pEHA101], EHA105[pEHA105], LBA4404[pAL4404], C58C1[pMP90] und C58C1[pGV2260] (Hood et al. (1993) Transgenic Res 2:208-218; Hoekema et al. (1983) Nature 303:179-181; Koncz and Schell (1986) Gen Genet 204:383-396; Deblaere et al. (1985) Nucl Acids Res 13: 4777-4788).

Werden Agrobakterien verwendet, so ist die Expressionskassette in spezielle Plasmide zu integrieren, entweder in einen Zwischenvektor (englisch: shuttle or intermediate vector) oder einen binären Vektor. Bevorzugt werden binäre Vektoren verwendet, die sowohl in E.coli als auch in Agrobakterium replizieren können. Sie enthalten in der Regel ein Selektionsmarkergen und einen Linker oder Polylinker, flankiert von der rechten und linken T-DNA Begrenzungssequenz. Sie können direkt in Agrobakterium transformiert werden (Holsters et al. (1978) Mol Gen Genet 163:181-187). Das in diesem Fall als Wirtsorganismus fungierende Agrobakterium sollte bereits ein Plasmid mit der vir-Region enthalten. Diese ist für die Übertragung der T-DNA auf die pflanzliche Zelle erforderlich. Ein so transformiertes Agrobakterium kann zur Transformation pflanzlicher Zellen verwendet werden. Die Verwendung von T-DNA zur Transformation pflanzlicher Zellen ist intensiv untersucht und beschrieben (EP-A 0 120 516; Hoekema, In: The Binary Plant Vector System, Offsetdrukkerij Kanters B.V., Alblasserdam, Chapter V; An et al. (1985) EMBO J 4:277-287). Verschiedene binäre Vektoren sind bekannt und teilweise kommerziell erhältlich wie zum Beispiel pBI101.2 oder pBIN19 (Clontech Laboratories, Inc. USA; Bevan et al.(1984) Nucl Acids Res 12:8711), pBinAR, pPZP200 oder pPTV.

Die mit einem solchen Vektor transformierten Agrobakterien können dann in bekannter Weise zur Transformation von Pflanzen, insbesondere von Kulturpflanzen, wie z.B. von Raps, verwendet werden, indem beispielsweise verwundete Blätter oder Blattstücke in einer Agrobakterienlösung gebadet und anschliessend in geeigneten Medien kultiviert werden. Die Transformation von Pflanzen durch Agrobakterien ist beschrieben (White FF (1993) Vectors for Gene Transfer in Higher Plants; in Transgenic Plants, Vol. 1, Engineering and Utilization, herausgegeben von SD Kung und R Wu, Academic Press, S. 15-38; Jenes B et al.(1993) Techniques for Gene Transfer, in: Transgenic Plants, Vol. 1, Engineering and Utilization, herausgegeben von S.D. Kung und R. Wu, Academic Press, S.128-143; Potrykus (1991) Annu Rev Plant Physiol Plant Molec Biol 42:205-225). Aus den transformierten Zellen der verwundeten Blätter bzw. Blattstücke können in bekannter Weise transgene Pflanzen regeneriert werden, die integriert die oben beschriebenen erfindungsgemässen Expressionssysteme enthalten.

Stabil transformierte Zellen (d.h. solche, die die eingeführte DNA integriert in die DNA der Wirtszelle enthalten) können von untransformierten selektioniert werden, wenn ein selektionierbarer Marker Bestandteil der eingeführten DNA ist. Als Marker kann beispielhaft jedes Gen fungieren, dass eine Resistenz gegen ein Biozid (z.B. ein Antibiotikum oder Herbizid (s.o.) zu verleihen vermag (s.o.). Transformierte Zellen, die ein solches Markergen exprimieren, sind in der Lage, in der Gegenwart von Konzentrationen eines entsprechenden Biozids zu überleben, die einen untransformierten Wildtyp abtöten. Der Selektionsmarker erlaubt die Selektion von transformierten Zellen von untransformierten (McCormick et al. (1986) Plant Gell Reports 5:81-84). Die erhaltenen Pflanzen können in üblicher Weise gezüchtet und gekreuzt werden. Zwei oder mehr Generationen sollten kultiviert werden, um sicherzustellen, dass die genomische Integration stabil und vererblich ist.

Sobald eine transformierte Pflanzenzelle hergestellt wurde, kann eine vollständige Pflanze unter Verwendung von dem Fachmann bekannten Verfahren erhalten werden. Hierbei geht man beispielhaft von Kalluskulturen, einzelnen Zellen (z.B. Protoplasten) oder Blattscheiben aus (Vasil et al. (1984) Cell Culture and Somatic Cel Genetics of Plants, Vol I, II and III, Laboratory Procedures and Their Applications, Academic Press; Weissbach and Weissbach (1989) Methods for Plant Molecular Biology, Academic Press). Aus diesen noch undifferenzierten Kallus-Zellmassen kann die Bildung von Spross und Wurzel in bekannter Weise induziert werden. Die erhaltenen Sprösslinge können ausgepflanzt und gezüchtet werden. Entsprechende Verfahren sind beschrieben (Fennell et al. (1992) Plant Cell Rep. 11: 567-570; Stoeger et al (1995) Plant Cell Rep. 14:273-278; Jahne et al. (1994) Theor Appl Genet 89:525-533).

Die Wirksamkeit der Expression der transgen exprimierten Nukleinsäuren kann beispielsweise in vitro durch Sprossmeristemvermehrung unter Verwendung einer der oben beschriebenen Selektionsmethoden ermittelt werden. Zudem kann eine in Art und Höhe veränderte Expression eines Zielgens und die Auswirkung auf den Phänptyp der Pflanze an Testpflanzen in Gewächshausversuchen getestet werden.

Ein weiterer Gegenstand der Erfindung betrifft transgene Organismen, transformiert mit wenigstens einer erfindungsgemässen Expressionskassette oder einem erfindungsgemässen Vektor, sowie Zellen, Zellkulturen, Gewebe, Teile - wie zum Beispiel bei pflanzlichen Organismen Blätter, Wurzeln usw.- oder Vermehrungsgut abgeleitet von solchen Organismen.

Unter Organismus, Ausgangs- oder Wirtsorganismen werden prokaryotische oder eukaryotische Mikroorganismen oder pflanzliche Organismen verstanden. Bevorzugte Mikroorganismen sind Bakterien, Hefen, Algen oder Pilze.

Bevorzugte Bakterien sind Bakterien der Gattung Escherichia, Erwinia, Agrobakterium, Flavobacterium, Alcaligenes, Pseudomonas, Bacillus oder Cyanobakterien zum Beispiel der Gattung Synechocystis und weitere in Brock Biology of Microorganisms Eighth Edition auf den Seiten A-8, A-9, A10 und A11 beschriebenen Bakteriengattungen.

Bevorzugt sind vor allem Mikroorganismen, welche zur Infektion von Pflanzen und damit zur Übertragung der erfindungsgemässen Konstrukte befähigt sind. Bevorzugte Mikroorganismus sind solche aus der Gattung Agrobakterium und insbesondere der Art Agrobakterium tumefaciens. Besonders bevorzugte Mikroorganismen sind solche, die zur Produktion von Toxinen (z.B. Botulinus Toxin), Pigmenten (z.B. Carotinoiden oder Flavonoiden), Antibiotika (z.B. Penicillin), Phenylpropanoiden (z.B. Tocopherol), Polyungesättigten Fettsäuren (z.B.Arachidonsäure) oder Vitaminen (z.B. Vitamin B12) befähigt sind.

Bevorzugte Hefen sind Candida, Saccharomyces, Hansenula oder Pichia.

Bevorzugte Pilze sind Aspergillus, Trichoderma, Ashbya, Neurospora, Fusarium, Beauveria oder weitere in Indian Chem Engr. Section B. Vol 37, No 1,2 (1995) auf Seite 15, Tabelle 6 beschriebene Pilze.

Als transgene Organismen bevorzugte Wirts- oder Ausgangsorganismen sind vor allem pflanzliche Organismen.

"Pflanzlicher Organismus oder von diesem abgeleitete Zellen" meint allgemein jede Zelle, Gewebe, Teile oder Vermehrungsgut (wie Samen oder Früchte) eines zur Photosynthese befähigten Organismus. Eingeschlossen sind im Rahmen der Erfindung alle Gattungen und Arten höherer und niederer Pflanzen des Pflanzenreiches. Einjährige, mehrjährige, monocotyledone und dicotyledone Pflanzen sind bevorzugt.

"Pflanze" im Rahmen der Erfindung meint alle Gattungen und Arten höherer und niederer Pflanzen des Pflanzenreiches. Eingeschlossen unter dem Begriff sind die reifen Pflanzen, Saatgut, Sprosse und Keimlinge, sowie davon abgeleitete Teile, Vermehrungsgut (zum Beispiel Knollen, Samen oder Früchte), Pflanzenorgane, Gewebe, Protoplasten, Kallus und andere Kulturen, zum Beispiel Zell- oder Kalluskulturen, sowie alle anderen Arten von Gruppierungen von Pflanzenzellen zu funktionellen oder strukturellen Einheiten. Reife Pflanzen meint Pflanzen zu jedem beliebigen Entwicklungsstadium jenseits des Keimlings. Keimling meint eine junge, unreife Pflanze in einem frühen Entwicklungsstadium.

Pflanzliche Organismen im Sinne der Erfindung sind weiterhin weitere photosynthetisch aktive Organismen, wie zum Beispiel Algen, Cyanobakterien sowie Moose. Bevorzugte Algen sind Grünalgen, wie beispielsweise Algen der Gattung Haematococcus, Phaedactylum tricornatum, Volvox oder Dunaliella. Insbesondere bevorzugt sind Synechocystis, Chlamydomonas und Scenedesmus.

Im Rahmen des erfindungesgemäßen Verfahrens sind insbesondere pflanzliche Organismen bevorzugt ausgewählt aus der Gruppe der Blütenpflanzen (Phylum Anthophyta "Angiospermen"). Umfasst sind alle einjährigen und mehrjährige, monokotyledonen und dikotyledonen Pflanzen. Bevorzugt ist die Pflanze aus nachfolgenden Pflanzenfamilien ausgewählt: Amaranthaceae, Asteraceae, Brassicaceae, Caryophyllaceae, Chenopodiaceae, Compositae, Cruciferae, Cucurbitaceae, Labiatae, Leguminosae, Papilionoideae, Liliaceae, Linaceae, Malvaceae, Rosaceae, Rubiaceae, Saxifragaceae, Scrophulariaceae, Solanacea, Sterculiaceae, Tetragoniacea, Theaceae und Umbelliferae.

Die Erfindung wird ganz besonders bevorzugt auf dikotyledone pflanzliche Organismen angewendet. Bevorzugte dikotyle Pflanzen sind insbesondere ausgewählt aus den dikotylen Kulturpflanzen, wie zum Beispiel den nachfolgenden
1) Kategorie: Dicotyledonae (Dicotyledonen). Bevorzugte Familien:
   - Aceraceae (Ahornhölzer)
   - Cactaceae (Kakteen)
   - Rosaceae (Rosen, Äpfel, Mandeln, Erdbeeren)
   - Salicaceae (Weiden)
   - Asteraceae (Compositae) besonders die Gattung Lactuca, ganz besonders die Art sativa (Salat), sowie Sonnenblume, Löwenzahn, Tagetes oder Calendula und andere mehr,
   - Cruciferae (Brassicaceae), besonders die Gattung Brassica, ganz besonders die Arten napus (Raps), campestris (Rübe), oleracea (z.B. Kohl, Blumenkohl oder Broccoli und weitere Kohlarten); und der Gattung Arabidopsis, ganz besonders die Art thaliana sowie Kresse, Rettich, Canola und andere mehr,
   - Cucurbitaceae wie Melone, Kürbis, Gurken oder Zucchini und andere mehr,
   - Leguminosae (Fabaceae) besonders die Gattung Glycine, ganz besonders die Art max (Sojabohne) Soja sowie Alfalfa, Erbse, Bohnengewächsen, Lupine oder Erdnuss und andere mehr,
   - Malvaceae insbesondere Malve, Baumwolle, eßbarer Eibisch, Hibiscus und andere mehr,
   - Rubiaceae, bevorzugt der Unterklasse Lamiidae wie beispielsweise Coffea arabica oder Coffea liberica (Kaffeestrauch) und andere mehr,
   - Solanaceae besonders die Gattung Lycopersicon, ganz besonders die Art esculentum (Tomate) und die Gattung Solanum, ganz besonders die Art tuberosum (Kartoffel) und melongena (Aubergine) und die Gattung Capsicum, ganz besonders die Art annum (Paprika), sowie Tabak, Petunie und andere mehr,
   - Sterculiaceae, bevorzugt der Unterklasse Dilleniidae wie beispielsweise Theobroma cacao (Kakaostrauch) und andere mehr,
   - Theaceae, bevorzugt der Unterklasse Dilleniidae wie beispielsweise Camellia sinensis oder Thea sinensis (Teestrauch) und andere mehr,
   - Umbelliferae (Apiaceae), besonders die Gattung Daucus (ganz besonders die Art carota (Karotte)), Apium (ganz besonders die Art graveolens dulce (Sellerie)) sowie Petersilie und andere mehr;
   sowie Lein, Hanf, Flachs, Spinat, Möhre, Zuckerrübe und den verschiedenen Baum-, Nuss- und Weinarten, insbesondere Banane und Kiwi.

Darüberhinaus sind jedoch auch monokotyle Pflanzen geeignet. Bevorzugt sind diese ausgewählt aus den monokotylen Kulturpflanzen, wie zum Beispiel den Familien
- Arecaceae (Palmen)
- Bromeliaceae (Ananas, spanisches Moos)
- Cyperaceae (Seggen)
- Liliaceae (Lillien, Tulpen, Hyazinthen, Zwiebel, Knoblauch)
- Orchidaceae (Orchideen)
- Poaceae (Gräser, Bambusse, Mais, Zuckerrohr, Weizen)
- Iridaceae (Blenden, Gladiolen, Krokusse)

Ganz besonders bevorzugt sind Gramineae wie Reis, Mais, Weizen oder andere Getreidearten wie Gerste, Hirse, Roggen, Triticale oder Hafer sowie dem Zuckerrohr sowie alle Arten von Gräsern.

Im Rahmen der erfindungsgemässen Expressionskassette kann die Expression einer bestimmten Nukleinsäure durch einen Promotor mit Spezifität für die nicht reproduktiven Organe der Blüte zu Bildung von sense-RNA, antisense RNA oder doppelsträngiger RNA in Form einer inversen Wiederholung (dsRNAi) führen. Die sense-RNA kann infolge in bestimmte Polypeptide translatiert werden. Mit der antisense-RNA und dsRNAi kann die Expression bestimmter Gene herunterreguliert werden.

Das Verfahren der Genregulation mittels doppelsträngiger RNA ("double-stranded RNA interference"; dsRNAi) ist vielfach in tierischen und pflanzlichen Organismen beschrieben (z.B. Matzke MA et al. (2000) Plant Mol Biol 43:401-415; Fire A et al (1998) Nature 391:806-811; WO 99/32619; WO 99/53050; WO 00/68374; WO 00/44914; WO 00/44895; WO 00/49035; WO 00/63364). Auf die in den angegebenen Zitaten beschriebenen Verfahren und Methoden wird ausdrücklich Bezug genommen.

Die Spezifität der erfindungsgemässen Expressionskonstrukte und Vektoren für pflanzliche Blüten ist besonders vorteilhaft. Die Blüte hat eine Funktion im Anlocken von Nutzinsekten durch Pigmenteinlagerung oder Synthese flüchtiger Chemikalien.

Oft sind die natürlichen Abwehrmechanismen der Pflanze zum Beispiel gegen Pathogene unzureichend. Die Einführung fremder Gene aus Pflanzen, Tieren, oder mikrobiellen Quellen kann die Abwehr verstärken. Beispiel sind der Schutz gegen Insektenfrass in Tabak durch Expression des Bacillus thuringiensis Endotoxin (Vaeck et al. (1987) Nature 328:33-37) oder der Schutz des Tabaks gegen Pilzbefall durch Expression einer Chitinase aus der Bohne (Broglie et al. (1991) Science 254:1194-1197).

Kälteeinbrüche in der Blütezeit führen jedes Jahr zu erheblichen Ernteverlusten. Eine gezielte Expression schützender Proteine gezielt in der Blüteperiode kann einen Schutz gewähren.

Für eine hohe Effizienz solcher gentechnischer Ansätze ist eine konzentrierte Expression der entsprechenden transgen zu exprimierenden Nukleinsäuresequenz vor allem in der äussersten Hülle der Blüte vorteilhaft. Eine konstitutive Expression in der gesamten Pflanze kann den Effekt zum Beispiel durch eine Verdünnung in Frage stellen oder das Wachstum der Pflanze bzw. die Qualität des Pflanzenproduktes beeinträchtigen. Außerdem kann es durch eine konstitutive Expression verstärkt zum Abschalten des Transgens kommen ("gene silencing").

Hierzu sind Promotoren mit Spezifität für die Blüte vorteilhaft. Dem Fachmann ist eine Vielzahl von Proteinen bekannt, deren rekombinante Expression in der Blüte vorteilhaft sind. Ferner sind dem Fachmann eine Vielzahl von Genen bekannt, durch deren Reprimierung oder Ausschaltung mittels Expression einer entsprechenden antisense-RNA ebenfalls vorteilhafte Effekte erreicht werden können. Beispielhaft jedoch nicht einschränkend für vorteilhafte Effekte seien zu nennen: Das Erzielen einer Resistenz gegen abiotische Stressfaktoren (Hitze, Kälte, Trockenheit, erhöhte Feuchtigkeit, Umweltgifte, UV-Strahlung) und biotische Stressfaktoren (Pathogene, Viren, Insekten und Krankheiten), die Verbesserung von Nahrungs- oder Futtereigenschaften, die Verbesserung der Wachstumsrate oder des Ertrages, das Erzielen einer längeren oder früheren Blütezeit, die Veränderung oder Verstärkung des Duftes oder der Farbgebung der Blüten. Für die in diesen Anwendungen einsetzbaren Nukleinsäuresequenzen oder Polypeptide seien beispielhaft, aber nicht einschränkend, zu nennen:
1. Verbesserter UV-Schutz der pflanzlichen Blüte durch Veränderung der Pigmentierung durch Expression bestimmer Polypeptide wie Enyzme oder Regulatoren der Flavonoidbiosynthese (z.B. Chalconsynthasen, Phenylalaninammoniumlyasen), der DNA-Reparatur (z.B. Photolyasen; Sakamoto A et al.(1998) DNA Seq 9(5-6):335-40), der Isoprenoidbiosynthese (z.B. Deoxyxylulose-5-phosphatsynthasen), der IPP-Synthese oder der Carotinoidbiosynthese (z.B. Phytoensynthasen, Phytoendesaturasen, Lycopincyclasen, Hydroxylasen oder Ketolasen). Bevorzugt sind Nukleinsäuren, die für die Chalconsynthase aus Arabidopsis thaliana (GenBank Acc.-No.: M2030B), die 6-4 Photolyase aus Arabidopsis thaliana (GenBank Acc.-No.:BAB00748) oder das Blaulicht-Photorezeptor/Photolyase-Homolog (PHH1) aus Arabidopsis thaliana (GenBank Acc.-No.: U62549) oder funktionelle Äquivalente derselben kodieren.
2. Verbesserter Schutz der pflanzlichen Blüte gegen abiotische Stressfaktoren wie Trockenheit, Hitze, oder Kälte zum Beispiel durch Überexpression von dem "antifreeze"-Polypeptiden (z.B. aus Myoxocephalus Scorpius; WO 00/00512), dem Arabidopsis thaliana Transkriptionsaktivator CBF1, Glutamatdehydrogenasen (WO 97/12983, WO 98/11240), einem späten Embryogenesegen (LEA) zum Beispiel aus Gerste (WO 97/13843), Calcium-abhängigen Proteinkinasegenen (WO 9B/26045), Calcineurinen (WO 99/05902), Farnesyltransferasen (WO 99/06580; Pei ZM et al. (1998) Science 282:287-290), Ferritin (Deak M et al. (1999) Nature Biotechnology 17:192-196), Oxalatoxidase (WO 99/04013; Dunwell JM (1998) Biotechnology and Genetic Engeneering Reviews 15:1-32), DREBlA-Faktor (dehydration response element B 1A; Kasuga M et al. (1999) Nature Biotechnology 17:276-286), Genen der Mannitol- oder Trehalosesynthese (z.B. Trehalosephosphatsynthasen; Trehalosephosphatphosphatasen, WO 97/42326); oder durch Inhibition von Genen wie der Trehalase (WO 97/50561). Besonders bevorzugt sind Nukleinsäuren, die für den transkriptionellen Aktivator CBF1 aus Arabidopsis thaliana (Gen-Bank Acc.-No.: U77378) oder das "antifreeze"-Protein" aus Myoxocephalus octodecemspinosus (GenBank Acc.-No.: AF306348) oder funktionelle Äquivalente derselben kodieren.
3. Erreichen einer Resistenz zum Beispiel gegen Pilze, Insekten, Nematoden und Krankheiten durch gezielte Absonderung oder Anreicherung bestimmter Metaboliten oder Proteine in der Blüte. Beispielhaft seien genannt Glucosinolate (Nematodenabwehr), Chitinasen oder Glucanasen und andere Enzyme, die die Zellwand von Parasiten zerstören, Ribosom-inaktivierende Proteine (RIPs) und andere Proteine der pflanzlichen Resistenz- und Stressreaktion, wie sie bei Verletzung oder mikrobiellen Befall von Pflanzen oder chemisch durch zum Beispiel Salicylsäure, Jasmonsäure oder Ethylen induziert werden, Lysozyme aus nicht-pflanzlichen Quellen wie zum Beispiel T4 Lysozym oder Lysozm aus verschiedenenen Säugern, insektizide Proteine wie Bacillus thuringiensis Endotoxin, α-Amylaseinhibitor oder Proteaseinhibitoren (cowpea Trypsininhibitor), Glucanasen, Lektine (z.B. Phytohemagglutinin, Schneeglöckchenlectin, Weizenkeimagglutinin), RNAsen oder Ribozyme. Besonders bevorzugt sind Nukleinsäuren, die für die chit42 Endochitinase aus Trichoderma harzianum (GenBank Acc.-No.: S78423) oder für das N-hydroxylierende, multifunktionelle Cytochrom P-450 (CYP79) aus Sorghum bicolor (GenBank Acc.-No.: U32624) oder funktionelle Äquivalente derselben kodieren.
4. Erreichen einer Insektenabwehr oder -anlockung zum Beispiel durch erhöhte Freisetzung flüchtiger Duft- oder Botenstoffe durch zum Beispiel Enzyme der Terpenbiosynthese.
5. Erreichen einer Speicherfähigkeit in Blütengeweben, die normalerweise keine Speicherproteine oder -lipide enthalten mit dem Ziel, den Ertrag an diesen Substanzen zu erhöhen, z.B. durch Expression einer Acetyl-CoA-Carboxylase oder von Enzymen zur Veresterung von Metaboliten. Bevorzugt sind Nukleinsäuren, die für die Acetyl-CoA Carboxylase (Accase) aus Medicago sativa (GenBank Acc.-No.: L25042) oder funktioneile Äquivalente derselben kodieren.
6. Expression von Transportproteinen, die die Aufnahme von Metaboliten, Nährstoffen oder Wasser in die Blüte verbessern und so das Blütenwachstum, die Metabolitenzusammensetzung oder den Ertrag optimieren, zum Beispiel durch Expression eines Aminosäuretransporters, der die Aufnahme von Aminosäuren beschleunigt, oder eines Monosaccharid-Transporters, der die Aufnahme von Zuckern fördert. Bevorzugt sind Nukleinsäuren, die für den kationische Aminosäure-Transporter aus Arabidopsis thaliana (GenBank Acc.-No.: X92657) oder für den Monosaccharid-Transporter aus Arabidopsis thaliana (Gen-Bank Acc. No.: AJ002399) oder funktionelle Äquivalente deselben kodieren.
7. Expression von Genen, die eine Akkumulation von Feinchemikalien, wie von Tocopherolen, Tocotrienolen, Phenylpropanoiden, Isoprenoiden oder Carotiniden, in der Blüte bewirken. Beispielhaft seien die Deoxyxylulose-5-phosphatsynthasen, Phytoensynthasen, Lycopin-β-cyklasen und die β-Carotinketolasen genannt. Bevorzugt sind Nukleinsäuren, die für die Haematoccus pluvialis NIES-144 (Acc. No. D45881) Ketolase oder funktionelle Äquivalente derselben kodieren.
8. Modifikation der Wachsesterbildung oder der Zusammensetzung der eingelagerten Oligosaccharide zur Verbesserung des Schutzes gegen Umwelteinflüsse oder zur Verbesserung der Verdaubarkeit beim Einsatz in Futter- oder Nahrungsmitteln. Beispielhaft sein die Überexpression der Endoxyloglucantransferase genannt. Bevorzug sind Nukleinsäuren, die für die Endo-xyloglucantransferase (EXGT-A1) aus Arabidopsis thaliana (Gen-Bank Acc.-No.:AF163819} oder funktionelle Äquivalente derselben kodieren.
9. Expression von Genen, DNA Bindeproteinen, dsRNA und antisense Konstruktionen, zur Veränderung der Blütenmorphologie, des Blühzeitpunktes und der Blütenseneszenz sowie des Blütenmetabolismus. Bevorzugt sind Konstruktionen, die die Anzahl der Petalen erhöhen z.B. durch Herunterregulation von AGAMOUS und dessen homologen Genen (Yanofsky MF et al. (1990) Nature 346:35-39) den Blühzeitpunkt verfrühen z.B. durch Herunterregulation von FLOWERING LOCUS C (FLC) (Tadege M et al. (2001) Plant J 28(5):545-53) oder verspäten z.B. durch Überexpression von FLC und die Seneszenz verzögern z.B. durch Vermittlung einer blütenspezifischen Ethyleninsensitivität.
10. Erzeugung von sterilen Pflanzen durch Verhinderung der Befruchtung und/oder der Keimung mit Hilfe der Expression eines geeigneten Inhibitors zum Beispiel eines Toxins in Blüten.
11. Produktion von Nutraceuticals wie zum Beispiel
   a) Carotinoide und/oder Phenylpropanoide z.B. durch Optimierung der blüteneigenen Stoffwechselwege z.B. durch Expression von Enzymen und Regulatoren der Isoprenoidbiosynthese. Bevorzugt sind Nukleinsäuren, die für die Chalconsynthase aus Arabidopsis thaliana (GenBank Acc.-No.: M20308), die 6-4 Photolyase aus Arabidopsis thaliana (GenBank Acc.No.:BAB00748) oder den Blaulicht-Photorezeptor / Photolyase Homolog (PHH1) aus Arabidopsis thaliana (GenBank Acc.-No.: U62549) oder funktionelle Äquivalente derselben kodieren. Ebenso bevorzugt sind Nukleinsäuren, die für Enzyme und Regulatoren der Isoprenoidbiosynthese wie die Deoxyxylulose-5-phosphatsynthasen und der Carotinoidbiosynthese wie die Phytoensynthasen, Lycopincyclasen und Ketolasen wie von Tocopherolen, Tocotrienolen, Phenylpropanoiden, Isoprenoiden oder Carotiniden, in der Blüte bewirken. Beispielhaft seien die Deoxyxylulose-5-phosphatsynthasen, Phytoensynthasen, Lycopincyclasen und die Carotinketolasen genannt. Besonders devorzugt sind Nukleinsäuren, die für die Haematoccus pluvialis, NIES-144 (Acc. No. D45881) Ketolase oder funktionelle Äquivalente kodieren.
   b) Polyungesättigte Fettsäuren wie beispielsweise Arachidonsäure oder EP (Eicosapentaensäure) oder DHA (Docosahexaensäure) durch Expression von Fettsäureelongasen und/oder - desaturasen oder Produktion von Proteinen mit verbessertem Nahrungswert wie zum Beispiel mit einem hohen Anteil an essentiellen Aminosäuren (z.B. das methioninreiche 2S Albumingens der Brasilnuss). Bevorzugt sind Nukleinsäuren, die für das methioninreiche 2S-Albumin aus Bertholletia excelsa (GenBank Acc.-No.:AB044391), die Δ6-Acyllipiddesaturase aus Physcomitrella patens (GenBank Acc.-No.: AJ222980; Girke et al. (1998) Plant J 15:39-48), die Δ6-Desaturase aus Mortierella alpina (Sakura-dani et al 1999 Gene 238:445-453), die Δ5-Desaturase aus Caenorhabditis elegans (Michaelson et al. (1998) FEBS Letters 439:215-218), die Δ5-Fettsäuredesaturase (des-5) aus Caenorhabditis elegans (GenBank Acc.-No.: AF078796), die Δ5-Desaturase aus Mortierella alpina (Michaelson et al. J Biol Chem 273:19055-19059), die Δ6-Elongase aus Caenorhabditis elegans (Beaudoin et al. (2000) Proc Natl. Acad. Sci. 97:6421-6426), die A6-Elongase aus Physcomitrella patens (Zank et al. (2000,) Biochemical Society Transactions 28:654-657) oder funktionelle Äquivalente derselben kodieren.
11. Produktion von Pharmazeutika, wie zum Beispiel Antikörpern, Vakzinen, Hormonen und/oder Antibiotika wie z.B. beschrieben bei Hood EE & Jilka JM (1999) Curr Opin Biotechnol 10(4):382-6; Ma JK & Vine ND (1999) CurrTop Microbiol Immunol 236:275-92.

Weitere Beispiele für vorteilhafte Gene sind zum Beispiel genannt bei Dunwell JM (2000) Transgenic approaches to crop improvement. J Exp Bot. 51 Spec No:487-96.

Ein weiterer Gegenstand der Erfindung betrifft die Verwendung der oben beschriebenen erfindungsgemässen, transgenen Organismen und der von ihnen abgeleitete Zellen, Zellkulturen, Teile - wie zum Beispiel bei transgenen pflanzlichen Organismen Wurzeln, Blätter etc.- , und transgenes Vermehrungsgut wie Saaten oder Früchte, zur Herstellung von Nahrungs- oder Futtermitteln, Pharmazeutika oder Feinchemikalien.

Bevorzugt ist ferner ein Verfahren zur rekombinanten Herstellung von Pharmazeutika oder Feinchemikalien in Wirtsorganismen, wobei ein Wirtsorganismus mit einer der oben beschriebenen Expressionskassetten transformiert wird und diese Expressionskassette ein oder mehrere Strukturgene enthält, die für die gewünschte Feinchemikalie kodieren oder deren Biosynthese katalysieren, der transformierte Wirtsorganismus gezüchtet wird und die gewünschte Feinchemikalie aus dem Züchtungsmedium isoliert wird. Dieses Verfahren ist für Feinchemikalien wie Enzyme, Vitamine, Aminosäuren, Zucker, Fettsäuren, natürliche und synthetische Geschmacks-, Aroma- und Farbstoffe breit anwendbar. Besonders bevorzugt ist die Produktion von Tocopherolen und Tocotrienolen sowie Carotinoiden wie beispielsweise Astaxanthin. Die Züchtung der transformierten Wirtsorganismen sowie die Isolierung aus den Wirtsorganismen bzw. aus dem Züchtungsmedium erfolgt mit dem Fachmann bekannten Verfahren. Die Produktion von Pharmazeutika, wie zum Beispiel Antikörpern oder Vakkzinen ist beschrieben bei Hood EE & Jilka JM (1999) Curr Opin Biotechnol 10 (4)382-6; Ma JK & Vine ND (1999) Curr Top Microbiol Immunol 236:275-92.

### Sequenzen

| | | |
|---|---|---|
| 1. | SEQ ID NO: 1 | 2051bp Fragment von Promotor (und ggf. 5'-untranslatierter Region des Arabidopsis thaliana Genlocus At3g01980 (76L-Promotor) |
| 2. | SEQ ID NO: 2 | 2192bp Fragment von Promotor (und ggf. 5'-untranslatierter Region des Arabidopsis thaliana Genlocus At1g63140 (84L-Promotor) |
| 3. | SEQ ID NO: 3 | Funktionell äquivalentes Fragment (1045 bp) von Promotor (und ggf. 5'-untranslatierter Region des Arabidopsis thaliana Genlocus At3g01980 (76S-Promotor) |
| 4. | SEQ ID NO: 4 | Funktionell äquivalentes Fragment (1109 bp) von Promotor (und ggf. 5'-untranslatierter Region des Arabidopsis thaliana Genlocus At1g63140 (84L-Promotor) |
| 5. | Seq ID No: 5 | Oligonukleotid-Primer 76sSmaI 5'-CCCGGGTGCCAAAGTAACTCTTTAT-3' |
| 6. | Seq ID No: 6 | Oligonukleotid-Primer 76assSalI 5'-GTCGACAGGTGCATGACCAAGTAAC-3' |
| 7. | Seq ID No: 7 | Oligonukleotid-Primer 76aslSalI 5'-GTCGACTATCCTCTGCGCAATGAAT-3' |
| 8. | Seq ID No: 8 | Oligonukleotid-Primer 84sSmaI 5'-CCCGGGAAATCGAGAAAGATAGGTA-3' |
| 9. | Seq ID No; 9 | Oligonukleotid-Primer 84assSalI 5'-GTCGACAAAGGGTTATAGGAGACTG-3' |
| 10. | Seq ID No: 10 | Oligonukleotid-Primer 84aslSalI 5'-GTCGACCATGTTTCAGAGGATATGT-3' |
| 11. | SEQ ID NO: 11 | Nukleinsäuresequenz (cDNA) kodierend für das Genprodukt des Arabidopsis thaliana Genlocus At3g01980 |
| 12. | SEQ ID NO: 12 | Aminosäuresequenz kodierend für das Genprodukt des Arabidopsis thaliana Genlocus At3g01980 |
| 13. | SEQ ID NO: 13 | Nukleinsäuresequenz (cDNA) kodierend für das Genprodukt des Arabidopsis thaliana Genlocus At1g63140 |
| 14. | SEQ ID NO: 14 | Aminosäuresequenz kodierend für das Genprodukt des Arabidopsis thaliana Genlocus At1g63140 |
| 15. | SEQ ID NO: 15 | Nukleinsäuresequenz (cDNA) kodierend für Raps Homolog (H2) zum At3g01980 Genprodukt |
| 16. | SEQ ID NO: 16 | Aminosäuresequenz kodierend für Raps Homolog (H2) zum At3g01980 Genprodukt |
| 17. | SEQ ID NO: 17 | Nukleinsäuresequenz (cDNA) kodierend für Raps Homolog (H3) zum At3g01980 Genprodukt |
| 18. | SEQ ID NO: 18 | Aminosäuresequenz kodierend für Raps Homolog (H3) zum At3g01980 Genprodukt |
| 19. | SEQ ID NO: 19 | Nukleinsäuresequenz (cDNA) kodierend für Raps Homolog (H4) zum At1g63140 Genprodukt |
| 20. | SEQ ID NO: 20 | Aminosäuresequenz kodierend für Raps Homolog (H4) zum At1g63140 Genprodukt |
| 21. | SEQ ID NO: 21 | Nukleinsäuresequenz (cDNA) kodierend für Raps Homolog (H5) zum At1g63140 Genprodukt |
| 22. | SEQ ID NO: 22 | Aminosäuresequenz kodierend für Raps Homolog (H5) zum At1g63140 Genprodukt |
| 23.-32 | SEQ ID NO: 23 bis 32: | Sequenzmotive für Proteine mit einer spezifischen Expression in den nicht reproduktiven Blütengewebe. |
| 33. | Seq ID No: 33 | Oligonukleotid-Primer GUS for 5'-cac ttt tcc cgg caa taa cat-3" |
| 34. | Seq ID No: 34 | Oligonukleotid-Primer GUS rev 5'-atc agg aag tga tgg agc atc-3' |
| 35. | Seq ID No: 35 | Oligonukleotid-Primer TUB for 5'-gac cct gtc cca cct cca a-3' |
| 36. | Seq ID No: 36 | Oligonukleotid-PrimerTUB rev 5'-tga gaa ctg cga ttg ttt gca-3' |

### Abbildungen

Die in nachfolgenden Abbildungen verwendeten allgemeinen Abkürzungen haben folgende Bedeutung:

| | |
|---|---|
| GUS: | Reportergen (bakterielle β-Glucuronidase) |
| Int: | Intron |
| NosT: | Terminatorsequenz der Nopalin-Synthase (NOS) |
| NptII: | BASTA Resistenz |
| NosP: | Promotorsequenz der Nopalin-Synthase (NOS) |
| AadA: | bakterielle Spectinomycin Resistenz |

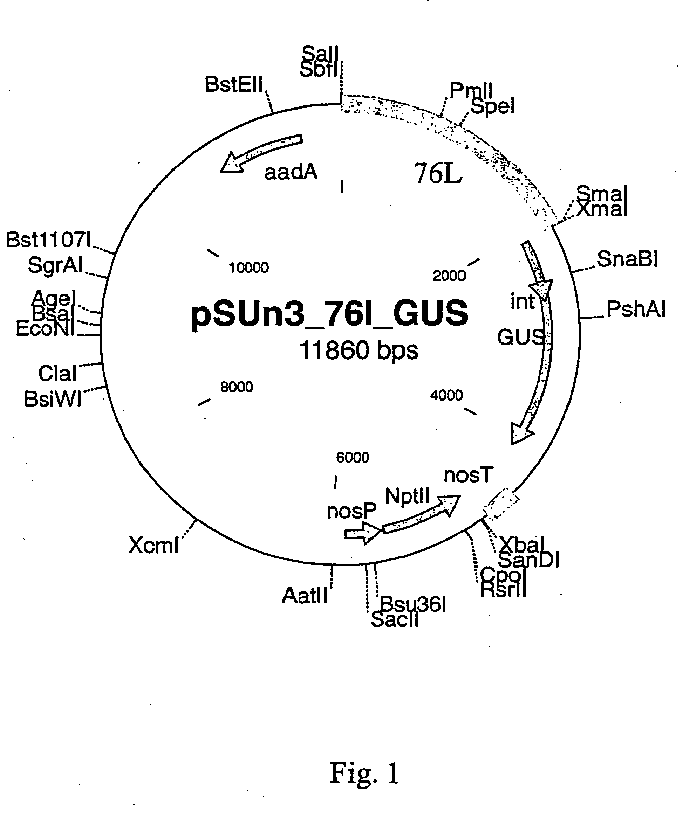

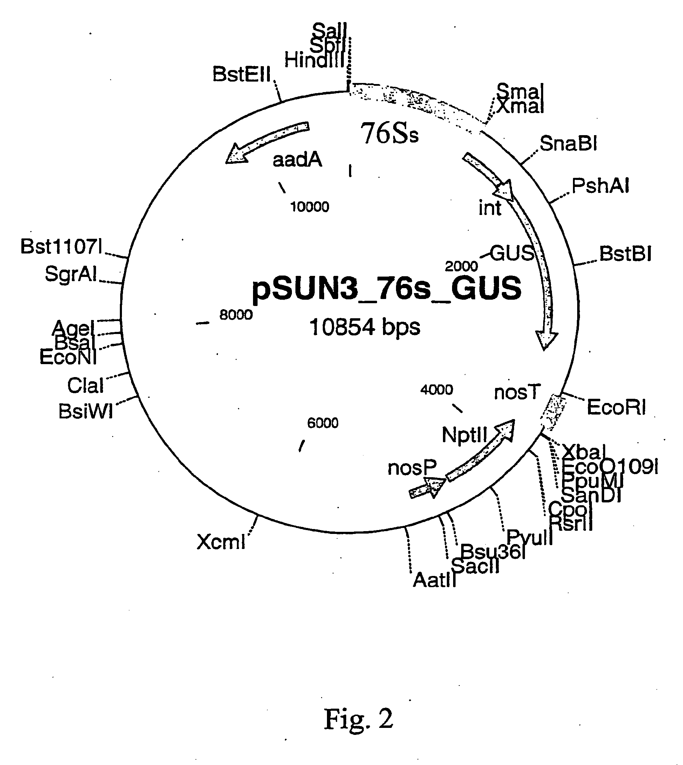

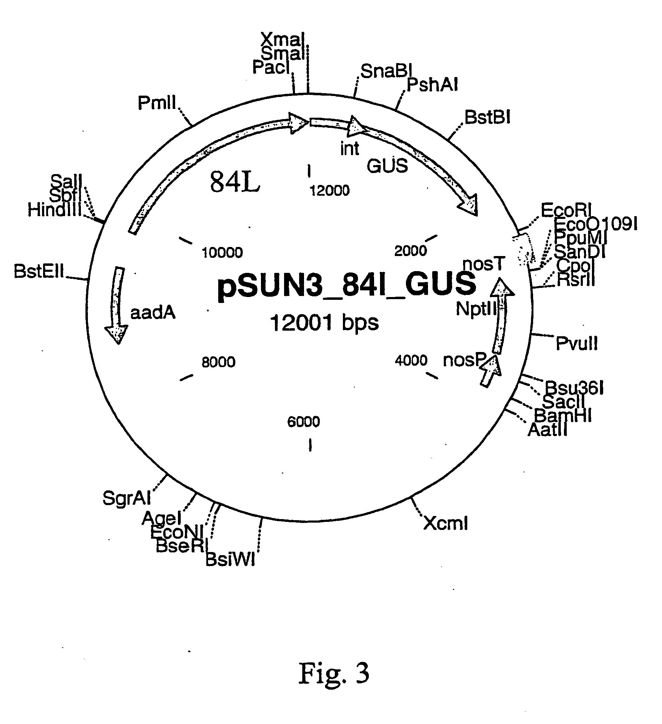

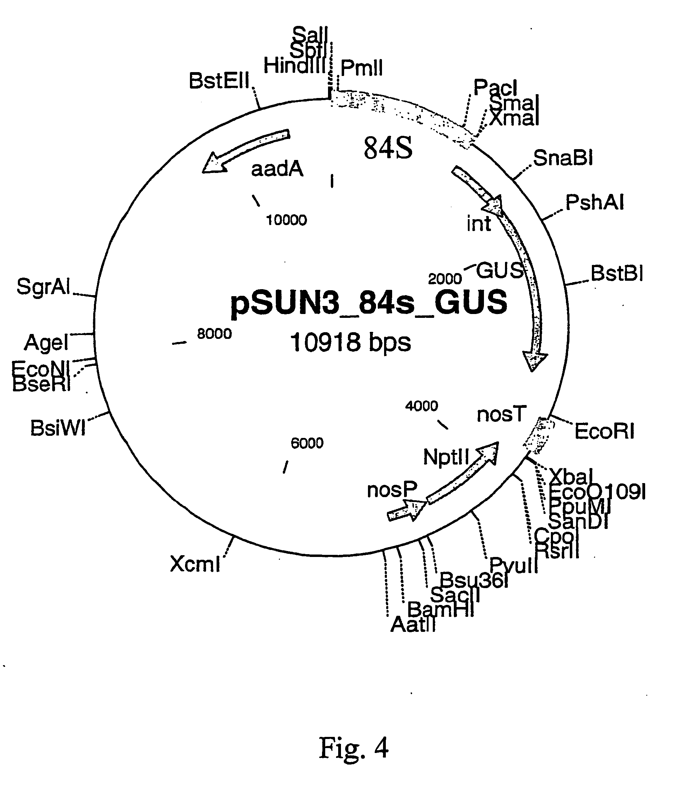

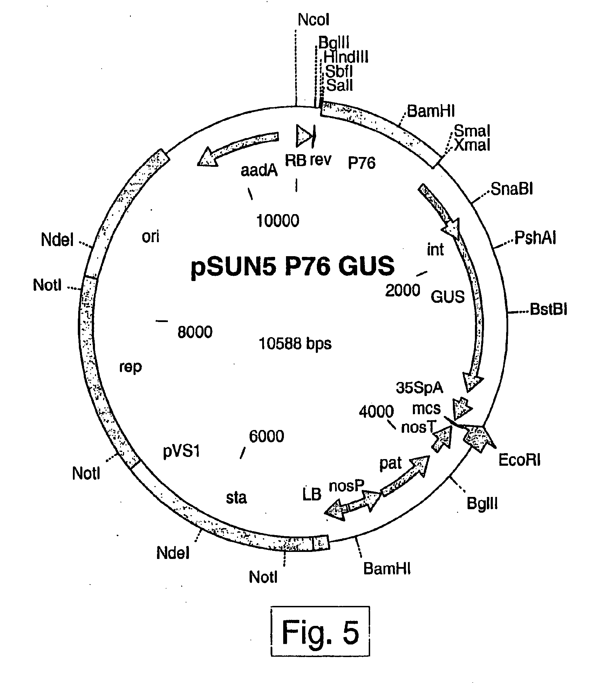

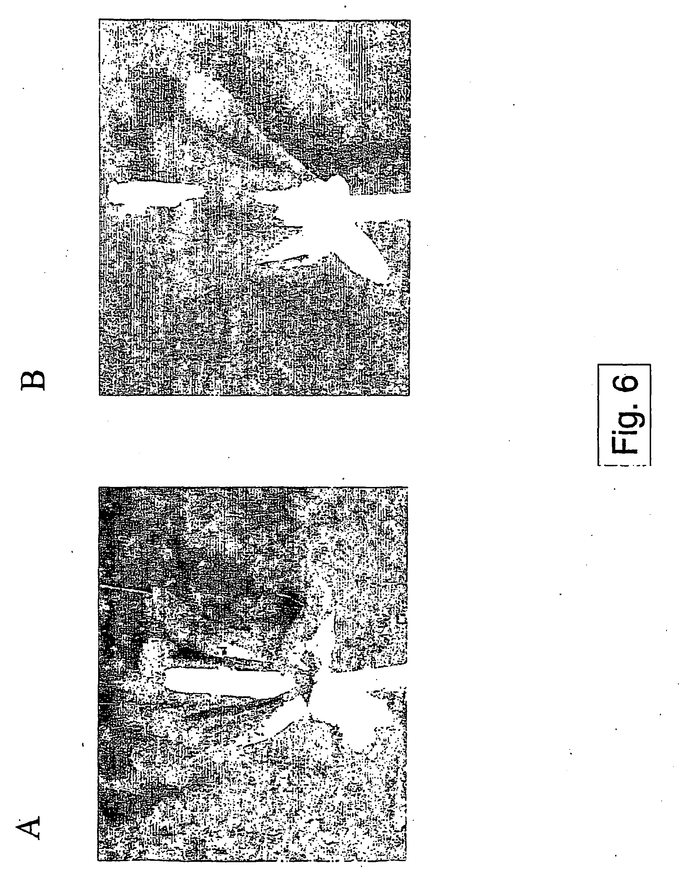

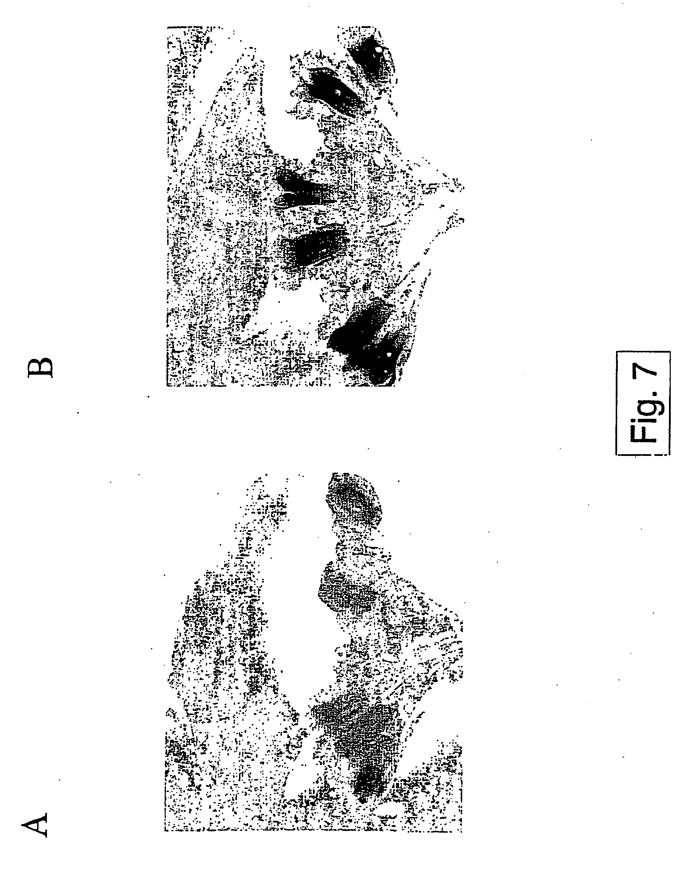

1. Fig. 1: Gezeigt eine schematische Darstellung des Vektors pSUN3-76L-GUS. Weitere Abkürzungen haben folgende Bedeutung:
   76L: 76L-Promotor gemäß SEQ ID NO:1
2. Fig. 2: Gezeigt ist eine schematische Darstellung des Vektors pSUN3-76S-GUS. Weitere Abkürzungen haben folgende Bedeutung:
   76S: 76S-Promotor gemäß SEQ ID NO: 3
3. Fig. 3: Gezeigt ist eine schematische Darstellung des Vektors pSUN3-84L-GUS. Weitere Abkürzungen haben folgende Bedeutung:
   84L: 84L-Promotor gemäß SEQ ID NO: 2
4. Fig. 4: Gezeigt ist eine schematische Darstellung des Vektors pSUN3-84S-GUS. Weitere Abkürzungen haben folgende Bedeutung:
   84S: 84S-Promotor gemäß SEQ ID NO: 4
5. Fig. 5: Gezeigt ist eine schematische Darstellung des Vektors pSUN5-P76-GUS. Weitere Abkürzungen haben folgende Bedeutung:
   P76: 76S-Promotor gemäß SEQ ID NO: 3
6. Fig.: 6: Gezeigt sind die Expressionsmuster der Promotoren 76 (A) und 84 (B) in der Blüte von Arabidopsis thaliana. Weiße/hellgraue Flächen bezeichnen Gewebe ohne Promotoraktivität.
7. Fig.: 7: Gezeigt sind die Expressionsmuster der Promotoren 76 (A) und 84 (B) in Blütenständen und Blättern von Arabidopsis thaliana. Weiße/hellgraue Flächen bezeichnen Gewebe ohne Promotoraktivität.

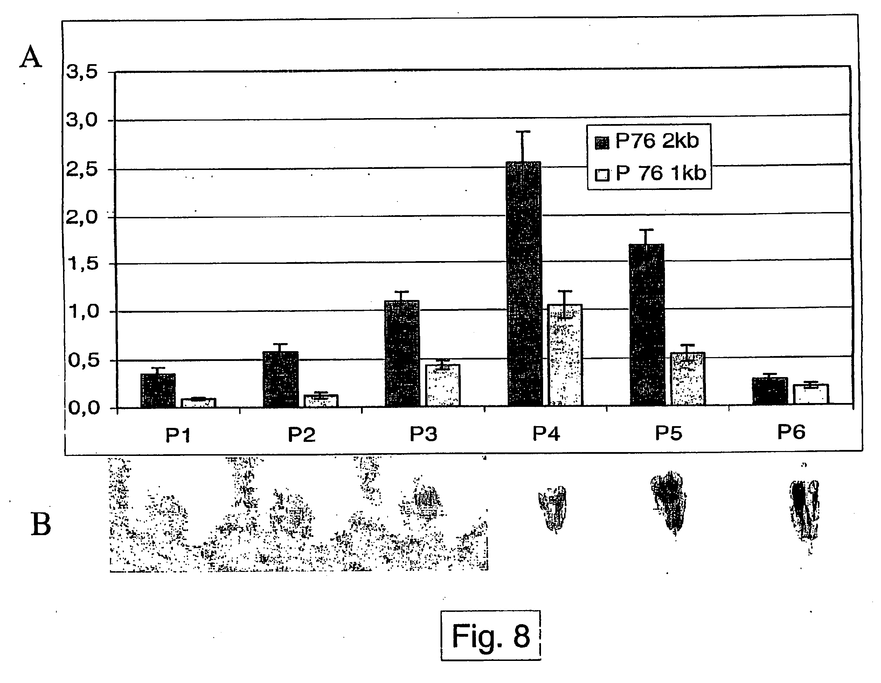

8. Fig. 8: Gezeigt ist eine zeitliche Auflösung der Promotoraktivität des Promotors 76 während der Blütenentwicklung von Arabidopsis thaliana. A:β-Glucuronidase mRNA Mengen in sechs Stadien der Blütenentwicklung (P1 bis P6) von Arabidopsis thaliana für den Promotor 76. Die Daten wurden mittels quantitativer "Real Time" PCR ermittelt und auf den 1 kb P76 Promotor im Blütenstadium P4 normiert (dazu wurde der entsprechende Wert gleich 1 gesetzt). B: Gezeigt sind die zu den Blütenstadien P1 bis P6 korrespondierenden Entswicklungszeitpunkte der Arabidopsisblüten.

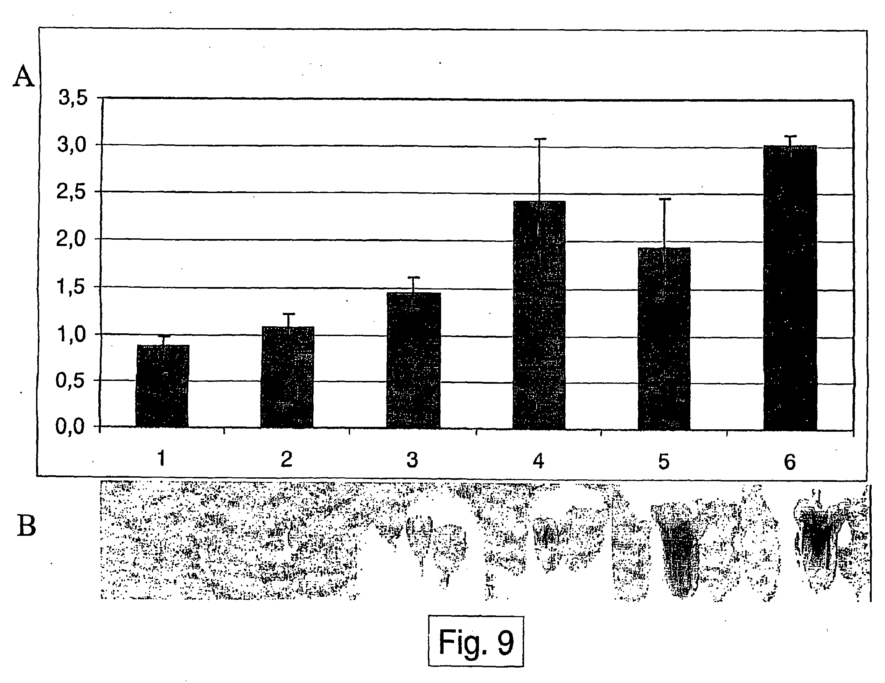

9. Fig. 9: Gezeigt ist eine zeitliche Auflösung der Promotoraktivität des Promotors 84 während der Blütenentwicklung von Arabidopsis thaliana. A:β-Glucuronidase mRNA Mengen in sechs Stadien der Blütenentwicklung (P1 bis P6) von Arabidopsis thaliana für den Promotor 84. Die Daten wurden mittels quantitativer "Real Time" PCR ermittelt und auf den das Blütenstadium P2 normiert (dazu wurde der entsprechende Wert gleich 1 gesetzt). B: Gezeigt sind die zu den Blütenstadien P1 bis P6 korrespondierenden Entswicklungszeitpunkte der Arabidopsisblüten.
10. Fig.10: Gezeigt ist eine zeitliche Auflösung der Promotoraktivität des Promotors 76 während der Blütenentwicklung von Tagetes erecta. A:β-Glucuronidase Enzymaktivität (angegeben in pmol Methylumbelliferon/min/mg Protein) in sechs Stadien der Blütenentwicklung (P1 bis P6) von Tagetes erecta für den Promotor 76. Gezeigt sind jeweils 3 Einzelmessungen (Schwarze Balken, graue Balken, weiße Balken).
   B: Gezeigt sind die zu den Blütenstadien P1 bis P6 korrespondierenden Entswicklungszeitpunkte der Tagetesblüten.

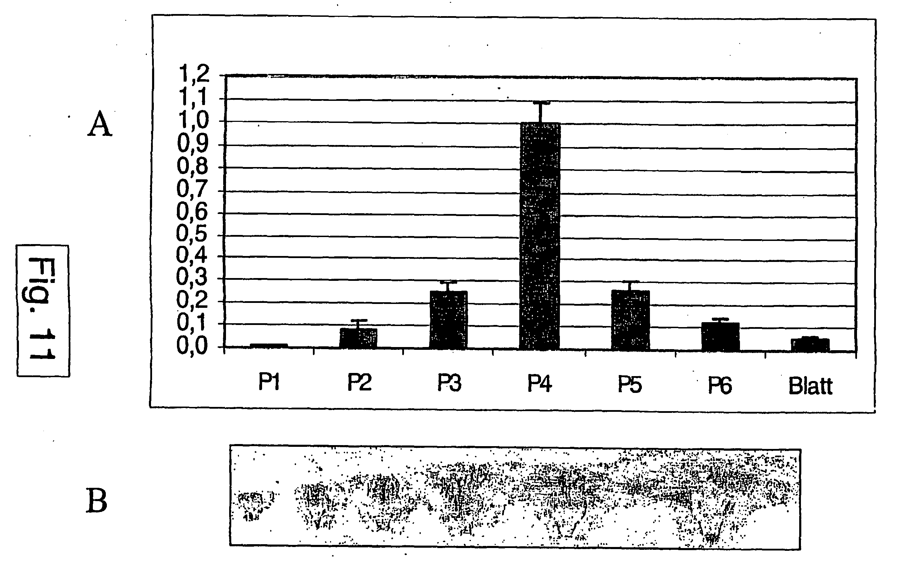

11. Fig. 11: Gezeigt ist eine zeitliche Auflösung der Promotoraktivität des Promotors 76 während der Blütenentwicklung von Tagetes erecta. A:β-Glucuronidase mRNA Mengen in sechs Stadien der Blütenentwicklung (P1 bis P6) von Tagetes erecta für den Promotor 76. Die Daten wurden mittels quantitativer "Real Time" PCR ermittelt und auf den das Blütenstadium P4 normiert (dazu wurde der entsprechende Wert gleich 1 gesetzt). B: Gezeigt sind die zu den Blütenstadien P1 bis P6 korrespondierenden Entswicklungszeitpunkte der Tageteslüten.

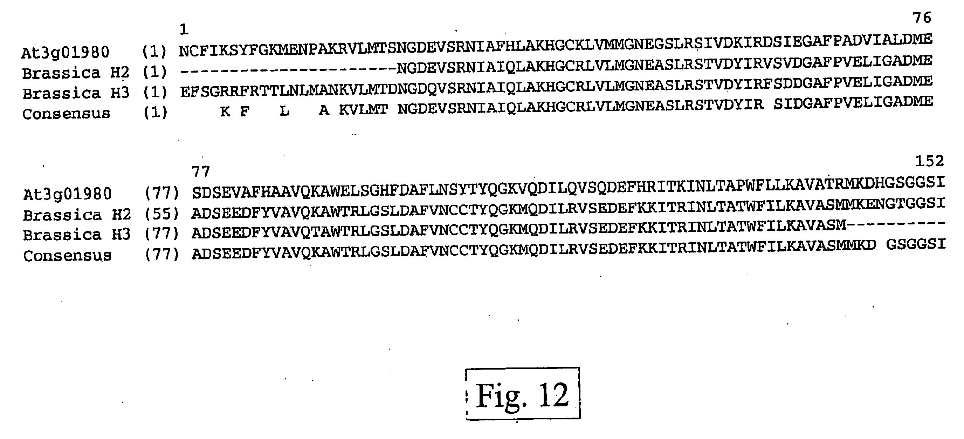

12. Fig. 12: Protein-Sequenzvergleich zwischen SEQ ID NO: 12 Aminosäuresequenz (cDNA) kodierend für das Genprodukt des Arabidopsis thaliana Genlocus At3g01980 und einem cDNA Klon aus einer Blüten cDNA Bank aus Brassica napus

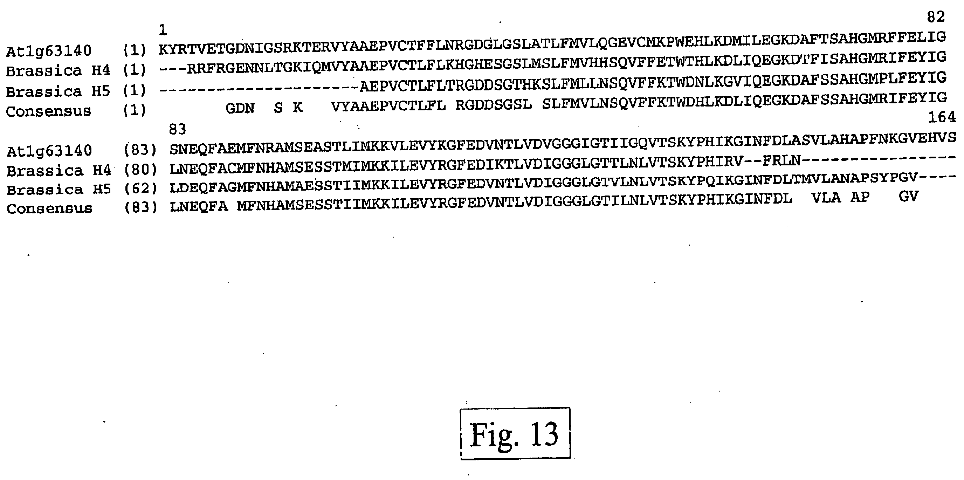

13. Fig. 13: Protein-Sequenzvergleich zwischen SEQ ID NO: 14 Aminosäuresequenz (cDNA) kodierend für das Genprodukt des Arabidopsis thaliana Genlocus At1g63140 und einem cDNA Klon aus einer Blüten cDNA Bank aus Brassica napus.

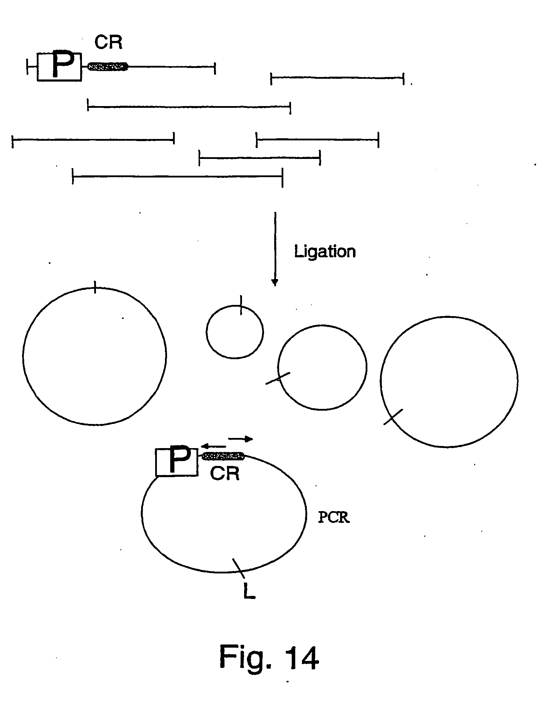

14. Fig. 14: Schematische Darstellung der inverse PCR ("iPCR") Für die "iPCR" wird genomische DNA eines Zielorganismus mit der zu isolierenden Promotorsequenz mit einem gegebenen Restriktionsenzym komplett verdaut und anschließend werden in einem verdünnten Ansatz die einzelnen Fragmente rückligiert, also mit sich selbst zu einem ringförmigen Molekül verbunden. In der Vielzahl enstehender ringförmiger DNA-Moleküle befinden sich auch solche, die die bekannte Sequenz (d.h. die Sequenz kodierend für ein homologes Protein) enthalten. Ausgehend davon kann das ringförmige Molekül mittels PCR amplifiziert werden, indem ein Primerpaar verwendet wird, bei dem beide Primer sich an den bekannten Sequenzabschnitt anlagern können. Abkürzungen: P - Promotorsequenz; CR - kodierende Region; L - Ligationsstelle; PCR - Polymerasekettenreaktion. Pfeile geben die Bindestelle potentieller Oligonukleotidprimer im Bereich der kodierenden Region wieder.

### Beispiele

### Allgemeine Methoden:

Die chemische Synthese von Oligonukleotiden kann beispielsweise, in bekannter Weise, nach der Phosphoamiditmethode (Voet & Voet (1995), 2. Auflage, Wiley Press New York, Seite 896-897) erfolgen. Die im Rahmen der vorliegenden Erfindung durchgeführten Klonierungsschritte wie z.B. Restriktionsspaltungen, Agarosegelelektrophorese, Reinigung von DNA-Fragmenten, Transfer von Nukleinsäuren auf Nitrozellulose und Nylonmembranen, Verknüpfen von DNA-Fragmenten, Transformation von E. coli Zellen, Anzucht von Bakterien, Vermehrung von Phagen und Sequenzanalyse rekombinanter DNA werden wie bei Sambrook et al. (1989) Cold Spring Harbor Laboratory Press; ISBN 0-87969-309-6 beschrieben durchgeführt. Die Sequenzierung rekombinanter DNA-Moleküle erfolgt mit einem Laserfluoreszenz-DNA-Sequenzierer der Firma ABI nach der Methode von Sanger (Sanger et al.(1977) Pro Natl Acad Sci USA 74:5463-5467).

Zur Herstellung transgener Arabidopsis Pflanzen wird Agrobakterium tumefaciens (Stamm C58C1 pMP90) mit verschiedenen Promoter-GUS Vektorkonstrukten transformiert. Die Agrobakterienstämme werden anschliessend zur Herstellung transgener Pflanzen verwendet. Dazu wird eine einzelne transformierte Agrobakterium Kolonie in einer 4 ml Kultur (Medium: YEB-Medium mit 50 µg/ml Kanamycin und 25 µg/ml Rifampicin über Nacht bei 28°C inkubiert. Mit dieser Kultur wird anschliessend eine 400 ml Kultur in demselben Medium angeimpft, über Nacht inkubiert (28 °C, 220 U/min) und abzentrifugiert (GSA-Rotor, 8.000 U/min, 20 min). Das Pellet wird in Infiltrationsmedium (1/2 MS-Medium; 0,5 g/l MES, pH 5,8; 50 g/l Saccharose) resuspendiert. Die Suspension wird in eine Pflanzenbox (Duchefa) eingebracht und 100 ml SILVET L-77 (mit Polyalkylenoxid modifiziertes Heptamethyltrisiloxan; Osi Special-ties Inc., Cat. P030196) wurde zu einer Endkonzentration von 0.02% hinzugegeben. Die Pflanzenbox mit 8 bis 12 Pflanzen wird in einem Exikator für 10 bis 15 Minuten einem Vakuum mit anschliessender spontaner Belüftung ausgesetzt. Dies wird 2 bis 3 Mal wiederholt. Hernach werden alle Pflanzen in Pflanztöpfe mit Feuchterde gepflanzt und unter Langtagbedingungen (16 h Beleuchtung) gezüchtet (Tagestemperatur 22 bis 24°C, Nachtemperatur 19°C; 65 % relative Luftfeuchte). Nach 6 Wochen werden die Samen geerntet.

### Beispiel 1: Wachstumsbedingungen der Pflanzen für gewebsspezifische RT-PCR Analyse

Um 4 bzw. 7 Tage alte Keimlinge zu erhalten, werden jeweils ungefähr 400 Samen (Arabidopsis thaliana Ökotyp Columbia) oberflächig mit einer 80%igen Ethanollösung für 2 Minuten sterilisiert, mit einer Natriumhypochloritlösung (0.5 % v/v) 5 Minuten behandelt, dreimal mit destilliertem Wasser gewaschen und bei 4°C für 4 Tage inkubiert, um eine gleichmässige Keimung sicherzustellen. Anschliessend werden die Samen auf Petrischalen mit MS Medium (Sigma M5519) unter Zusatz von 1% Saccharose, 0.5 g/l MES (Sigma M8652), 0.8 % Difco-BactoAgar (Difco 0140-01), pH 5.7 inkubiert. Die Sämlinge werden in einem 16-stündigen Licht / 8-stündigen Dunkelheitszyklus (Philips 58W/33 Weisslichtlampen) bei 22°C gezüchtet und nach 4 bzw. 7 Tagen nach Beginn der Keimphase geerntet.

Für die Gewinnung von Wurzeln werden 100 Samen wie oben beschrieben sterilisiert, bei 4°C 4 Tage inkubiert und dann in 250ml Flasehen mit MS Medium (Sigma M5519) unter Zusatz von weiteren 3 % Saccharose und 0.5 g/l MES (Sigma M8652), pH 5.7 gezüchtet. Die Sämlinge werden in einem 16-stündigen Licht- / 8-stündigen Dunkelheitszyklus (Philips 58W/33 Weisslichtlampen) bei 22°C, 120 U/min gezüchtet und nach 3 Wochen geerntet. Für alle anderen verwendeten pflanzlichen Organe werden die Samen auf Einheitserde (Typ VM, Manna-Italia, Via S. Giacomo 42, 39050 San Giacomo/Laives, Bolzano, Italien) ausgesäht, 4 Tage bei 4°C inkubiert um eine gleichmässige Keimung zu gewährleisten und dann in einem 16-stündigen Licht- / 8-stündigen Dunkelheitszyklus (OSRAM Lumilux Daylight 36W/12 Leuchtstoffröhren) bei 22°C gezüchtet. Junge Rosettenblätter werden im 8-Blattstadium (nach 3 Wochen) geerntet, reife Rosettenblätter werden nach 8-Wochen kurz vor der Stengelbildung geerntet. Blütenstände (Apices) der ausschießenden Stengel werden kurz nach dem Ausschießen geerntet. Stengel, Stengelblätter und Blütenknospen werden in der Entwicklungsstufe 12 (Bowmann J (ed.), Arabidopsis, Atlas of Morphology, Springer New York, 1995) vor der Staubblattentwicklung geerntet. Geöffnete Blüten werden in Stadium 14 sofort nach der Staubblattentwicklung geerntet. Welkende Blüten werden in Stadium 15 bis 16 geerntet. Die verwendeten grünen und gelben Schoten hatten eine Länge von 10 bis 13 mm.

### Beispiel 2: RNA Extraktion und cDNA Synthese

Gesamt-RNA wird aus den in Beispiel 1 beschriebenen Organen der Pflanze zu verschiedenen Zeitpunkten der Entwicklung wie beschrieben isoliert (Prescott A, Martin C (1987) Plant Mol Biol Rep 4:219-224). Die Reverse Transkriptase Polymerasekettenreaktion (RT-PCR) wird verwendet, um die cDNA der Gentranskripte von AT3g01980 und At1G63140 nachzuweisen. Alle RNA Proben werden mit DNasel (15 Units, Boehringer, Mannheim) vor der cDNA Synthese behandelt. Die Erststrang cDNA Synthese wird ausgehend von 6 µg Gesamt-RNA mit einem oligo-(dT) Primer und RT Superscript^{™}II Enzym (300 Units) nach Angaben des Herstellers in einem Gesamtvolumen von 20 µl (Life Technologies, Gaithersburg, MD) durchgeführt. Zur RNA werden dazu 150 ng "Random Hexamer Primer" in einem Endvolumen von 12 µl gegeben. Der Ansatz wird für 10 min bei 70°C erhitzt und anschließend sofort auf Eis gekühlt. Dann werden 4 µl des 5X Erststrangpuffers, 2 µl 0,1 M DTT, 1 µl 10 mM dNTP-Mix (jeweils 10 mM dATP, dCTP, dGTP und dTTP) und RNase Inhibitor (5 Units, Böhringer Mannheim) zugegeben. Der Ansatz wird für 2 min auf 42°C erhitzt, RT Superscript^{™}II Enzym (300 Units, Life Technologies) zugegeben und für 50 min bei 42°C inkubiert.

### Beispiel 3: Nachweis der gewebespezifischen Expression

Um die Eigenschaften des Promotors zu bestimmen und die essentiellen Elemente desselben, die seine Gewebespezifität ausmachen, zu identifizieren, ist es erforderlich, den Promotor selbst und verschiedene Fragmente desselben vor ein sogenanntes Reportergen zu setzen, das eine Bestimmung der Expressionsaktivität ermöglicht. Beispielhaft sei die bakterielle β-Glucuronidase genannt (Jefferson et al. (1987) EMBO J 6:3901-3907). Die β-Glucuronidase Aktivität kann in planta mittels eines chromogenen Substrates wie 5-Bromo-4-Chloro-3-Indolyl-β-D-Glucuronsäure im Rahmen einer Aktivitätsfärbung bestimmt werden (Jefferson et al. (1987) Plant Mol Biol Rep 5:387-405). Für die Untersuchungen der Gewebespezifität wird das pflanzliche Gewebe geschnitten, eingebettet, gefärbt und analysiert wie beschrieben (z.B. Bäumlein H et al. (1991) Mol Gen Genet 225:121-128).

Für die quantitative Aktivitätsbestimmung der β-Glucuronidase wird als Substrat MUG (Methylumbelliferylglucuronid) verwendet, das in MU (Methylumbelliferon) und Glucuronsäure gespalten wird. Unter alkalischen Bedingungen kann diese Spaltung quantitativ fluorometrisch verfolgt werden (Anregung bei 365 nm, Messung der Emission bei 455 nm; SpectroFluorimeter Thermo Life Sciences Fluoroscan) wie beschrieben (Bustos MM et al. (1989) Plant Gell 1:839-853).

### Beispiel 4: Klonierung der Promotoren

Um die vollständigen Promotoren gemäß Seq ID NO: 1 oder 2 zu isolieren, wird genomische DNA aus Arabidopsis thaliana (Ökotyp Landsberg erecta) extrahiert wie beschrieben (Galbiati M et al. Funct Integr Genomics 2000, 20 1:25-34). Die isolierte DNA wird als Matrizen-DNA in einer PCR unter Verwendung folgender Primer eingesetzt:

| Promotor | Forward Primer | Reverse Primer |
|---|---|---|
| 761 (SEQ ID NO:2) | SEQ ID NO: 5 (76s) | SEQ ID NO: 7 (76asl) |
| 841 (SEQ ID NO:3) | SEQ ID NO: 8 (84s) | SEQ ID NO: 10 (84asl) |
| 76s (SEQ ID NO:4) | SEQ ID NO: 5 (76s) | SEQ ID NO: 6 (76ass) |
| 84s (SEQ ID NO:5) | SEQ ID NO: 8 (84s) | SEQ ID NO: 9 (84ass) |

Die Amplifikation wird wie folgt durchgeführt:
80 ng genomische DNA
1X Expand^{™} Long Template PCR Puffer
2,5 mM MgC12,
je 350 µM von dATP, dCTP, dGTP und dTTP
je 300 nM eines jeden Primers -(SEQ ID NO: 5 und 7 für Promoter 761 und SEQ ID NO 8 und 10 für Promoter 84s)
2,5 Units Expand^{™} Long Template Polymerase (Roche Diagnostics).
   in einem Endvolumen von 25 µl

Folgendes Temperaturprogramm wird verwendet (PTC-100TM Modell QfiV; MJ Research, Inc., Watertown, Massachussetts):
1 Zyklus mit 120 sec bei 94°C
35 Zyklen mit 94°C für 10 sec, 55°C für 30 sec und 68°C für 3min.
1 Zyklus mit 68°C für 30 min 45

Die PCR-Produkte wurden mit den Restriktionsendonucleasen SmaI und SalI geschnitten und in den Vektor pSUN::GUS kloniert. Die resultierenden Konstrukte sind pSUN3-76L::GUS (Fig.9), pSUN3-76S::GUS (Fig.10), pSUN3-84L::GUS .(Fig.11) und pSUN3-84S::GUS (Fig.11). Nach der stabilen Transformation dieser Konstrukte in Arabidopsis thaliana kann RNA aus den verschiedenen Geweben gewonnen werden und die Expression des GUS Gens mittels RT PCR qualitativ und mittels "Real Time" PCR quantitativ dargestellt werden.

Die Methode zur quantitativen "Real Time" PCR ist beschrieben z.B. in Bustin SA (2000) J Mol Endocrinol 25(2):169-93.

Zur Detektion und Quantifizierung der GUS mRNA wurden die Primer

| | |
|---|---|
| GUS for | 5'-cac ttt tcc cgg caa taa cat-3' |
| GUS rev | 5'-atc agg aag tga tgg agc atc-3' |

verwendet. Zur Normalisierung wurden die Werte auf das konstitutiv exprimierte Tubulin bezogen. Zur Detektion und Quantifizierung von Tubulin wurden die Primer

| | |
|---|---|
| TUB for | 5'-gac cct gtc cca cct cca a-3' |
| TUB rev | 5'-tga gaa ctg cga ttg ttt gca-3' |

verwendet.

### Beispiel 5: TAIL-PCR

Die "TAIL-PCR" wird entsprechend einem adaptrierten Protokoll der Methode von Liu et al. (1995) Plant J 8(3):457-463 und Tsugeki et al. (1996) Plant J 10(3):479-489 durchgeführt (vgl. Fig. 9). Für eine erste PCR-Reaktion wird folgender Mastermixes (Angaben pro Reaktionsansatz) eingesetzt
11 µl steriles H₂O (bidestilliert)
2 µl Primer-Stocklösung des spezifischen Primers 1 (5mM)
3 µl AD2 Primer-Stocklösung (20mM)
2 µl 10x-PCR-Puffer
2 µl 10xdNTP
0,2 µl Taq Polymerase
19 µl dieses Mastermixes werden in einem PCR-Gefäß zu 1 µl einer Präparation genomischer DNA des jeweiligen Zielorganismus (Präparation gemäß Galbiati M et al. (2000) Funct Integr Genomics 20(1):25-34)) hinzupipettiert und durch Pipettieren gut gemischt.

Die primäre PCR-Reaktion wird unter folgenden Bedingungen durchgeführt:
- 94°C für 1 min
- Vier Zyklen mit 94°C für 10s, 62°C für 1 min und 72°C für 150s
- 94°C für 10s, 25°C für 3 min, 0,2°C/s bis 72°C und 72°C für 150s
- Vierzehn Zyklen mit 94°C für 10s, 69°C für 1min, 72°C für 150s, 94°C für 10s, 68°C für 1 min, 72°C für 150s, 94°C für 10s, 44°C für 1 min und 72°C für 150s
- 72°C für 5 min, dann 4°C bis zur Weiterverwendung.

Das Produkt der PCR-Reaktion wird 1:50 verdünnt und je 1 µl jeder verdünnten Probe wird für eine zweite PCR-Reaktion (sekundäre PCR) verwendet. Dazu wird folgender Mastermix (Angaben pro Reaktionsansatz) eingesetzt:
12 µl steriles H₂O (bidestilliert)
2 µl 10x-PCR-Puffer (1,5 mM MgCl₂)
2 µl 10xdNTP
2 µl Primer-Stocklösung des spezifischen Primers 2 (5 mM)
2 µl AD2 Primer-Stocklösung
0,2µl Taq Polymerase

Je 20,2 µl des zweiten Mastermix werden zu je 1 µl des 1:50 verdünnten primären PCR-Produktes gegeben und die sekundäre PCR wird unter folgenden Bedingungen durchgeführt:
- 11 Zyklen mit 94°C für 10s, 64°C für 1 min, 72°C für 150s, 94°C für 10s, 64°C für 1 min, 72°C für 150s, 94°C für 10s, 44°C für 1 min, 72°C für 150s,
- 72°C für 5min, dann 4°C bis zur Weiterverwendung.

Das PCR-Produkt der vorhergehenden Reaktion wird 1:10 verdünnt und je 1 µl jeder verdünnten Probe wird für eine dritte PCR-Reaktion (tertiäre PCR) verwendet. Dazu wird folgender Mastermix (Angaben pro Reaktionsansatz) eingesetzt:
18 µl steriles H₂O (bidestilliert)
3 µl 10x-PCR-Puffer (1,5 mM MgCl₂)
3 µl 10xdNTP
3 µl Primer-Stocklösung des spezifischen Primers 3 (5mM)
3 µl AD2 Primer-Stocklösung
0,5 µl Taq Polymerase

Je 30,3 µl dieses Mastermixes werden zu je 1 µl des 1:10 verdünnten sekundären PCR-Produktes gegeben und die tertiäre PCR wird unter folgenden Bedingungen durchgeführt:
- 19 Zyklen mit 94°C für 15s, 44°C für 1 min, 72°C für 150s,
- 72°C für 5 min, dann 4°C bis zur Weiterverwendung.

Von den Produkten der PCR 1, 2 und 3 jeder Probe werden je 5 µl auf einem 2%igen Agarosegel aufgetrennt. Diejenigen PCR-Produkte, die wegen der versetzten spezifischen Primer die erwartete Größenverringerung aufweisen, werden bei Bedarf aus dem Gel gereinigt und mit dem zuletzt verwendeten Primerpaar erneut amplifiziert und sequenziert.

### Reagenzien:

Taq-Polymerase 5U/µl
10x PCR-Puffer (1,5 mM MgCl₂)
10x dNTP-Stocklösung: 2 mM

### Primer:

Degenerierte Zufallsprimer (Stocklösungen 20 µM):
AD1: 5'-NTCGA(G/C)T(A/T)T(G/C)G(A/T)GTT-3'
AD2: 5'-NGTCGA(G/C)(A/T)GANA(A/T)GAA-3'
AD5: 5'-(A/T)CAGNTG(A/T)TNGTNCTG-3'

### Beispiel 6: Inverse PCR (iPCR) für die Amplifikation Insert-flankierdender DNA

Die "iPCR" wird entsprechend einem adaptierten Protokoll der Methode von Long et al.(1993) PNAS 90:10370 (vgl. Fig.8) durchgeführt:
1. Restriktion von ca. 2 µg genomischer DNA mit *Bst*YI für ca. 2h bei 37°C in einem Volumen von insgesamt 50 µl.
2. Ligation von 25 µl des Restriktionsansatzes in einem Gesamtvolumen von 300 µl mit 3U T4-DNA-Ligase bei 15°C über Nacht.
3. Phenol/Chloroform-Extraktion und anschließende Chloroform Extraktion des Ligationsansatzes. Nach Ethanolfällung DNA in 10 µl sterilem H₂O (bidestilliert) aufnehmen.
4. Für die PCR 2,5µl der DNA-Lösung einsetzen

### Reaktionsansatz:

2,5 µl der DNA-Lösung
10 µl 10x PCR-Puffer
2 µl dNTP (je 10mM im Gemisch)
5 µl Primer 1 (25pmol)
5 µl primer 2 (25pmol))
1,5 µl Taq-Polymerase
74 µl H₂O (bidestilliert, steril)
auf 100 µl Gesamtvolumen

PCR-Protokoll: 4 min für 94°C. Dann 35 Zyklen mit 1 min für 94°C, 2 min für 55°C und 3 min für 72°C. Abschließend 8 min für 72°C, dann 4°C bis zur Weiterverwendung.

Das PCR-Produkt wird per Gelelektrophorese kontrolliert, aufreinigt und anschließend als PCR-Produkt sequenziert.

### Beispiel 6: Herstellung transgener Tagetes Pflanzen

Tagetessamen werden sterilisiert und auf Keimungsmedium (MS-Medium; Murashige and Skoog (1962) Physiol Plant 15:473-497) pH 5,8, 2% Saccharose) aufgelegt. Die Keimung erfolgt in einem Temperatur/Licht/Zeitintervall von 18 bis 28°C, 20 bis 200 µE, 3 bis 16 Wochen, bevorzugt jedoch bei 21°C, 20 bis 70 µE, für 4 bis 8 Wochen.

Alle Blätter der sich bis dahin entwickelten in vitro Pflanzen werden geerntet und quer zur Mittelrippe geschnitten. Die dadurch entstehenden Blattexplantate mit einer Größe von 10 bis 60 mm² werden im Verlaufe der Präparation in flüssigem MS - Medium bei Raumtemperatur für maximal 2 h aufbewahrt.

Ein beliebiger Agrobakterium tumefaciens Stamm, bevorzugt aber ein supervirulenter Stamm, wie z.B. EHA105 mit einem entsprechenden Binärplasmid, das ein Selektionsmarkergen (bevorzugt bar oder pat) sowie ein oder mehrere Trait- oder Reportergene tragen kann wird (beispielsweise pS5KETO2 und pS5AP3PKETO2), über Nacht angezogen und für die Co-Kultivierung mit dem Blattmaterial verwendet. Die Anzucht des Bakterienstammes kann wie folgt erfolgen: Eine Einzelkolonie des entsprechenden Stammes wird in YEB (0,1 % Hefeextrakt, 0,5 % Rindfleischextrakt, 0,5 % Pepton, 0,5 % Saccharose, 0,5 % Magnesiumsulfat x 7 H20) mit 25 mg/l Kanamycin angeimpft und bei 28°C für 16 bis 20 h angezogen. Anschließend wird die Bakteriensuspension durch Zentrifugation bei 6000 g für 10 min geerntet und derart in flüssigem MS Medium resuspendiert, daß eine OD600 von ca. 0,1 bis 0,8 entstand. Diese Suspension wird fuer die C-Kultivierung mit dem Blattmaterial verwendet.

Unmittelbar vor der Co-Kultivierung wird das MS-Medium, in dem die Blätter aufbewahrt worden sind, durch die Bakteriensuspension ersetzt. Die Inkubation der Blättchen in der Agrobakteriensuspension erfolgte für 30 min unter leichtem Schütteln bei Raumtemperatur. Anschließend werden die infizierten Explantate auf ein mit Agar (z.B. 0,8 % Plant Agar (Duchefa, NL) verfestigtes MS-Medium mit Wachstumsregulatoren, wie beispielsweise 3 mg/l Benzylaminopurin (BAP) sowie 1 mg/l Indolylessigsäure (IAA) aufgelegt. Die Orientierung der Blätter auf dem Medium ist bedeutungslos. Die Kultivierung der Explantate findet für 1 bis 8 Tage, bevorzugt aber für 6 Tage statt, dabei können folgende Bedingungen angewendet werden: Lichtintensität: 30 bis 80 µMol/m² x sec, Temperatur: 22 bis 24°C, hell/dunkel Wechsel von 16/8 Stunden. Anschließend werden die co-kultivierten Explantate auf frisches MS-Medium, bevorzugt mit den gleichen Wachstumsregulatoren übertragen, wobei dieses zweite Medium zusätzlich ein Antibiotikum zur Unterdrükkung des Bakterienwachstums enthält. Timentin in einer Konzentration von 200 bis 500 mg/l ist für diesen Zweck sehr geeignet. Als zweite selektive Komponente wird eine für die Selektion des Transformationserfolges eingesetzt. Phosphinothricin in einer Konzentration von 1 bis 5 mg/l selektiert sehr effizient, aber auch andere selektive Komponenten gemäß des zu verwendenden Verfahrens sind denkbar.

Nach jeweils ein bis drei Wochen erfolgt der Transfer der Explantate auf frisches Medium bis sich Sproßknospen und kleine Sprosse entwickeln, die dann auf das gleiche Basalmedium einschließlich Timentin und PPT oder alternative Komponenten mit Wachstumsregulatoren, nämlich z.B. 0,5 mg/l Indolylbuttersäure (IBA) und 0,5 mg/l Gibberillinsäure GA3, zur Bewurzelung übertragen werden. Bewurzelte Sprosse können ins Gewächshaus überführt werden.

Zusätzlich zu der beschriebenen Methode sind folgende vorteilhafte Modifikationen möglich:
**·** Bevor die Explantate mit den Bakterien infiziert werden, können sie für 1 bis 12 Tage, bevorzugt 3 - 4, auf das oben beschriebene Medium für die Co-Kultur vorinkubiert werden. Anschließend erfolgt die Infektion, Co-Kultur und selektive Regeneration wie oben beschrieben.
**·** Der pH Wert für die Regeneration (normalerweise 5,8) kann auf pH 5,2 gesenkt werden. Dadurch wird die Kontrolle des Agrobakterienwachstums verbessert.
**·** Die Zugabe von AgNO₃ (3 bis 10 mg/l) zum Regenerationsmedium verbessert den Zustand der Kultur einschließlich der Regeneration selbst.
· Komponenten, die die Phenolbildung reduzieren und dem Fachmann bekannt sind, wie z.B. Zitronensäure, Ascorbinsäure, PVP u.v.a.m., wirken sich positiv auf die Kultur aus.
· Für das gesamte Verfahren kann auch flüssiges Kulturmedium Verwendung finden. Die Kultur kann auch auf handelsüblichen Trägern, die auf dem flüssigen Medium positioniert werden inkubiert werden

## Patentansprüche

1. Verfahren zur gezielten, transgenen Expression von Nukleinsäuresequenzen in nicht-reproduktiven Blütengeweben von Pflanzen, wobei nachfolgende Schritte umfasst sind
I. Einbringen einer transgenen Expressionskassette in pflanzliche Zellen, wobei die transgene Expressionskassette mindestens nachfolgende Elemente enthält
a) mindestens eine Promotorsequenz, vermittelnd eine Expression allen nicht-reproduktiven Blütengeweben jedoch im wesentlichen nicht im Pollen und den Ovarien,ausgewählt aus der Gruppe von Sequenzen bestehend aus
i) den Promotorsequenzen gemäß SEQ ID NO : 1 oder 2 und
ii) funktionellen Äquivalenten der Promotorsequenzen gemäß SEQ ID NO: 1 oder 2 mit im wesentlichen der gleichen Promotoraktivität wie ein Promotor gemäß SEQ ID NO: 1 oder 2 und die eine Identität von mindestens 80% zu der Sequenz eines der Promotoren gemäß SEQ ID NO.: 1 oder 2 aufweisen und
b) mindestens eine weitere Nukleinsäuresequenz, und
c) gegebenenfalls weitere genetische Kontrollelemente,
wobei mindestens eine Promotorsequenz und eine weitere Nukleinsäuresequenz funktionell miteinander verknüpft sind und die weitere Nukleinsäuresequenz in Bezug auf die Promotorsequenz heterolog ist, und
II. Auswahl von transgenen Zellen, die besagte Expressionskassette stabil in das Genom integriert enthalten, und
III.Regeneration von vollständigen Pflanzen aus besagten transgenen Zellen, wobei mindestens eine der weiteren Nukleinsäuresequenzen in im wesentlichen allen nicht-reproduktiven Blütengeweben jedoch im wesentlichen nicht im Pollen und den Ovarien exprimiert wird.

2. Verfahren nach Anspruch 1, wobei das funktionell äquivalente Fragment eine Sequenz gemäß SEQ ID NO: 3 oder 4 umfasst.

3. Verfahren zur Herstellung einer transgenen Expressionskassette mit Spezifität für nicht-reproduktive Blütengewebe, umfassend nachfolgende Schritte:
I. Isolation eines Promotors mit Spezifität für nicht-reproduktive Blütengewebe, wobei bei der Isolation mindestens eine Nukleinsäuresequenz oder ein Teil derselben zum Einsatz kommt, wobei besagte Nukleinsäuresequenz für eine Aminosäuresequenzen kodiert, die mindestens eine Sequenz gemäß SEQ ID NO: 12, 14, 16, 18, 20, 22, 23, 24, 25, 26, 27, 28, 29, 30, 31 oder 32 umfasst oder die mindestens eine Sequenz mit einer Homologie von mindestens 80% zu einer Sequenz gemäß SEQ ID No: 12 oder 14 umfasst.
II. Funktionelle Verknüpfung besagten Promotors mit einer weiteren Nukleinsäuresequenz, wobei besagte Nukleinsäuresequenz in Bezug auf den Promotor heterolog ist.

4. Verfahren nach Anspruch 3, wobei besagte Nukleinsäuresequenz eine Sequenz gemäß SEQ ID NO: 5, 7, 11, 13, 15, 17, 19 oder 21 umfasst.

5. Verfahren nach einem der Ansprüche 3 oder 4, wobei das Verfahren unter Einsatz der Polymerasekettenreaktion durchgeführt wird und die besagte Nukleinsäuresequenz oder ein Teil derselben als Primer eingesetzt wird.

6. Verwendung mindestens einer Nukleinsäuresequenz oder eines Teils derselben in Verfahren zur Identifikation und/oder Isolation von Promotoren mit Spezifität für nicht-reproduktive Blütengewebe, wobei besagte Nukleinsäuresequenz für eine Aminosäuresequenz kodiert die mindestens eine Sequenz gemäß SEQ ID NO: 12, 14, 16, 18, 20, 22, 23, 24, 25, 26, 27, 28, 29, 30, 31 oder 32 oder die mindestens eine Sequenz mit einer Homologie von mindestens 80% zu einer Sequenz gemäß SEQ ID No: 12 oder 14 umfasst.

7. Verwendung nach Anspruch 6, wobei besagte Nukleinsäuresequenz eine Sequenz gemäß SEQ ID NO: 5, 7, 11, 13, 15, 17, 19 oder 21 umfasst.

8. Verfahren nach einem der Ansprüche 3 bis 5, oder Verwendung nach Anspruch 6 oder 7, wobei besagte Nukleinsäuresequenz für eine Aminosäuresequenz kodiert, die mindestens eine Sequenz mit einer Homologie von mindestens 90% zu einer Sequenz gemäß SEQ ID No: 12 oder 14 umfasst.

9. Verfahren nach einem der Ansprüche 3 bis 5, 8 oder Verwendung nach Anspruch 6 oder 7, wobei besagte Nukleinsäuresequenz für eine Aminosäuresequenz kodiert, die mindestens eine Sequenz mit einer Homologie von mindestens 95% zu einer Sequenz gemäß SEQ ID No: 12 oder 14 umfasst.

10. Transgene Expressionskassette zur gezielten, transgenen Expression von Nukleinsäuresequenzen in nicht-reproduktiven Blütengeweben von Pflanzen, umfassend
a) mindestens eine Promotorsequenz, vermittelnd eine Expression allen nicht-reproduktiven Blütengeweben jedoch im wesentlichen nicht im Pollen und den Ovarien, ausgewählt aus der Gruppe von Sequenzen bestehend aus
i) den Promotorsequenzen gemäß SEQ ID NO: 1 oder 2 und
ii) funktionellen Äquivalenten der Promotorsequenzen gemäß SEQ ID NO: 1 oder 2 mit im wesentlichen der gleichen Promotoraktivität wie ein Promotor gemäß SEQ ID NO: 1 oder 2 und die eine Identität von mindestens 80% zu der Sequenz eines der Promotoren gemäß SEQ ID NO.: 1 oder 2 aufweisen und
b) mindestens eine weitere Nukleinsäuresequenz, und
c) gegebenenfalls weitere genetische Kontrollelemente,
wobei mindestens eine Promotorsequenz und eine weitere Nukleinsäuresequenz funktionell miteinander verknüpft sind und die weitere Nukleinsäuresequenz in Bezug auf die Promotorsequenz heterolog ist.

11. Transgene Expressionskassette nach Anspruch 10, wobei das funktionell äquivalente Fragment eine Sequenz gemäß SEQ ID NO: 3 oder 4 umfasst.

12. Tranagene Expressionskassette nach Anspruch 10 oder 11, wobei
a) die zu exprimierende Nukleinsäuresequenz mit weiteren genetischen Kontrollsequenzen funktionell verknüpft ist, oder
b) die Expressionskassette zusätzliche Funktionselemente enthält, oder
c) a) und b) gegeben sind.

13. Transgene Expressionskassette nach einem der Ansprüchen 10 bis 12, wobei die transgen zu exprimierende Nukleinsäuresequenz
a) die Expression eines von besagter Nukleinsäuresequenz kodierten Proteins, oder
b) die Expression eines von besagter Nukleinsäuresequenz kodierter sense-RNA, anti-sense-RNA oder doppelsträngigen RNA ermöglicht.

14. Transgene Expressionskassette nach einem der Ansprüche 10 bis 13, wobei die transgen zu exprimierende Nukleinsäuresequenz ausgewählt ist der Gruppe von Nukleinsäuresequenzen kodierend für Chalconsynthasen, Phenylalaninammoniumlyasen, Photolyasen, Deoxyxylulose-5-phosphatsynthasen, Phytoensynthasen, Phytoendesaturasen, Lycopincyclasen, Hydroxylasen, "antifreeze"-Polypeptide, CBF1-Transkriptionsaktivatoren, Glutamatdehydrogenasen, Calcium-abhängigen Proteinkinasen, Calcineurin, Farnesyltransferasen, Ferritin, Oxalatoxidasen, DREBlA-Faktor, Trehalosephosphatphosphatasen, Chitinasen, Glucanasen, Ribosom-inaktivierende Protein, Lysozyme, Bacillus thuringiensis Endotoxine, Amylaseinhibitoren, Proteaseinhibitoren, Lectinen, RNAsen, Ribozymen, Endochitinase, Cytochrom P-450, Acetyl-CoA Carboxylasen, Aminosäure-Transporter, Monosaccharid-Transporter, Lycopincyklasen, Carotinketolasen, Endoxyloglucantransferasen, Δ6-Acyllipiddesaturasen, Δ6-Desaturasen, Δ5-Fettsäuredesaturase, Δ6-Elongasen und IPP-Isomerasen.

15. Transgene Expressionskassette nach einem der Ansprüche 10 bis 14, wobei die transgen zu exprimierende Nukleinsäuresequenz ausgewählt ist der Gruppe von Nukleinsäuresequenzen beschrieben durch GenBank Acc.-No.: M20308, BAB00748, U62549, U77378, S78423, U32624, L25042, X92657, AJ002399, D45881, AF163819, AB044391, AJ222980 und AF078796.

16. Transgener Expressionsvektor enthaltend eine Expressionskassette gemäss einem der Ansprüche 10 bis 15.

17. Transgener Organismus transformiert mit einer Expressionskassette gemäß den Ansprüchen 10 bis 15 oder einem Expressionsvektor gemäß Anspruch 16, wobei der transgene Organismus ein Mikroorganismus oder pflanzlicher Organismus ist oder von diesen abgeleitete Zellen, Zellkulturen, Teile, Gewebe, Organe oder Vermehrungsgut.

18. Transgener Organismus nach Anspruch 17 ausgewählt aus der Gruppe der Blütenpflanzen oder von diesen abgeleitete Zellen, Zellkulturen, Teile, Gewebe, Organe oder Vermehrungsgut.

19. Transgener Organismus nach Anspruch 17 oder 18 ausgewählt aus der Gruppe der landwirtschaftlichen Nutzpflanzen.

20. Verwendung eines transgenen Organismus nach einem der Ansprüche 17 bis 18 oder von diesem abgeleitete abgeleitete Zellen, Zellkulturen, Teile, Gewebe, Organe oder Vermehrungsgut zur Herstellung von Nahrungs-, Futtermitteln, Saatgut, Pharmazeutika oder Feinchemikalien.

21. Verfahren zur Herstellung von Pharmazeutika oder Feinchemikalien in transgenen Organismen nach einem der Ansprüche 17 bis 19 oder von diesem abgeleitete abgeleitete Zellen, Zellkulturen, Teile, Gewebe, Organe oder Vermehrungsgut, wobei der transgene Organismus oder von diesem abgeleitete Zellen, Zellkulturen, Teile, Gewebe, Organe oder Vermehrungsgut gezüchtet oder kultiviert werden und das gewünschte Pharmazeutikon oder die gewünschte Feinchemikalie isoliert werden.

## Claims

1. A method for the targeted transgenic expression of nucleic acid sequences in nonreproductive floral tissues of plants, comprising the following steps
I. introduction of a transgenic expression cassette into plant cells, where the transgenic expression cassette comprises at least the following elements
a) at least one promoter sequence, mediating an expression of all nonreproductive floral tissues, but essentially not in the pollen and the ovaries, selected from the group of sequences consisting of
i) the promoter sequences of SEQ ID NO: 1 or 2 and
ii) functional equivalents of the promoter sequences of SEQ ID NO: 1 or 2 with essentially the same promoter activity as a promoter of SEQ ID NO: 1 or 2 and which have an identity of at least 80% to the sequence of one of the promoters of SEQ ID NO: 1 or 2, and
b) at least one further nucleic acid sequence, and
c) where appropriate further genetic control elements,
where at least one promoter sequence and one further nucleic acid sequence are functionally linked together, and the further nucleic acid sequence is heterologous in relation to the promoter sequence, and
II. selection of transgenic cells which comprise said expression cassette stably integrated into the genome, and
III. regeneration of complete plants from said transgenic cells, where at least one of the further nucleic acid sequences is expressed essentially in all nonreproductive floral tissues, but essentially not in the pollen and the ovaries.

2. The method according to claim 1, where the functionally equivalent fragment comprises a sequence as shown in SEQ ID NO: 3 or 4.

3. A method for producing a transgenic expression cassette having specificity for nonreproductive floral tissue, comprising the following steps:
I. isolation of a promoter with specificity for nonreproductive floral tissue, where at least one nucleic acid sequence or a part thereof is employed in the isolation, where said nucleic acid sequence encodes an amino acid sequence which comprises at least one sequence of SEQ ID NO: 12, 14, 16, 18, 20, 22, 23, 24, 25, 26, 27, 28, 29, 30, 31 or 32 or which comprises at least one sequence having a homology of at least 80% with a sequence of SEQ ID No: 12 or 14, and
II. functional linkage of said promoter with a further nucleic acid sequence, where said nucleic acid sequence is heterologous in relation to the promoter.

4. The method according to claim 3, where said nucleic acid sequence comprises a sequence as shown in SEQ ID NO: 5, 7, 11, 13, 15, 17, 19 or 21.

5. The method according to either of claims 3 or 4, where the method is carried out with use of the polymerase chain reaction, and said nucleic acid sequence or a part thereof is employed as primer.

6. The use of at least one nucleic acid sequence or a part thereof in methods for identifying and/or isolating promoters with specificity for nonreproductive floral tissue, where said nucleic acid sequence encodes an amino acid sequence which comprises at least one sequence of SEQ ID NO: 12, 14, 16, 18, 20, 22, 23, 24, 25, 26, 27, 28, 29, 30, 31 or 32 or which comprises at least one sequence having a homology of at least 80% with a sequence of SEQ ID No: 12 or 14.

7. The use according to claim 6, where said nucleic acid sequence comprises a sequence of SEQ ID NO: 5, 7, 11, 13, 15, 17, 19 or 21.

8. The method according to one of claims 3 to 5, or use according to claim 6 or 7, where said nucleic acid sequence encodes an amino acid sequence which comprises at least one sequence having a homology of at least 90% with a sequence of SEQ ID No: 12 or 14.

9. The method according to one of claims 3 to 5, 8 or use according to claim 6 or 7, where said nucleic acid sequence encodes an amino acid sequence which comprises at least one sequence having a homology of at least 95% with a sequence of SEQ ID No: 12 or 14.

10. A transgenic expression cassette for the targeted transgenic expression of nucleic acid sequences in nonreproductive floral tissues of plants, comprising
a) at least one promoter sequence, mediating an expression of all nonreproductive floral tissues, but essentially not in the pollen and the ovaries, selected from the group of sequences consisting of
i) the promoter sequences of SEQ ID NO: 1 or 2 and
ii) functional equivalents of the promoter sequences of SEQ ID NO: 1 or 2 with essentially the same promoter activity as a promoter of SEQ ID NO: 1 or 2 and which have an identity of at least 80% to the sequence of one of the promoters of SEQ ID NO: 1 or 2, and
b) at least one further nucleic acid sequence, and
c) where appropriate further genetic control elements,
where at least one promoter sequence and one further nucleic acid sequence are functionally linked together, and the further nucleic acid sequence is heterologous in relation to the promoter sequence.

11. The transgenic expression cassette according to claim 10, where the functionally equivalent fragment comprises a sequence of SEQ ID NO: 3 or 4.

12. The transgenic expression cassette according to claim 10 or 11, where
a) the nucleic acid sequence to be expressed is functionally linked with further genetic control sequences, or
b) the expression cassette comprises additional functional elements, or
c) a) and b) apply.

13. The transgenic expression cassette according to one of claims 10 to 12, where the nucleic acid sequence to be expressed transgenically makes possible
a) the expression of a protein encoded by said nucleic acid sequence, or
b) the expression of a sense-RNA, anti-sense-RNA or double-stranded RNA encoded by said nucleic acid sequence.

14. The transgenic expression cassette according to one of claims 10 to 13, where the nucleic acid sequence to be expressed transgenically is selected from the group of nucleic acid sequences encoding chalcone synthases, phenylalanine ammonium lyases, photolyases, deoxyxylulose-5-phosphate synthases, phytoene synthases, phytoene desaturases, lycopene cyclases, hydroxylases, "antifreeze" polypeptides, CBF1-transcription activators, glutamate dehydrogenases, calcium-dependent protein kinases, calcineurin, farnesyltransferases, ferritin, oxalate oxidases, DREBlA factor, trehalose-phosphate phosphatases, chitinases, glucanases, ribosome-inactivating protein, lysozyme, Bacillus thuringiensis endotoxins, amylase inhibitors, protease inhibitors, lectins, RNAses, ribozymes, endochitinase, cytochrome P-450, acetyl-CoA carboxylases, amino acid transporters, monosaccharide-transporters, lycopine cyclases, carotene ketolases, endoxyloglucan transferases, Δ6-acyllipid desaturases, Δ6-desaturases, Δ5-fatty acid desaturases, Δ6-elongases and IPP-isomerases.

15. The transgenic expression cassette according to one of claims 10 to 14, where the nucleic acid sequence to be expressed trangenically is selected from the group of nucleic acid sequences described by GenBank Acc.-No.: M20308, BAB00748, U62549, U77378, S78423, U32624, L25042, X92657, AJ002399, D45881, AF163819, AB044391, AJ222980 and AF078796.

16. A transgenic expression vector comprising an expression cassette of one of claims 10 to 15.

17. A transgenic organism, transformed with an expression cassette of claims 10 to 15 or an expression vector of claim 16, where the transgenic organism is a microorganism or plant organism or comprises cells, cell cultures, parts, tissues, organs or propagation material derived therefrom.

18. The transgenic organism according to claim 17 selected from the group consisting of flowering plants or of cells, cell cultures, parts, tissues, organs or propagation material derived therefrom.

19. The transgenic organism according to claim 17 or 18 selected from the group of agricultural crop plants.

20. The use of a transgenic organism according to one of claims 17 to 18 or cells, cell cultures, parts, tissues, organs or propagation material derived therefrom to produce human or animal foods, seeds, pharmaceuticals or fine chemicals.

21. A method for producing pharmaceuticals or fine chemicals in transgenic organisms according to one of claims 17 to 19 or cells, cell cultures, parts, tissues, organs or propagation material derived therefrom, where the transgenic organism or cells, cell cultures, parts, tissues, organs or propagation material derived therefrom is/are cultured or grown, and the desired pharmaceutical or the desired fine chemical is isolated.

## Revendications

1. Procédé pour l'expression transgénique ciblée de séquences d'acide nucléique dans des tissus floraux non reproducteurs de plantes, dans lequel sont comprises les étapes suivantes
I. introduction d'une cassette d'expression transgénique dans des cellules végétales, la cassette d'expression transgénique contenant au moins les éléments suivants
a) au moins une séquence de promoteur, induisant une expression dans tous les tissus floraux non reproducteurs mais essentiellement non dans le pollen ni les ovaires, choisie dans le groupe de séquences constitué par
i) les séquences de promoteurs selon SEQ ID n° 1 ou 2 et
ii) des équivalents fonctionnels des séquences de promoteurs selon SEQ ID n° 1 ou 2 ayant pratiquement la même activité de promoteur qu'un promoteur selon SEQ ID n° 1 ou 2 et qui présentent une identité d'au moins 80 % avec la séquence d'un des promoteurs selon SEQ ID n° 1 ou 2 et
b) au moins une autre séquence d'acide nucléique, et
c) éventuellement d'autres éléments régulateurs génétiques,
au moins une séquence de promoteur et une autre séquence d'acide nucléique étant fonctionnellement liées l'une à l'autre et l'autre séquence d'acide nucléique étant hétérologue eu égard à la séquence de promoteur, et
II. sélection de cellules transgéniques qui contiennent ladite cassette d'expression intégrée de façon stable dans le génome, et
III. régénération de plantes complètes à partir desdites cellules transgéniques, au moins l'une des autres séquences d'acide nucléique étant exprimée dans pratiquement tous les tissus floraux non reproducteurs mais essentiellement non dans le pollen ni les ovaires.

2. Procédé selon la revendication 1, dans lequel le fragment équivalent fonctionnel comprend une séquence selon SEQ ID n° 3 ou 4.

3. Procédé pour la production d'une cassette d'expression transgénique à spécificité pour les tissus floraux non reproducteurs, comprenant les étapes suivantes :
I. isolement d'un promoteur à spécificité pour les tissus floraux non reproducteurs, au moins une séquence d'acide nucléique ou une partie de celle-ci étant utilisée dans l'isolement, ladite séquence d'acide nucléique codant pour une séquence d'aminoacides qui comprend au moins une séquence selon SEQ ID n° 12, 14, 16, 18, 20, 22, 23, 24, 25, 26, 27, 28, 29, 30, 31 ou 32 ou qui comprend au moins une séquence ayant une homologie d'au moins 80 % avec une séquence selon SEQ ID n° 12 ou 14, et
II. liaison fonctionnelle dudit promoteur avec une autre séquence d'acide nucléique, ladite séquence d'acide nucléique étant hétérologue eu égard au promoteur.

4. Procédé selon la revendication 3, dans lequel ladite séquence d'acide nucléique comprend une séquence selon SEQ ID n° 5, 7, 11, 13, 15, 17, 19 ou 21.

5. Procédé selon la revendication 3 ou 4, le procédé étant effectué avec utilisation de la réaction d'amplification en chaîne par polymérase et dans lequel on utilise comme amorce ladite séquence d'acide nucléique ou une partie de celle-ci.

6. Utilisation d'au moins une séquence d'acide nucléique ou d'une partie de celle-ci dans des procédés pour l'identification et/ou l'isolement de promoteurs à spécificité pour des tissus floraux non reproducteurs, ladite séquence d'acide nucléique codant pour une séquence d'aminoacides qui comprend au moins une séquence selon SEQ ID n° 12, 14, 16, 18, 20, 22, 23, 24, 25, 26, 27, 28, 29, 30, 31 ou 32 ou qui comprend au moins une séquence ayant une homologie d'au moins 80 % avec une séquence selon SEQ ID n° 12 ou 14.

7. Utilisation selon la revendication 6, dans laquelle ladite séquence d'acide nucléique comprend une séquence selon SEQ ID n° 5, 7, 11, 13, 15, 17, 19 ou 21.

8. Procédé selon l'une quelconque des revendications 3 à 5, ou utilisation selon la revendication 6 ou 7, dans lequel ou laquelle ladite séquence d'acide nucléique code pour une séquence d'aminoacides qui comprend au moins une séquence ayant une homologie d'au moins 90 % avec une séquence selon SEQ ID n° 12 ou 14.

9. Procédé selon l'une quelconque des revendications 3 à 5 et 8 ou utilisation selon la revendication 6 ou 7, dans lequel ou laquelle ladite séquence d'acide nucléique code pour une séquence d'aminoacides qui comprend au moins une séquence ayant une homologie d'au moins 95 % avec une séquence selon SEQ ID n° 12 ou 14.

10. Cassette d'expression transgénique pour l'expression transgénique ciblée de séquences d'acide nucléique dans des tissus floraux non reproducteurs de plantes, comprenant
a) au moins une séquence de promoteur, induisant une expression dans tous les tissus floraux non reproducteurs mais essentiellement non dans le pollen ni les ovaires, choisie dans le groupe de séquences constitué par
i) les séquences de promoteurs selon SEQ ID n° 1 ou 2 et
ii) des équivalents fonctionnels des séquences de promoteurs selon SEQ ID n° 1 ou 2 ayant pratiquement la même activité de promoteur qu'un promoteur selon SEQ ID n° 1 ou 2 et qui présentent une identité d'au moins 80 % avec la séquence d'un des promoteurs selon SEQ ID n° 1 ou 2 et
b) au moins une autre séquence d'acide nucléique, et
c) éventuellement d'autres éléments régulateurs génétiques,
au moins une séquence de promoteur et une autre séquence d'acide nucléique étant fonctionnellement liées l'une à l'autre et l'autre séquence d'acide nucléique étant hétérologue eu égard à la séquence de promoteur.

11. Cassette d'expression transgénique selon la revendication 10, dans laquelle le fragment fonctionnellement équivalent comprend une séquence selon SEQ ID n° 3 ou 4.

12. Cassette d'expression transgénique selon la revendication 10 ou 11, dans laquelle
a) la séquence d'acide nucléique à exprimer est fonctionnellement liée à d'autres séquences régulatrices génétiques, ou
b) la cassette d'expression contient des éléments fonctionnels supplémentaires, ou
c) a) et b) coexistent.

13. Cassette d'expression transgénique selon l'une quelconque des revendications 10 à 12, dans laquelle la séquence d'acide nucléique à exprimer par voie transgénique
a) permet l'expression d'une protéine codée par ladite séquence d'acide nucléique, ou
b) l'expression d'un ARN sens, d'un ARN antisens ou d'un ARN double brin, codé par ladite séquence d'acide nucléique.

14. Cassette d'expression transgénique selon l'une quelconque des 10 à 13, dans laquelle la séquence d'acide nucléique à exprimer est choisie dans le groupe de séquences d'acide nucléique codant pour les chalcone synthases, les phénylalanine ammonium-lyases, les photolyases, les désoxyxylulose-5-phosphate synthases, les phytoène synthases, les phytoène désaturases, les lycopène cyclases, les hydroxylases, les polypeptides "antifreeze", les activateurs de transcription de CBF1, les glutamate déshydrogénases, les protéine kinases dépendant du calcium, la calcineurine, les farnésyltransférases, la ferritine, les oxalate oxydases, le facteur DREB1A, les tréhalose-phosphate phosphatases, les chitinases, les glucanases, la protéine inactivant les ribosomes, le lysozyme, les endotoxines de *Bacillus thuringiensis*, les inhibiteurs d'amylase, les inhibiteurs de protéases, les lectines, les RNases, les ribozymes, l'endochitinase, le cytochrome P-450, les acétyl-CoA carboxylases, les transporteurs d'aminoacides, les transporteurs de monosaccharides, les lycopène cyclases, les carotène cétolases, les endoxyloglucane transférases, les D6-acyllipide désaturases, les D6-désaturases, les D5-acide gras désaturases, les D6-élongases et les IPP-isomérases.

15. Cassette d'expression transgénique selon l'une quelconque des revendications 10 à 14, dans laquelle la séquence d'acide nucléique à exprimer par voie transgénique est choisie dans le groupe des séquences d'acide nucléique décrites par les numéros d'accès GenBank : M20308, BAB00748, U62549, U77378, S78423, U32624, L25042, X92657, AJ002399, D45881, AF163819, AB044391, AJ222980 et AF078796.

16. Vecteur d'expression transgénique contenant une cassette d'expression selon l'une quelconque des revendications 10 à 15.

17. Organisme transgénique transformé par une cassette d'expression selon les revendications 10 à 15 ou par un vecteur d'expression selon la revendication 16, l'organisme transgénique étant un micro-organisme ou un organisme végétal ou des cellules, cultures de cellules, parties, tissus, organes ou matériel de multiplication de celui-ci.

18. Organisme transgénique selon la revendication 17, choisi dans le groupe des plantes à fleur ou des cellules, cultures de cellules, parties, tissus, organes ou matériel de multiplication de celles-ci.

19. Organisme transgénique selon la revendication 17 ou 18, choisi dans le groupe des plantes utiles en agriculture.

20. Utilisation d'un organisme transgénique selon l'une quelconque des revendications 17 à 18 ou de cellules, cultures de cellules, parties, tissus, organes ou matériel de multiplication dérivés de celui-ci, pour la production de produits alimentaires, d'aliments pour animaux, de semences, de produits pharmaceutiques ou de produits de chimie fine.

21. Procédé pour la production de produits pharmaceutiques ou de produits de chimie fine dans des organismes transgéniques selon l'une quelconque des revendications 17 à 19 ou des cellules, cultures de cellules, parties, tissus, organes ou matériel de multiplication dérivés de ceux-ci, dans lequel on élève ou cultive l'organisme transgénique ou des cellules, cultures de cellules, parties, tissus, organes ou matériel de multiplication de celui-ci et on isole le produit pharmaceutique désiré ou le produit de chimie fine désiré.


### SEQUENZPROTOKOLL

<110> SunGene GmbG & Co.KGaA
<120> Transgene Expressionskassetten zur Expression von Nukleinsäuren in nicht-reproduktiven Blütengeweben von Pflanzen
<130> PF53896-AT
<140>
   <141>
<160> 36
<170> PatentIn Ver. 2.1
<210> 1
   <211> 2039
   <212> DNA
   <213> Arabidopsis thaliana
<220>
   <221> promoter
   <222> (1)..(2039)
   <223> 76L promoter (including 5'-untranslated region) of genelocus At3g01980
<400> 1
   tatcctctgc gcaatgaatt caacaaacgt tttacacaaa gaaaaaaacc tgttgaaaat 60
   gaaagaaaaa aagtgatgaa atacattaga aaccttttgg atatagtcaa ggacttgagt 120
   gtctctgatc ttattgccat cttggtcgat gactttgaaa acgtccatga accaacctcc 180
   atcggaagag atgtaagctt tcttgatcac aagattcata tcagtgagca cttgtactac 240
   ttccaataga gtcccatgtt tgttcacact atcaaccttt tatttagcac cacaaacaaa 300
   cagggaactc tgttttctca aaatctcttt gtttggtaca gaaccacttg aacaatttga 360
   agagacagat ttatttacct gaataacagt ggcatcgtcg cttgcgttgt tgtctattac 420
   aactctgtat ccaccatgaa acatacataa ataaagatga atgttttagc agctggaatc 480
   tttacaaggg tatagaggct cacaaacgga gcatggagaa acagaggacc tagaaaacaa 540
   taaaagagta tggagagaaa gagaaaaacc tgggaggatt cattctcctg atgagcttgg 600
   catattcatc atcatccatg gttgtagtat gtatctcctt ggtgatttgg ctgcagcaga 660
   aacccagaag agtgtgagtc ctccaatttc tgaggaaaat tcctaaaaga gagagggaga 720
   gggaagttga aggaggataa aaatggtagg ccaccggaac cgaaccttgt tttcattagt 780
   tgatcgagca cgtgccacta aagattctta gagatgacgt ggcacgggca cagcaacttt 840
   tagattctgt tataattgtt cgaatactac caaaagtcgg gtgaagattt ggggtcaatt 900
   tgatgatcat aaaggggatt atattctcct tctcaagcaa gatgtggtat ttactagtat 960
   aatagatcat tcgttatctt gaggtagacc tctccgtaac gttcacaggt gcatgaccaa 1020
   gtaacaattt gattcctttc cagcataacg tcatgttggt tgcaaaaaga aggcaaagta 1080
   gagcaagcaa gcaagcaaag catttttctt attttatatt ttgttgcgga ttccaccacc 1140
   cacttgaaaa attgacatgt cacaatgatt tcgtatccta gtcttttatt atttaacact 1200
   ctcacaatcc cattactcta cacctctttc attaagtcaa cacacggttt tcaaaaatcc 1260
   actaccctcc caccacctag aatcttttgt tacctaccaa caccctcctt tgttctcttt 1320
   atatattggt ccaactaaat caataaggga aagcatcctt ttggttggag gaattgcttt 1380
   cattctcact ctttgtgtgt tgatcaatgg actagctaat aacaagttcc tcctctatat 1440
   atttcaaaag aatggaacag aaacataaac gaaagacaga gtacctgatg ttgatgattc 1500
   attgtctgtc tggagctccc aaatgccttt tatgcttaca tattcataac caacaacggc 1560
   tattaattat aaaccaaaaa cacgaaataa gtttgtagca aagtgaaatt aggaatcttg 1620
   gagatggatc cattagtagt aggataatag gatatgatgg aatttggttg gggaacagtg 1680
   ataacttacg cttgcttccg gcgccgggaa agttggaaaa cctacaaagt acagaaatgg 1740
   atctgggcct tgaagtgggc tttttattaa agaaaaaaat acatctccgt tatcaatcac 1800
   catcttcttc tatctacaaa ttaaagaagg taacaacaga acgtggtgga tcatgtggtt 1860
   aggcattaat tatttgcttt gtttcgccgt tttggtaaca cacagacaca gttccggtaa 1920
   gagcttttgc agccactctt tatagttatt tagaattggc gatcgaatca atctcactcc 1980
   ctccctccct taagtcttgt tgaatctgct gaattgtttt ataaagagtt actttggca  2039
<210> 2
   <211> 2180
   <212> DNA
   <213> Arabidopsis thaliana
<220>
   <221> promoter
   <222> (1)..(2180)
   <223> 84L promoter (including 5'-untranslated region) of genelocus At1g63140
<400> 2
   catgtttcag aggatatgtt cacaacaatt ccaaaagcag atgccatctt catgaaagta 60
   aactcatata tatttcattc atggaaaagt ttaatcaata acaaatacta tcttcatttc 120
   ataaaagcct tgtttcagat tttttcacac acatttaaaa gtgttttaag tttatcttat 180
   acctttaagt acatttatga tttttttttt ctcctattaa ataagctgaa agattataga 240
   taaatggttt tgttttatta aaaatgatgc attgaaaata taaaaggaca ttttatatat 300
   agatactaca tgattaggcc gatggaacca aagatgagcg actcttcttg taacattgtg 360
   tttgctacgc aatgctcggt tttttttttc ttagatcgag actttgcctg agattctggt 420
   tttcttcgat tgtgaaatca tatatgtctt gccttctcat ataggttcaa cattgaccaa 480
   caaaaactac gggtggttta gttttttttg ggtggagagt ggagactaac cttgaccttt 540
   ttcatttgta atgatttttc tttcttgtat gaactattgt ttgtttattg gcgctgttca 600
   tttgttccga gtgttttcgt atatgcttta aacaagcacg tactatcagc aatagcaaaa 660
   gtaacatgat atttgttaat cccgttggaa attcatgtcc attattttgt atatatatat 720
   attatataat agtagaattt ggttatgtag tgcatattct ctaaatctat gctttctaag 780
   gttaaaaaca ggcgcccata tggacgacat aaatgtcgat atttaagagg cactgcaagt 840
   tgaacaaaaa aaaaaaagta taggcactgc aaaagttatc caacgtattt aagactaagg 900
   actaaagatt caaagataat attcagaaaa agaaaaagaa aaaaagagaa gataatattc 960
   ggaaacatcc acaagcattc taaatctaga aaacataaat aatacagcaa agatggggat 1020
   gaagatatga tccaactcca tcacagattc tcaagacaga tttagaaagt gtcaagctca 1080
   ccaaaagggt tataggagac tgactgttaa ttgaaatgct ttctacacgt ggacgcactg 1140
   atatcatatt aaaacctgat tgtttgttga acattcacta actcatacca aacggtccaa 1200
   acctatgtct ccattttctt aaatgttgat ttcgattcca tacctacttt gcatacatta 1260
   ttgaatgtgt ttcttaagtt gtgattaaaa ttaaatgagc acaatatcac agtcgaatgg 1320
   tatatcgatg taacacttta ggattgaatc aatatgaaaa gttatacacc gaatttgtga 1380
   gaaacgagta tagcttagac aaaatttgtt tttcttaaat taagcggaaa aataattaaa 1440
   cagagaccaa attaagcgtt cttcttgaac tgaaatcact aaagtaaagt taacccgtta 1500
   gtagagtgtt aactatttaa acaaagaaaa ctccaaaccc aattgagaaa ctactcaaac 1560
   atagaaacaa cacataatga ttcagtagct accaatatca tattcaactt tgtttcgatt 1620
   cctttaaaac aaaatataat taaccaaata aaataggtca taatcgattc agaaacaatt 1680
   tcatattctt ctctagttta gttcagtttc attctaccgg agttgtatac aatctataat 1740
   tttatcgctt attaccttaa aagcgtcctc aaaccaacca aaacaaaaat agttgcatca 1800
   atgaatccat caaagcatat aaattcacac cgtcttaaaa tggagtgttg atggataagt 1860
   accaacaatt ttagaccatt cacactgaat gagtatgact aacattcaca ttcacattca 1920
   attaggaaag ttgtactaat gaacacacaa taaaagtgaa aacaaatctc tacatattct 1980
   tgtacaccaa tctatattag atgatcattt taaatataca cgaatattaa ttttataaat 2040
   gaaaaatacg tgcccatatt ttaattaatt tatatattta gctatcaaat attaggcata 2100
   atgttggtga ggtttctgag tataaaaaat gacaaagtat gaataccatc tataccttta 2160
   ttacctatct ttctcgattt 2180
<210> 3
   <211> 1033
   <212> DNA
   <213> Arabidopsis thaliana
<220>
   <221> promoter
   <222> (1)..(1033)
   <223> 76S promoter (including 5'-untranslated region) of genelocus At3g01980
<400> 3
   aggtgcatga ccaagtaaca atttgattcc tttccagcat aacgtcatgt tggttgcaaa 60
   aagaaggcaa agtagagcaa gcaagcaagc aaagcatttt tcttatttta tattttgttg 120
   cggattccac cacccacttg aaaaattgac atgtcacaat gatttcgtat cctagtcttt 180
   tattatttaa cactctcaca atcccattac tctacacctc tttcattaag tcaacacacg 240
   gttttcaaaa atccactacc ctcccaccac ctagaatctt ttgttaccta ccaacaccct 300
   cctttgttct ctttatatat tggtccaact aaatcaataa gggaaagcat ccttttggtt 360
   ggaggaattg ctttcattct cactctttgt gtgttgatca atggactagc taataacaag 420
   ttcctcctct atatatttca aaagaatgga acagaaacat aaacgaaaga cagagtacct 480
   gatgttgatg attcattgtc tgtctggagc tcccaaatgc cttttatgct tacatattca 540
   taaccaacaa cggctattaa ttataaacca aaaacacgaa ataagtttgt agcaaagtga 600
   aattaggaat cttggagatg gatccattag tagtaggata ataggatatg atggaatttg 660
   gttggggaac agtgataact tacgcttgct tccggcgccg ggaaagttgg aaaacctaca 720
   aagtacagaa atggatctgg gccttgaagt gggcttttta ttaaagaaaa aaatacatct 780
   ccgttatcaa tcaccatctt cttctatcta caaattaaag aaggtaacaa cagaacgtgg 840
   tggatcatgt ggttaggcat taattatttg ctttgtttcg ccgttttggt aacacacaga 900
   cacagttccg gtaagagctt ttgcagccac tctttatagt tatttagaat tggcgatcga 960
   atcaatctca ctccctccct cccttaagtc ttgttgaatc tgctgaattg ttttataaag 1020
   agttactttg gca                                                    1033
<210> 4
   <211> 1097
   <212> DNA
   <213> Arabidopsis thaliana
<220>
   <221> promoter
   <222> (1)..(1097)
   <223> 84S promoter (including 5'-untranslated region) of genelocus At1g63140
<400> 4
   aaagggttat aggagactga ctgttaattg aaatgctttc tacacgtgga cgcactgata 60
   tcatattaaa acctgattgt ttgttgaaca ttcactaact cataccaaac ggtccaaacc 120
   tatgtctcca ttttcttaaa tgttgatttc gattccatac ctactttgca tacattattg 180
   aatgtgtttc ttaagttgtg attaaaatta aatgagcaca atatcacagt cgaatggtat 240
   atcgatgtaa cactttagga ttgaatcaat atgaaaagtt atacaccgaa tttgtgagaa 300
   acgagtatag cttagacaaa atttgttttt cttaaattaa gcggaaaaat aattaaacag 360
   agaccaaatt aagcgttctt cttgaactga aatcactaaa gtaaagttaa cccgttagta 420
   gagtgttaac tatttaaaca aagaaaactc caaacccaat tgagaaacta ctcaaacata 480
   gaaacaacac ataatgattc agtagctacc aatatcatat tcaactttgt ttcgattcct 540
   ttaaaacaaa atataattaa ccaaataaaa taggtcataa tcgattcaga aacaatttca 600
   tattcttctc tagtttagtt cagtttcatt ctaccggagt tgtatacaat ctataatttt 660
   attgcttatt accttaaaag cgtcctcaaa ccaaccaaaa caaaaatagt tgcatcaatg 720
   aatccatcaa agcatataaa ttcacaccgt cttaaaatgg agtgttgatg gataagtacc 780
   aacaatttta gaccattcac actgaatgag tatgactaac attcacattc acattcaatt 840
   aggaaagttg tactaatgaa cacacaataa aagtgaaaac aaatctctac atattcttgt 900
   acaccaatct atattagatg atcattttaa atatacacga atattaattt tataaatgaa 960
   aaatacgtgc ccatatttta attaatttat atatttagct atcaaatatt aggcataatg 1020
   ttggtgaggt ttctgagtat aaaaaatgac aaagtatgaa taccatctat acctttatta 1080
   cctatctttc tcgattt                                                1097
<210> 5
   <211> 25
   <212> DNA
   <213> Künstliche Sequenz
<220>
   <223> Beschreibung der künstlichen Sequenz: Oligonucleotide primer
<400> 5
   gaccctgtcc cacctccaa 19
<210> 6
   <211> 25
   <212> DNA
   <213> Künstliche Sequenz
<220>
   <223> Beschreibung der künstlichen Sequenz: Oligonucleotide primer
<400> 6
   tgägaactgc gattgtttgc a 21
<210> 7
   <211> 25
   <212> DNA
   <213> Künstliche Sequenz
<220>
   <223> Beschreibung der künstlichen Sequenz: Oligonucleotide primer
<400> 7
   gtcgactatc ctctgcgcaa tgaat 25
<210> 8
   <211> 25
   <212> DNA
   <213> Künstliche Sequenz
<220>
   <223> Beschreibung der künstlichen Sequenz: Oligonucleotide primer
<400> 8
   cccgggaaat cgagaaagat aggta 25
<210> 9
   <211> 25
   <212> DNA
   <213> Künstliche Sequenz
<220>
   <223> Beschreibung der künstlichen Sequenz: Oligonucleotide primer
<400> 9
   gtcgacaaag ggttatagga gactg 25
<210> 10
   <211> 25
   <212> DNA
   <213> Künstliche Sequenz
<220>
   <223> Beschreibung der künstlichen Sequenz: Oligonucleotide primer
<400> 10
   gtcgaccatg tttcagagga tatgt 25
<210> 11
   <211> 1187
   <212> DNA
   <213> Arabidopsis thaliana
<220>
   <221> CDS
   <222> (132)..(764)
   <223> coding for gene product of Arabidopsis thaliana gene locus At3g01980
<400> 11
   gttccggtaa gagcttttgc agccactctt tatagttatt tagaattggc gatcgaatca 60
   atctcactcc ctccctccct taagtcttgt tgaatctgct gaattgtttt ataaagagtt 120
   actttggcaa a atg gaa aat ccg gcg aag aga gtg ttg atg aca tcc aac  170
                Met Glu Asn Pro Ala Lys Arg Val Leu Met Thr Ser Asn
                1               5                   10
   ggc gac gag gtg tcc cga aac atc gct ttc cat cta gcc aaa cac ggt  218
   Gly Asp Glu Val Ser Arg Asn Ile Ala Phe His Leu Ala Lys His Gly
       15                  20                  25
   tgc aag ttg gta atg atg gga aat gag ggt tcc cta agg agc att gta  266
   Cys Lys Leu Val Met Met Gly Asn Glu Gly Ser Leu Arg Ser Ile Val
   30                  35                  40                  45
   gac aag att aga gat tcc att gag gga gcc ttc cct gcc gat gtt ata  314
   Asp Lys Ile Arg Asp Ser Ile Glu Gly Ala Phe Pro Ala Asp Val Ile
                   50                  55                  60
   gca ctc gac atg gaa tct gac tct gaa gtt gct ttt cat gcc gct gtc  362
   Ala Leu Asp Met Glu Ser Asp Ser Glu Val Ala Phe His Ala Ala Val
               65                  70                  75
   caa aag gca tgg gaa ctt tcc ggc cat ttc gat gct ttt ctc aac tct  410
   Gln Lys Ala Trp Glu Leu Ser Gly His Phe Asp Ala Phe Leu Asn Ser
           80                  85                  90
   tat acc tac caa ggt tta att tgc ttc ttg ttt ttc act acc ctg cct  458
   Tyr Thr Tyr Gln Gly Leu Ile Cys Phe Leu Phe Phe Thr Thr Leu Pro
       95                  100                 105
   ttg atg ctc ttg tgt gtt gat cat tcc ttt att caa caa tct ttc ttt  506
   Leu Met Leu Leu Cys Val Asp His Ser Phe Ile Gln Gln Ser Phe Phe
   110                 115                 120                 125
   ctt gca gga aag gtg cag gac att ctt caa gtc tct caa gat gag ttc  554
   Leu Ala Gly Lys Val Gln Asp Ile Leu Gln Val Ser Gln Asp Glu Phe
                   130                 135                 140
   cac aga atc aca aag atc aat ctc acc gct cca tgg ttt ctc ttg aag  602
   His Arg Ile Thr Lys Ile Asn Leu Thr Ala Pro Trp Phe Leu Leu Lys
               145                 150                 155
   gct gta gcc aca agg atg aag gac cat gga tca gga ggc tcc att gtc  650
   Ala Val Ala Thr Arg Met Lys Asp His Gly Ser Gly Gly Ser Ile Val
           160                 165                 170
   ttc atg gcc act atc gcc agc gga gag agg gcg ctt tac cct ggc gct  698
   Phe Met Ala Thr Ile Ala Ser Gly Glu Arg Ala Leu Tyr Pro Gly Ala
       175                 180                 185
   gat gcc tac gct tca act tct gcc gct att cac cag ctc gtc cgg gta  746
   Asp Ala Tyr Ala Ser Thr Ser Ala Ala Ile His Gln Leu Val Arg Val
   190                 195                 200                 205
   tgc atc cta gct cct aat tagacacatc gcgttcgtaa cttgaatatg         794
   Cys Ile Leu Ala Pro Asn
                   210
   tttgttgatg attgggtttc aggcatcagc catgagtctc gggaagcaca agatacgggt 854
   caacatgatc tctagagggc tgcatctgga tgatgagtat acagcttctg tgggaagaga 914
   ccgagcgcag aagctggtca aggacgctgc acccctcggc cagtggctca acccggacac 974
   agacctctac tccactgtta tctacttgat cagcgatggc tcacgcttca tgacaggcac 1034
   cactgtcttg gtggatggag cgcagtccct tacgcgaccc cgtctcaaat cctacatgtg 1094
   atcaatgcct agtattatta taattctatg ttgtgtgtaa aaagtgaata tgaatcaagt 1154
   ttgaataact atggagggat gaataatcca tcc                             1187
<210> 12
   <211> 211
   <212> PRT
   <213> Arabidopsis thaliana
<400> 12
   Met Glu Asn Pro Ala Lys Arg Val Leu Met Thr Ser Asn Gly Asp Glu
   1               5                   10                  15
   Val Ser Arg Asn Ile Ala Phe His Leu Ala Lys His Gly Cys Lys Leu
               20                  25                  30
   Val Met Met Gly Asn Glu Gly Ser Leu Arg Ser Ile Val Asp Lys Ile
           35                  40                  45
   Arg Asp Ser Ile Glu Gly Ala Phe Pro Ala Asp Val Ile Ala Leu Asp
       50                  55                  60
   Met Glu Ser Asp Ser Glu Val Ala Phe His Ala Ala Val Gln Lys Ala
   65                  70                  75                  80
   Trp Glu Leu Ser Gly His Phe Asp Ala Phe Leu Asn Ser Tyr Thr Tyr
                   85                  90                  95
   Gln Gly Leu Ile Cys Phe Leu Phe Phe Thr Thr Leu Pro Leu Met Leu
               100                 105                 110
   Leu Cys Val Asp His Ser Phe Ile Gln Gln Ser Phe Phe Leu Ala Gly
           115                 120                 125
   Lys Val Gln Asp Ile Leu Gln Val Ser Gln Asp Glu Phe His Arg Ile
       130                 135                 140
   Thr Lys Ile Asn Leu Thr Ala Pro Trp Phe Leu Leu Lys Ala Val Ala
   145                 150                 155                 160
   Thr Arg Met Lys Asp His Gly Ser Gly Gly Ser Ile Val Phe Met Ala
                   165                 170                 175
   Thr Ile Ala Ser Gly Glu Arg Ala Leu Tyr Pro Gly Ala Asp Ala Tyr
               180                 185                 190
   Ala Ser Thr Ser Ala Ala Ile His Gln Leu Val Arg Val Cys Ile Leu
           195                 200                 205
   Ala Pro Asn
       210
<210> 13
   <211> 1146
   <212> DNA
   <213> Arabidopsis thaliana
<220>
   <221> CDS
   <222> (1)..(1143)
   <223> coding for transcript (cDNA) of genelocus At1g63140
<400> 13
   atg gag aac cat ctt caa cat tcc tta acc atc att cct aaa ccg gat    48
   Met Glu Asn His Leu Gln His Ser Leu Thr Ile Ile Pro Lys Pro Asp
    1               5                  10                  15
   cta atc aaa gaa gaa caa cgt tat cac gaa gat acg gtg agc ttg caa    96
   Leu Ile Lys Glu Glu Gln Arg Tyr His Glu Asp Thr Val Ser Leu Gln
               20                  25                  30
   gcg gag agg att ttg cat gcc atg acc ttc ccc atg gtt ctc aaa act    144
   Ala Glu Arg Ile Leu His Ala Met Thr Phe Pro Met Val Leu Lys Thr
           35                  40                  45
   gct ttg gag ctt ggc gtt atc gac atg atc act tct gta gat gac ggc    192
   Ala Leu Glu Leu Gly Val Ile Asp Met Ile Thr Ser Val Asp Asp Gly
       50                  55                  60
   gtg tgg ctc tcg cct tct gag atc gct ctt ggt ctc cca acc aag ccc    240
   Val Trp Leu Ser Pro Ser Glu Ile Ala Leu Gly Leu Pro Thr Lys Pro
   65                  70                  75                  80
   acc aat ccg gag gca cca gta ttg ctg gac cgg atg cta gtt ttg tta    288
   Thr Asn Pro Glu Ala Pro Val Leu Leu Asp Arg Met Leu Val Leu Leu
                   85                  90                  95
   gcc agc cac tca atc ttg aag tac cgt acg gta gaa acc gga gat aac    336
   Ala Ser His Ser Ile Leu Lys Tyr Arg Thr Val Glu Thr Gly Asp Asn
               100                 105                 110
   att gga agt aga aag acc gag agg gtc tat gca gct gaa ccg gtt tgc    384
   Ile Gly Ser Arg Lys Thr Glu Arg Val Tyr Ala Ala Glu Pro Val Cys
           115                 120                 125
   acg ttt ttc ttg aac cgc gga gat ggc ttg ggc tct ctc gcc act ttg    432
   Thr Phe Phe Leu Asn Arg Gly Asp Gly Leu Gly Ser Leu Ala Thr Leu
       130                 135                 140
   ttc atg gta ctc caa ggg gaa gtc tgt atg aag cct tgg gaa cat ctc    480
   Phe Met Val Leu Gln Gly Glu Val Cys Met Lys Pro Trp Glu His Leu
   145                 150                 155                 160
   aaa gac atg ata tta gaa gga aaa gat gca ttc acc tct gct cat ggc    528
   Lys Asp Met Ile Leu Glu Gly Lys Asp Ala Phe Thr Ser Ala His Gly
                   165                 170                 175
   atg agg ttt ttc gaa ctc att ggt tcg aac gaa caa ttc gct gaa atg    576
   Met Arg Phe Phe Glu Leu Ile Gly Ser Asn Glu Gln Phe Ala Glu Met
               180                 185                 190
   ttt aac cgg gca atg tcg gaa gct tcc aca ttg att atg aag aag gtt    624
   Phe Asn Arg Ala Met Ser Glu Ala Ser Thr Leu Ile Met Lys Lys Val
           195                 200                 205
   tta gaa gtt tac aaa gga ttc gaa gat gta aat act ttg gtg gat gtg    672
   Leu Glu Val Tyr Lys Gly Phe Glu Asp Val Asn Thr Leu Val Asp Val
       210                 215                 220
   gga gga gga att gga aca atc ata ggt caa gtg act tcc aag tat cct    720
   Gly Gly Gly Ile Gly Thr Ile Ile Gly Gln Val Thr Ser Lys Tyr Pro
   225                 230                 235                 240
   cat att aaa ggc atc aat ttc gat cta gca tcg gtt tta gcc cat gct    768
   His Ile Lys Gly Ile Asn Phe Asp Leu Ala Ser Val Leu Ala His Ala
                   245                 250                 255
   cct ttt aat aaa gga gtg gag cat gtt tca gga gat atg ttt aaa gaa     816
   Pro Phe Asn Lys Gly Val Glu His Val Ser Gly Asp Met Phe Lys Glu
               260                 265                 270
   att cca aaa gga gat gcc atc ttc atg aaa tgg ata cta cat gat tgg     864
   Ile Pro Lys Gly Asp Ala Ile Phe Met Lys Trp Ile Leu His Asp Trp
           275                 280                 285
   act gac gaa gat tgt gta aag atc cta aaa aat tat tgg aaa agt ctt     912
   Thr Asp Glu Asp Cys Val Lys Ile Leu Lys Asn Tyr Trp Lys Ser Leu
       290                 295                 300
   ccc gag aaa gga aaa gtg ata ata gtc gag gtg gtt acg ccc gag gaa     960
   Pro Glu Lys Gly Lys Val Ile Ile Val Glu Val Val Thr Pro Glu Glu
   305                 310                 315                 320
   cca aag att aac gac att tct tct aac att gtg ttc ggt atg gac atg     1008
   Pro Lys Ile Asn Asp Ile Ser Ser Asn Ile Val Phe Gly Met Asp Met
                   325                 330                 335
   ctg atg tta gca gta agc tca ggt ggt aag gag agg tct ctt tcc caa     1056
   Leu Met Leu Ala Val Ser Ser Gly Gly Lys Glu Arg Ser Leu Ser Gln
               340                 345                 350
   ttc gag act cta gcc tct gat tcg ggt ttt ctt cgt tgt gaa atc att     1104
   Phe Glu Thr Leu Ala Ser Asp Ser Gly Phe Leu Arg Cys Glu Ile Ile
           355                 360                 365
   tgt cat gcc ttc tca tat agt gtt atc gaa tta cac aaa tag             1146
   Cys His Ala Phe Ser Tyr Ser Val Ile Glu Leu His Lys
       370                 375                 380
<210> 14
   <211> 381
   <212> PRT
   <213> Arabidopsis thaliana
<400> 14
   Met Glu Asn His Leu Gln His Ser Leu Thr Ile Ile Pro Lys Pro Asp
   1 5 10 15
   Leu Ile Lys Glu Glu Gln Arg Tyr His Glu Asp Thr Val Ser Leu Gln
   20 25 30
   Ala Glu Arg Ile Leu His Ala Met Thr Phe Pro Met Val Leu Lys Thr
   35 40 45
   Ala Leu Glu Leu Gly Val Ile Asp Met Ile Thr Ser Val Asp Asp Gly
   50 55 60
   Val Trp Leu Ser Pro Ser Glu Ile Ala Leu Gly Leu Pro Thr Lys Pro
   65 70 75 80
   Thr Asn Pro Glu Ala Pro Val Leu Leu Asp Arg Met Leu Val Leu Leu
   85 90 95
   Ala Ser His Ser Ile Leu Lys Tyr Arg Thr Val Glu Thr Gly Asp Asn
   100 105 110
   Ile Gly Ser Arg Lys Thr Glu Arg Val Tyr Ala Ala Glu Pro Val Cys
   115 120 125
   Thr Phe Phe Leu Asn Arg Gly Asp Gly Leu Gly Ser Leu Ala Thr Leu
   130 135 140
   Phe Met Val Leu Gln Gly Glu Val Cys Met Lys Pro Trp Glu His Leu
   145 150 155 160
   Lys Asp Met Ile Leu Glu Gly Lys Asp Ala Phe Thr Ser Ala His Gly
   165 170 175
   Met Arg Phe Phe Glu Leu Ile Gly Ser Asn Glu Gln Phe Ala Glu Met
   180 185 190
   Phe Asn Arg Ala Met Ser Glu Ala Ser Thr Leu Ile Met Lys Lys Val
   195 200 205
   Leu Glu Val Tyr Lys Gly Phe Glu Asp Val Asn Thr Leu Val Asp Val
   210 215 220
   Gly Gly Gly Ile Gly Thr Ile Ile Gly Gln Val Thr Ser Lys Tyr Pro
   225 230 235 240
   His Ile Lys Gly Ile Asn Phe Asp Leu Ala Ser Val Leu Ala His Ala
   245 250 255
   Pro Phe Asn Lys Gly Val Glu His Val Ser Gly Asp Met Phe Lys Glu
   260 265 270
   Ile Pro Lys Gly Asp Ala Ile Phe Met Lys Trp Ile Leu His Asp Trp
   275 280 285
   Thr Asp Glu Asp Cys Val Lys Ile Leu Lys Asn Tyr Trp Lys Ser Leu
   290 295 300
   Pro Glu Lys Gly Lys Val Ile Ile Val Glu Val Val Thr Pro Glu Glu
   305 310 315 320
   Pro Lys Ile Asn Asp Ile Ser Ser Asn Ile Val Phe Gly Met Asp Met
   325 330 335
   Leu Met Leu Ala Val Ser Ser Gly Gly Lys Glu Arg Ser Leu Ser Gln
   340 345 350
   Phe Glu Thr Leu Ala Ser Asp Ser Gly Phe Leu Arg Cys Glu Ile Ile
   355 360 365
   Cys His Ala Phe Ser Tyr Ser Val Ile Glu Leu His Lys
   370 375 380
<210> 15
   <211> 394
   <212> DNA
   <213> Brassica napus
<220>
   <221> CDS
   <222> (3)..(392)
   <223> coding for Brassica homologue H2
<400> 15
   cc aac ggc gac gag gtt tcc cgg aac atc gct atc caa cta gcc aaa     47
      Asn Gly Asp Glu Val Ser Arg Asn Ile Ala Ile Gln Leu Ala Lys
      1               5                   10                  15
   cac ggt tgt cgg ttg gtg ttg atg gga aac gag gct tct cta agg agc    95
   His Gly Cys Arg Leu Val Leu Met Gly Asn Glu Ala Ser Leu Arg Ser
                   20                  25                  30
   act gtg gac tac ata cga gtc tct gtt gat gga gcc ttc cca gtg gag   143
   Thr Val Asp Tyr Ile Arg Val Ser Val Asp Gly Ala Phe Pro Val Glu
               35                  40                  45
   ctc att gga gcc gac atg gaa gct gat agt gag gaa gat ttc tat gtt   191
   Leu Ile Gly Ala Asp Met Glu Ala Asp Ser Glu Glu Asp Phe Tyr Val
           50                  55                  60
   gct gtc caa aag gca tgg act cgt cta gga tct ttg gat gct ttt gtc   239
   Ala Val Gln Lys Ala Trp Thr Arg Leu Gly Ser Leu Asp Ala Phe Val
       65                  70                  75
   aac tgc tgt acc tac caa ggg aag atg cag gac att ctc cga gtg tct   287
   Asn Cys Cys Thr Tyr Gln Gly Lys Met Gln Asp Ile Leu Arg Val Ser
   80                  85                  90                  95
   gaa gat gag ttc aag aaa atc aca agg atc aat ctc acg gct aca tgg   335
   Glu Asp Glu Phe Lys Lys Ile Thr Arg Ile Asn Leu Thr Ala Thr Trp
                   100                 105                 110
   ttt atc ttg aag gct gtg gca agc atg atg aag gag aat gga aca gga   383
   Phe Ile Leu Lys Ala Val Ala Ser Met Met Lys Glu Asn Gly Thr Gly
               115                 120                 125
   ggc tcc att gg                                                    394
   Gly Ser Ile
           130
<210> 16
   <211> 130
   <212> PRT
   <213> Brassica napus
<400> 16
   Asn Gly Asp Glu Val Ser Arg Asn Ile Ala Ile Gln Leu Ala Lys His
   1 5 10 15
   Gly Cys Arg Leu Val Leu Met Gly Asn Glu Ala Ser Leu Arg Ser Thr
   20 25 30
   Val Asp Tyr Ile Arg Val Ser Val Asp Gly Ala Phe Pro Val Glu Leu
   35 40 45
   Ile Gly Ala Asp Met Glu Ala Asp Ser Glu Glu Asp Phe Tyr Val Ala
   50 55 60
   Val Gln Lys Ala Trp Thr Arg Leu Gly Ser Leu Asp Ala Phe Val Asn
   65 70 75 80
   Cys Cys Thr Tyr Gln Gly Lys Met Gln Asp Ile Leu Arg Val Ser Glu
   85 90 95
   Asp Glu Phe Lys Lys Ile Thr Arg Ile Asn Leu Thr Ala Thr Trp Phe
   100 105 110
   Ile Leu Lys Ala Val Ala Ser Met Met Lys Glu Asn Gly Thr Gly Gly
   115 120 125
   Ser Ile
   130
<210> 17
   <211> 429
   <212> DNA
   <213> Brassica napus
<220>
   <221> CDS
   <222> (2)..(427)
   <223> coding for Brassica homologue H3
<400> 17
   g gaa ttt tcg ggt cga cga ttt cgt act act ctg aat cta atg gcc aat 49
   Glu Phe Ser Gly Arg Arg Phe Arg Thr Thr Leu Asn Leu Met Ala Asn
   1               5                   10                  15
   aag gtg ttg atg aca gac aac ggc gac cag gtt tcc cgg aac atc gct     97
   Lys Val Leu Met Thr Asp Asn Gly Asp Gln Val Ser Arg Asn Ile Ala
               20                  25                  30
   atc caa cta gcc aaa cac ggt tgt cgg ttg gtg ttg atg gga aac gag     145
   Ile Gln Leu Ala Lys His Gly Cys Arg Leu Val Leu Met Gly Asn Glu
           35                  40                  45
   gct tct cta agg agc act gtg gac tac ata cga ttc tct gat gat gga     193
   Ala Ser Leu Arg Ser Thr Val Asp Tyr Ile Arg Phe Ser Asp Asp Gly
       50                  55                  60
   gcc ttc cca gtg gag ctc att gga gcc gac atg gaa gct gat agt gag     241
   Ala Phe Pro Val Glu Leu Ile Gly Ala Asp Met Glu Ala Asp Ser Glu
   65                  70                  75                  80
   gaa gat ttc tat gtt gct gtc caa acg gca tgg act cgt cta gga tct     289
   Glu Asp Phe Tyr Val Ala Val Gln Thr Ala Trp Thr Arg Leu Gly Ser
                   85                  90                  95
   ttg gat gct ttt gtc aac tgc tgt acc tac caa ggg aag atg cag gac     337
   Leu Asp Ala Phe Val Asn Cys Cys Thr Tyr Gln Gly Lys Met Gln Asp
               100                 105                 110
   att ctc cga gtg tct gaa gat gag ttc aag aaa atc aca cgg atc aat     385
   Ile Leu Arg Val Ser Glu Asp Glu Phe Lys Lys Ile Thr Arg Ile Asn
           115                 120                 125
   ctc acg gct aca tgg ttt atc ttg aag gct gtg gca agc atg at          429
   Leu Thr Ala Thr Trp Phe Ile Leu Lys Ala Val Ala Ser Met
       130                 135                 140
<210> 18
   <211> 142
   <212> PRT
   <213> Brassica napus
<400> 18
   Glu Phe Ser Gly Arg Arg Phe Arg Thr Thr Leu Asn Leu Met Ala Asn
    1               5                  10                  15
   Lys Val Leu Met Thr Asp Asn Gly Asp Gln Val Ser Arg Asn Ile Ala
               20                  25                  30
   Ile Gln Leu Ala Lys His Gly Cys Arg Leu Val Leu Met Gly Asn Glu
           35                  40                  45
   Ala Ser Leu Arg Ser Thr Val Asp Tyr Ile Arg Phe Ser Asp Asp Gly
       50                  55                  60
   Ala Phe Pro Val Glu Leu Ile Gly Ala Asp Met Glu Ala Asp Ser Glu
   65                  70                  75                  80
   Glu Asp Phe Tyr Val Ala Val Gln Thr Ala Trp Thr Arg Leu Gly Ser
                   85                  90                  95
   Leu Asp Ala Phe Val Asn Cys Cys Thr Tyr Gln Gly Lys Met Gln Asp
               100                 105                 110
   Ile Leu Arg Val Ser Glu Asp Glu Phe Lys Lys Ile Thr Arg Ile Asn
           115                 120                 125
   Leu Thr Ala Thr Trp Phe Ile Leu Lys Ala Val Ala Ser Met
       130                 135                 140
<210> 19
   <211> 436
   <212> DNA
   <213> Brassica napus
<220>
   <221> CDS
   <222> (3)..(419)
   <223> coding for Brassica homologue H4
<400> 19
   gt cga cga ttt cgt gga gaa aac aat cta act gga aag atc caa atg     47
      Arg Arg Phe Arg Gly Glu Asn Asn Leu Thr Gly Lys Ile Gln Met
       1               5                  10                  15
   gta tat gca gcc gag ccg gtt tgc acg ctt ttc tta aaa cat ggt cat    95
   Val Tyr Ala Ala Glu Pro Val Cys Thr Leu Phe Leu Lys His Gly His
                   20                  25                  30
   gag tcg ggt tca ctc atg tcc cta ttc atg gtg cac cat agc caa gtc   143
   Glu Ser Gly Ser Leu Met Ser Leu Phe Met Val His His Ser Gln Val
               35                  40                  45
   ttt ttc gaa act tgg aca cat ttg aaa gat ctg ata caa gaa gga aaa   191
   Phe Phe Glu Thr Trp Thr His Leu Lys Asp Leu Ile Gln Glu Gly Lys
           50                  55                  60
   gat aca ttc att tct gct cat ggc atg agg atc ttt gaa tac atc ggt   239
   Asp Thr Phe Ile Ser Ala His Gly Met Arg Ile Phe Glu Tyr Ile Gly
       65                  70                  75
   ttg aat gaa caa ttc gct tgt atg ttt aac cat gca atg tca gaa tct   287
   Leu Asn Glu Gln Phe Ala Cys Met Phe Asn His Ala Met Ser Glu Ser
   80                  85                  90                  95
   tct acc atg atc atg aag aag att tta gaa gtt tac aga gga ttc gaa   335
   Ser Thr Met Ile Met Lys Lys Ile Leu Glu Val Tyr Arg Gly Phe Glu
                   100                 105                 110
   gat att aaa act ttg gtg gat att gga gga gga ctt ggc acc aca cta   383
   Asp Ile Lys Thr Leu Val Asp Ile Gly Gly Gly Leu Gly Thr Thr Leu
               115                 120                 125
   aat ctg gtt act tcc aag tat cct cat ata agg gta taatttcgat        429
   Asn Leu Val Thr Ser Lys Tyr Pro His Ile Arg Val
           130                 135
   taaactc                                                           436
<210> 20
   <211> 139
   <212> PRT
   <213> Brassica napus
<400> 20
   Arg Arg Phe Arg Gly Glu Asn Asn Leu Thr Gly Lys Ile Gln Met Val
    1               5                  10                  15
   Tyr Ala Ala Glu Pro Val Cys Thr Leu Phe Leu Lys His Gly His Glu
               20                  25                  30
   Ser Gly Ser Leu Met Ser Leu Phe Met Val His His Ser Gln Val Phe
           35                  40                  45
   Phe Glu Thr Trp Thr His Leu Lys Asp Leu Ile Gln Glu Gly Lys Asp
       50                  55                  60
   Thr Phe Ile Ser Ala His Gly Met Arg Ile Phe Glu Tyr Ile Gly Leu
   65 70 75 80
   Asn Glu Gln Phe Ala Cys Met Phe Asn His Ala Met Ser Glu Ser Ser
   85 90 95
   Thr Met Ile Met Lys Lys Ile Leu Glu Val Tyr Arg Gly Phe Glu Asp
   100 105 110
   Ile Lys Thr Leu Val Asp Ile Gly Gly Gly Leu Gly Thr Thr Leu Asn
   115 120 125
   Leu Val Thr Ser Lys Tyr Pro His Ile Arg Val
   130 135
<210> 21
   <211> 418
   <212> DNA
   <213> Brassica napus
<220>
   <221> CDS
   <222> (1)..(417)
   <223> coding for Brassica homologue H5
<400> 21
   gct gaa ccg gtt tgc acg ctt ttt tta acc cgt ggt gac gac tcg ggt 48
   Ala Glu Pro Val Cys Thr Leu Phe Leu Thr Arg Gly Asp Asp Ser Gly
   1 5 10 15
   act cac aag tcc ctc ttc atg ttg ctc aat agc caa gta ttt ttc aag 96
   Thr His Lys Ser Leu Phe Met Leu Leu Asn Ser Gln Val Phe Phe Lys
   20 25 30
   aca tgg gat aat ctg aag ggt gtg ata caa gaa gga aaa gat gcg ttt 144
   Thr Trp Asp Asn Leu Lys Gly Val Ile Gln Glu Gly Lys Asp Ala Phe
   35 40 45
   agt tca gct cat ggc atg cca tta ttc gaa tac atc ggt ttg gat gag 192
   Ser Ser Ala His Gly Met Pro Leu Phe Glu Tyr Ile Gly Leu Asp Glu
   50 55 60
   caa ttc gct ggt atg ttt aac cat gca atg gca gaa tct tct acc atc 240
   Gln Phe Ala Gly Met Phe Asn His Ala Met Ala Glu Ser Ser Thr Ile
   65 70 75 80
   att atg aag aaa att tta gaa gtt tac aga gga ttc gaa gat gta aat 288
   Ile Met Lys Lys Ile Leu Glu Val Tyr Arg Gly Phe Glu Asp Val Asn
   85 90 95
   act ttg gtg gat att gga gga gga ctt ggc acc gta cta aac ctt gtc 336
   Thr Leu Val Asp Ile Gly Gly Gly Leu Gly Thr Val Leu Asn Leu Val
   100 105 110
   act tcc aag tat cct caa att aag ggt atc aat ttc gat tta acc atg 384
   Thr Ser Lys Tyr Pro Gln Ile Lys Gly Ile Asn Phe Asp Leu Thr Met
   115 120 125
   gtt tta gcc aat gct cct tct tat cca gga gtg g 418
   Val Leu Ala Asn Ala Pro Ser Tyr Pro Gly Val
   130 135
<210> 22
   <211> 139
   <212> PRT
   <213> Brassica napus
<400> 22
   Ala Glu Pro Val Cys Thr Leu Phe Leu Thr Arg Gly Asp Asp Ser Gly
   1               5                   10                  15
   Thr His Lys Ser Leu Phe Met Leu Leu Asn Ser Gln Val Phe Phe Lys
               20                  25                  30
   Thr Trp Asp Asn Leu Lys Gly Val Ile Gln Glu Gly Lys Asp Ala Phe
           35                  40                  45
   Ser Ser Ala His Gly Met Pro Leu Phe Glu Tyr Ile Gly Leu Asp Glu
       50                  55                  60
   Gln Phe Ala Gly Met Phe Asn His Ala Met Ala Glu Ser Ser Thr Ile
   65                  70                  75                  80
   Ile Met Lys Lys Ile Leu Glu Val Tyr Arg Gly Phe Glu Asp Val Asn
                   85                  90                  95
   Thr Leu Val Asp Ile Gly Gly Gly Leu Gly Thr Val Leu Asn Leu Val
               100                 105                 110
   Thr Ser Lys Tyr Pro Gln Ile Lys Gly Ile Asn Phe Asp Leu Thr Met
           115                 120                 125
   Val Leu Ala Asn Ala Pro Ser Tyr Pro Gly Val
       130                 135
<210> 23
   <211> 10
   <212> PRT
   <213> Künstliche Sequenz
<220>
   <223> Beschreibung der künstlichen Sequenz: protein motive
<220>
   <221> VARIANT
   <222> (4)
   <223> E/Q-variation
<400> 23
   Asn Gly Asp Glu Val Ser Arg Asn Ile Ala
   1               5                   10
<210> 24
   <211> 9
   <212> PRT
   <213> Künstliche Sequenz
<220>
   <223> Beschreibung der künstlichen Sequenz: protein motive
<220>
   <221> VARIANT
   <222> (7)
   <223> R/K-variation
<400> 24
   Leu Ala Lys His Gly Cys Arg Leu Val
   1 5
<210> 25
   <211> 15
   <212> PRT
   <213> Künstliche Sequenz
<220>
   <223> Beschreibung der künstlichen Sequenz: protein motive
<400> 25
   Met Gly Asn Glu Xaa Ser Leu Arg Ser Xaa Val Asp Xaa Ile Arg
   1 5 10 15
<210> 26
   <211> 17
   <212> PRT
   <213> Künstliche Sequenz
<220>
   <223> Beschreibung der künstlichen Sequenz: protein motive
<220>
   <221> VARIANT
   <222> (14)
   <223> Q/E-variation
<400> 26
   Thr Tyr Gln Gly Lys Xaa Gln Asp Ile Leu Xaa Val Ser Gln Asp Glu
   1 5 10 15
   Phe
<210> 27
   <211> 17
   <212> PRT
   <213> Künstliche Sequenz
<220>
   <223> Beschreibung der künstlichen Sequenz: protein motive
<220>
   <221> VARIANT
   <222> (3)
   <223> K/R-variation
<400> 27
   Ile Thr Lys Ile Asn Leu Thr Ala Xaa Trp Phe Xaa Leu Lys Ala Val
   1 5 10 15
   Ala
<210> 28
   <211> 9
   <212> PRT
   <213> Künstliche Sequenz
<220>
   <223> Beschreibung der künstlichen Sequenz: protein motive
<400> 28
   Ala Glu Pro Val Cys Thr Xaa Phe Leu
   1 5
<210> 29
   <211> 16
   <212> PRT
   <213> Künstliche Sequenz
<220>
   <223> Beschreibung der künstlichen Sequenz: protein motive
<400> 29
   Glu Gly Lys Asp Xaa Phe Xaa Ser Ala His Gly Met Xaa Xaa Phe Glu
   1 5 10 15
<210> 30
   <211> 11
   <212> PRT
   <213> Künstliche Sequenz
<220>
   <223> Beschreibung der künstlichen Sequenz: protein motive
<400> 30
   Glu Gln Phe Ala Xaa Met Phe Asn Xaa Ala Met
   1 5 10
<210> 31
   <211> 17
   <212> PRT
   <213> Künstliche Sequenz
<220>
   <223> Beschreibung der künstlichen Sequenz: protein motive
<220>
   <221> VARIANT
   <222> (8)
   <223> V/I-variation
<220>
   <221> VARIANT
   <222> (13)
   <223> K/R-variation
<400> 31
   Ala Thr Xaa Ile Met Lys Lys Val Leu Glu Val Tyr Lys Gly Phe Glu
   1 5 10 15
   Asp
<210> 32
   <211> 11
   <212> PRT
   <213> Künstliche Sequenz
<220>
   <223> Beschreibung der künstlichen Sequenz: protein motive
<220>
   <221> VARIANT
   <222> (5)
   <223> V/I-variation
<400> 32
   Thr Leu Val Asp Val Gly Gly Gly Xaa Gly Thr
   1 5 10
<210> 33
   <211> 21
   <212> DNA
   <213> Künstliche Sequenz
<220>
   <223> Beschreibung der künstlichen Sequenz: oligonucleotide primer
<400> 33
   cacttttccc ggcaataaca t 21
<210> 34
   <211> 21
   <212> DNA
   <213> Künstliche Sequenz
<220>
   <223> Beschreibung der künstlichen Sequenz: oligonucleotide primer
<400> 34
   atcaggaagt gatggagcat c 21
<210> 35
   <211> 19
   <212> DNA
   <213> Künstliche Sequenz
<220>
   <223> Beschreibung der künstlichen Sequenz: oligonucleotide primer
<400> 35
   gaccctgtcc cacctccaa 19
<210> 36
   <211> 21
   <212> DNA
   <213> Künstliche Sequenz
<220>
   <223> Beschreibung der künstlichen Sequenz: oligonucleotide primer
<400> 36
   tgagaactgc gattgtttgc a 21